# EUROPEAN PATENT SPECIFICATION

(11) **EP 2 558 510 B1**
(45) Date of publication and mention of the grant of the patent: **20.05.2015**
(21) Application number: 11715118.3
(22) Date of filing: 04.04.2011
(51) Int. Cl.: A61Q 19/00, A61Q 5/02, A61Q 19/08, A61K 8/81, C08F 220/38, C08F 290/06, A61Q 19/10, C08F 220/18

(54) **CATIONIC/CATIONOGENIC COMB COPOLYMER COMPOSITIONS AND PERSONAL CARE PRODUCTS CONTAINING THE SAME**
KATIONISCHE/KATIONOGENE KAMMCOPOLYMERZUSAMMENSETZUNGEN UND KÖRPERPFLEGEPRODUKTE DAMIT
COMPOSITIONS DE COPOLYMÈRES PEIGNES CATIONIQUES ET CATIONOGÉNIQUES ET PRODUITS DE SOINS PERSONNELS CONTENANT LESDITES COMPOSITIONS

(30) Priority: 13.04.2010 US 323484 P; 25.02.2011 US 201113035383
(43) Date of publication of application: 20.02.2013
(73) Proprietor: Air Products and Chemicals, Inc., Allentown, PA 18195-1501 (US); Landec Corporation, Menlo Park, California 94025 (US)
(72) Inventor: NOOR, Mussarat, Pittstown, New Jersey 12534 (US); GODDARD, Richard Joseph, Fogelsville, Pennsylvania 18051 (US); SCHAFER, Julian Everett, Montara, California 94037 (US); BITLER, Steven Paul, Menlo Park, California 94025 (US)
(74) Representative: Kador & Partner
(86) International application number: PCT/US2011/031044
(87) International publication number: WO 2011/130030

(56) References cited:
- EP-A2- 1 347 013
- US-A1- 2004 167 304

## Description

### CROSS-REFERENCE TO RELATED APPLICATIONS

This Application claims the benefit of U.S. Provisional Application No. 61/323,484, filed on April 13, 2010.

### BACKGROUND OF THE INVENTION

The present disclosure relates generally to the field of copolymers and more particularly to cationic/cationogenic comb copolymer compositions and their use in personal care and other products.

Polymeric thickeners are sometimes used for cosmetic and other personal care products. Many known thickeners use hydrophilic polymers with anionic functionality derived either from high levels of acid monomers, such as [meth]acrylic acid or 2-acrylamido-2-methylpropane sulfonic acid (AMPS), or from polyethylene glycol (PEG) content derived either from ethoxylated monomers or grafting. In some cases, thickening efficiency is enhanced will crosslinking and/or hydrophobic modification, often by the inclusion of associative monomers that add a very limited amount of hydrophobic character. While these polymers offer compatibility with anionic surfactants used for shampoo and other cleansing agents, they offer limited deposition and wash-off resistance.

Synthetic cationic polymers often include cross-linking, and/or are high molecular weight polymers with only limited deposition and sensory benefit. These polymers are frequently modified with nonionic monomers, usually with hydrophilic amide or hydroxyl functionality, to improve cosmetic formulation compatibility. Some cationic polymers are rendered ampholytic by inclusion of a limited amount of acid functionality to improve stability and compatibility at pH 5-6 as desirable for skin and hair formulations.

Overall, the use of such prior modifiers has generally been unsatisfactory as they fail to provide more than a modest benefit in any one category nor do they offer benefits across categories of desirable attributes, resulting in the need to incorporate multiple different modifiers and other additives to overcome their respective individual deficiencies. Cationically modified polysaccharides, for example, offer slip and sensory benefits but limited deposition.

CA 2,662,401 relates to an ampholytic copolymer based on quaternized nitrogen-containing monomers that has a molar excess of cationogenic/cationic groups over anionogenic/anionic groups, to cosmetic or pharmaceutical compositions including at least one such ampholytic copolymer.

U.S. 7,015,279 is directed to linear and crosslinked cationic polyelectrolytes obtained by copolymerization of at least one cationic monomer with at least one neutral monomer and at least one nonionic surfactant monomer and their use as a thickener for cosmetic or pharmaceutical compositions and other applications.

U.S. 7,279,154 relates to uses of comb polymers of acryloyldimethyltaurine and/or acryloyldimethyltaurates in cosmetic, pharmaceutical and dertnatological applications as thickeners, dispersing agents, suspending agents, emulsifiers, stabilizers, solubilizers, condiloning agents, consistency-giving agents, lubricants, bonding agents and/or conditioners. The copolymer is obtained by free-radical copolymerization of acryloyldimethyltaurine (AMPS) and/or acryloyldimethyltaurates; and optionally, one or more further olefinically unsaturated, noncationic comonomers; optionally, one or more olefinically unsaturated, cationic comonomers; optionally, one or more silicon-containing component(s); optionally, one or more fluorine-containing component(s); optionally, one or more macromonomers; and optionally, the copolymerization taking place in the presence of at least one polymeric additive, provided that the acryloyldimethyltaurine (AMPS) and/or acryloyldimethyltaurates is copolymerized with at least one silicon-containing component, fluorine-containing component, macromonomers, or polymeric additive.

In U.S. 2007/0248561, cosmetic and pharmaceutical preparations are described having one or more water-soluble noncrosslinked copolymers containing one or more structural units of a specific formula and one or more structural units of a second formula. The formula also includes one or more water-soluble orwater-swellable crosslinked or noncrosslinked copolymeric or homopolymeric thickeners. These polymers are generally of high ionic content and water solubility.

U.S. 2010/0056647 describes copolymers that contain at least one basic amino substituentthat is cationic at low pH and two or more hydrophobicallymodified polyoxyalkylene substituents derived from an associative vinyl monomer that can optionally contain substituent groups derived from other monomer units, such as crosslinking monomer units, hydroxy-substituted nonionic vinyl monomer units, chain transfer agent units, and polymeric stabilizers and which are said to generally exhibit associative properties in aqueous solution.

However, it is believed that none of these references disclose or recognize a composition that achieves both sensory and deposition benefits, combined with substantial rheology enhancement in many cosmetic formulations, nor the ability to deposit or retain active ingredients. Existing cationic polymers generally have substantivity to keratinous substances and do not significantly improve deposition/retention of actives. Furthermore, in most of the references that employ associative monomers in combination with ionic or ionogenic monomers at all, the ratio of ionic or ionogenicgroups to associative groups is very high.

It would be advantageous to provide a cationic copolymer that could achieve multiple benefits and provide a balance of desirable properties in personal care and other products, and particularly to accomplish both increased deposition effectiveness and good sensory attributes.

Exemplary embodiments of the present invention overcome these and other drawbacks by provding cationic copolymer compositions that provide a balance of desirable characteristics for use in personal care products to achieve superior results, while decreasing the number of separate ingredients that must be employed to address each desired attribute through the recognition of a relationship between the weight percentages of lipophilic associative groups and bnic/ionogenic groups in the backbone of the copolymer.

### BRIEF SUMMARY OF THE INVENTION

In one embodiment, a cationic or cationogenic comb copolymer comprises
A) one or more repeating units derived from olefinically unsaturated cationic or cationogenic comonomers; and
B) one or more repeating units having the formula 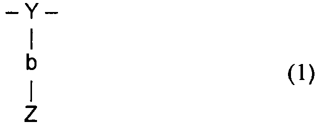 in which Y is a moiety forming part of the copolymer backbone, Z is a moiety that exhibits association in the presence of other Z moieties or with other moieties within the ultimate formulation in which the copolymer wil be employed, and b is a bond or moiety that links the Z moiety to the Y moiety, wherein the Z moiety is alkyl, aryl, aralkyl, fluoroalkyl, silicone and silane, and wherein the weight ratio of associative moieties Z to ionic and inorganic monomers is between 0, 1 to 5,5 or wherein the weight ratio of associative moieties Z to ionic monomers is between 0.15 and 6.5.

The copolymer generally further includes one or more repeating units derived from at least one of the following categories:
C) acrylamide monomers;
D) one or more olefinically unsaturated hydrophilic monomers that are not A, B or C; or
E) one or more olefinically unsaturated monomers that are not A, B, C or D.

In another embedment, the cationic/cationogeniccomb copolymer has a weight percent of ionic and ionogenic monomers incorporated into the copolymer of between about 2 and about 45%, the weight percent of associative groups Z in the copolymer is between about 2 and about 50%, and the weight ratio of associative groups Z to ionic and ionogenic monomers is between about 0.1 and about 5.5.

In another embodiment, the cationic/cationogeniccomb copolymer has a weight percent of ionic monomers incorporated into the copolymer of between about 2 and about 45%, the weight percent of associative groups Z in the copolymer is between about 2and about 50%, and the weight ratio of associative groups Z to ionic monomers is between about 0.15 and about 6.5.

In yet another embodiment, the cationic/cationogeniccomb copolymer has a weight percent of ionic and ionogenic monomers incorporated into the copolymer of between about 2 and about 45%, the weight percent of associative groups Z in the copolymer is between about 2 and about 50%, and the viscosity of a 3% by weight (solids) of the copolymer in distilled water is less than about 0.25 Pa·s (250 centipoise (cP)).

The cationic/cationogenic comb copolymers disclosed herein may be incorporated into any cosmetically acceptable media and in some embodiments a personal care product comprises a cosmetically acceptable base media and between about 0.1 to about 20% by weight of the cationic/cationogeniccomb copolymer.

In certain embodiments, the personal care product further comprises an active ingredient wherein the cationic/cationogenic comb copolymer increases the deposition of the active ingredient to a keratinous structure by at least about 10% over a personal care product having the same formulation except for the cationic/cationogenic comb copolymer. In still other embodiments, the use of the cationic/cationogeniccomb copolymer can itself demonstrate deposition to keratinous substrates.

In certain embodiments, the personal care product comprises a comb polymer wherein the polymer is not crosslinked (as defined below) and has relatively low water solubility (as defined below).

Compositions and methods in accordance with exemplary embodiments can be used in personal care applications to provide multiple benefits of rheology modification/thickening, deposition and retention of actives, water resistance, and sensory (conditioning, slip, silky feel) benefits, all of which can be achieved using a single polymer in cosmetic media. Certain embodiments exhibit good stability and provide particular benefits in low pH systems.

Exemplary embodiments provide a cationic/cationogeniccomb copolymer having a particular combination of repeating units to provide rheology modification with a fatty or lipophilic component of a personal care product. The combination of a sufficient amount of hydrophilic monomer for compatibility in aqueous formulations with substantial hydrophobic content both enhances thickening by association and dramatically improves deposition and wash-off resistance.

Other features and advantages of the present invention will be apparent from the following more detailed description of exemplary embodiments that illustrate, by way of example, the principles of the invention.

### DETAILED DESCRIPTION OF THE INVENTION

### Description of copolymer and its constituent groups

Embodiments are directed to cationic and cationogenic comb copolymers that comprise repeating units containing associative moieties and that are formed from at least two categories of monomers, and typically at least three categories of monomers. In some embodiments, four or five categories may be employed. Embodiments are also directed to personal care products and other useful compositions that include these copolymers as an ingredient.

The cationic or cationogenic comb copolymer comprises
A) one or more repeating units derived from olefinically unsaturated cationic or cationogenic comonomers; and
B) one or more repeating units having the formula 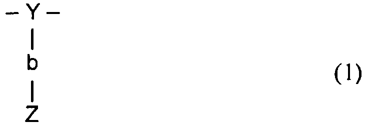 in which Y is a moiety forming part of the copolymer backbone, Z is a moiety that exhibits association in the presence of other Z moieties or with other moieties within the ultimate formulation in which the copolymer wil be employed, and b is a bond or moiety that links the Z moiety to the Y moiety. By "exhibits association" is meant that the Z moieties experience weak attractive bonding (e.g. Van der Waals interaction, hydrogen bonding) that cause them to associate with one another or with other moieties within the ultimate formulation, as will readily be appreciated and understood by those of ordinary skill in the art. The association can result in the formation of a dynamic three-dimensional network structure of micelles or having micelle-like features; although the associations in the network are dynamic and the forces are weak, the associations often have sufficient lifetime and strength to exhibit increased viscosity.

In typical embodiments, the copolymer further includes one or more repeating units derived from at least one of the following categories:
C) acrylamide monomers;
D) one or more olefinically unsaturated hydrophilic monomers that are not A, B or C; or
E) one or more olefinically unsaturated monomers that are not A, B, C or D.

That is, in typical embodiments, the copolymer includes at least one each of A and B and further includes repeating units derived from at least one of C, D or E. In other embodiments, the copolymer includes at least one each of A and B and further includes repeating units derived from monomers from two of the C, D, and E categories, while in a still other embodiment, the copolymer includes at least one each of A and B along wth repeating units derived from monomers from all three of groups C, D and E. It will be appreciated that in each case, multiple members from the same group may also be present.

The cationic and cationogenic monomers from which the group A constituent is selected is any cationic or cationogenic monomer. Particularly suitable cationic monomers include those which include quaternized nitrogen or otherwise introduce N⁺ into the backbone or a side chain of the formed copolymer. Suitable cationogenic monomers include those that contain nitrogen and which can be acid-neutralized to form charged cationic groups or which can be reacted with suitable alkylating agents to form quaternary ammonium groups, either prior or subsequent to copolymer formation.

Exemplary cationic monomers containing nitrogen include, but are not limited to, at least one member selected from the group consisting of ammonium salt-containing [meth]acrylates, trimethylammonium methylmethacrylate chloride, methacrylamidopropyl trimethylammonium chloride, trimethylammonium ethylmethacrylate chloride, N,N-(dimethyl or diethyl)aminoethyl [meth]acrylate methosulfate, [2-(methacryloyloxy)alkyl]trialkylammonium halide, [2-(methacryloyloxy)alkyl]trialkylammonium alkosulfate, [2-(methacryloyloxy)ethyl]trimethylammonium chloride, [2-(acryloyloxy)alkyl]trialkyl ammonium halide, [2-(acryloyloxy)alkyl]trialkyl ammonium alkosulfate, [2-(acryloyloxy)ethyl]trimethyl ammonium chloride, and [2-(acrylamido)ethyl]trimethyl ammonium chloride.

Examples of nitrogen containing monomers that can be converted to a cationic state, (i.e., are cationogenic) includebut are not limited to at least one member selected from the group consisting of: N,N-dialkylamino [meth]acrylates, such as dimethylaminoethyl [meth]acrylate, dimethylaminopropyl [meth]acrylate, diethylaminoethyl [meth]acrylate, and diethylaminopropyl [meth]acrylate. N,N-dialkylamino [meth]acrytamides may also be used, such as dimethylaminopropyl [meth]acrylamide and diethylaminopropyl [meth]acrylamide. Other exemplary cationogenic monomers include at least one member from the group consisting of 2-methacryloxy-N-ethylmorpholine, 2-t-butylaminoethyl methacrylate, acryloylmorpholine, 1-piperidinoethyl [meth]acrylate, and dimethylaminocyclohexyl [meth]acrylate.

It will be appreciated that the cationic monomers of Group A can incorporate one of or more counterions including chloride, bromide, sulfate, and phosphate by way of example only. In some cases, halide counterions may be desirable (e.g., over other counterions as the halides have a tendency not to introduce color or odor). Suitable alkylating agents include alkyl halides or sulfates having between one and four carbon atoms, such as ethyl or methyl chloride or bromide, dimethyl or diethyl sulfate. Other suitable alkylating agents include mineral acids, for example, phosphoric acid, sulfuric acid or hydrochloric acid.

In the repeating units of Group B, the associative moiety Z can be incorporated into the comb copolymer as a component of a monomer used in the copolymerization or it may result from a reaction of the associative group and a functional monomer during or subsequent to polymerization. Thus, it may be possible in some cases for the moiety Y of the copolymer backbone in the repeating unit from which the associative moiety Z extends to be a repeating unit derived from the monomer of groups A, C, D or E, but which falls into group B by virtue of its subsequent linkage to the associative moiety Z.

The Z moiety is generally hydrophobic in nature, and is alkyl, aryl, aralkyl, fluoroalkyl, silicone or silane. For example, hydrocarbon Z groups include linear, branched or cyclic alkyl, aryl or aralkyl groups having in the range of 8 to 50 carbon atoms, in one embodiment having in the range of 10 to 22 carbon atoms, and in another embodiment, the groups have in the range of 10 to 18 carbon atoms. For example, Z alkyl components include but are not limited to at least one member selected from the group consisting of alkyl groups (such as octyl, nonyl, decyl, dodecyl, tridecyl, tetradecyl, pentadecyl, hexadecyl, heptadecyl, octadecyl, nonadecyl, eicosyl, arachinyl, and behenyl) derived from fatty alcohols, fatty amines, or fatty acids. Suitable Z alkyl components also include mixtures of such compounds, and where mixtures of alkyl components are used the ranges of carbon atoms indicated above are based on the weight average of the mixture. Exemplary commercial mixtures include alcohols sold under the tradenames Alfol® and Novel® from Sasol, alcohols sold under the tradename Neodol® from Shell, as well as alcohols mixtures sold by Baker Petrolite under the tradename Unilin® alcohols, in which the average chain length is C25, C30, C40 or C50, as well as alcohols derived from coconut or other natural oils. Also included may be predominantly linear alkenyl chains including at least one member selected from the group consisting of dodecenyl, tridecenyl, tetradecenyl, pentadecenyl, hexadecenyl, octadecenyl, and docosenyl.

Silicone containing Z groups can be linear, branched or cyclic having between 3 and 25 linked SiO units. In one embodiment, it has between 3 and 8 SiO units, and in another embodiment has between 3 and 5 SiO units. Fluoroalkyl containing Z groups include fluorocarbon groups having in the range of 6 to 50 carbons, and in the range of 8 to 30 carbons in another embodiment.

Examples of the linkage b include covalent bonds formed by at least one of ester, carbonyl, amide, amine oxide, hydrocarbon, amino, ether, and polyoxyalkylene groups linking the copolymer backbone to the associative group Z. The linkage b may also occur through ionic salt linkages.

The following is a generic structure of a monomer that forms a repeating unit of Group B, having an optional polyoxyalkylene component:

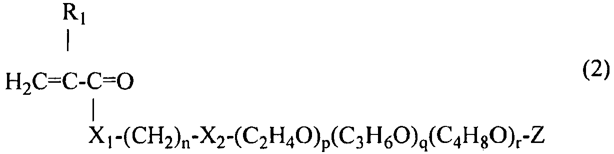

X₁ and X₂ are independently either O, S, NH, or NHCOO; R₁ is either H or CH₃; n is between 0-6 (in which X₂ is omitted when n=0); p is between 0-50, q is between 0-50 and r is between 0-50. Repeating units of p, q and r may be incorporated in random, alternating, block or gradient structures. Z is any moiety as previously described. In one embodiment, wherein p = q = r = 0 the weight percentage of the Group B monomer can be less than about 50wt.%, in some cases less than about 25wt.%, in another case, less than about 10wt% and in a further case, less than about 5wt.%. For example, in one aspect of this embodiment, at least one of p, q or r is an integer between one and fifty (e.g., greater than or equal to 1). For example, in another aspect of this embodiment, p is between 1 and 50, in some cases between 3 and 30 and in other cases between 2 and 10. In certain embodiments, X₁=O, n=q=r=0, p is between 3 and 30 and Z is an alkyl group having in the range of 10 to 18 carbon atoms. In another embodiment, R₁=H, X₁=O, n=q=r-0, p is between 2 and 10 and Z is an alkyl group having in the range of 10 to 16 carbon atoms. Examples of monomers of structure (2) that will form Group B repeating units include at least one member selected from the group consisting of stearyl poly(oxyethyl)₂₅ methacrylate, lauryl poly(oxyethyl)₄ acrylate, octyl poly(oxypropyl)₆poly(oxyethyl)₁₂-N-methyl acrylamide, N-octadecylmethacrylamide, and lauryl [meth]acrylate, for example.

The following is a generic structure of a monomer that forms a repeating unit of Group B having polyalkylamine groups:

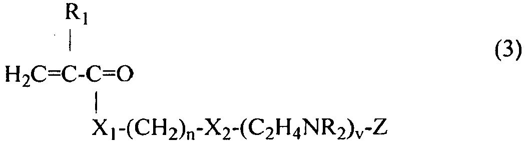

X₁ and X₂ are independently either O, S, NH, or NHCOO; R₁ and R₂ are independently either H or CH₃; n is between 0-6 (and X₂ is omitted when n=0); and v is between 1-50. The Z moiety is as previously described.

The acrylamide monomers of Group C may be any monomer having the following formula

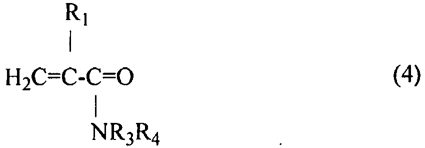

R₁ is either H or CH₃, R₃ and R₄ are independently H, C1-C6 alkyl, C1-C6 alkenyl, C1-C6 alkoxyalkyl, or alkylaminoalkyl. Exemplary Group C monomers include, but are not limited to at least one member selected from the group consisting of acrylamide, methacrylamide, N-methylacrylamide, N-methylmethacrylamide, N,N-dimethylacrylamide, N,N-dimethylmethacrylamide, N,N-diethylacrylamide, diacetoneacrylamide, N,N-dimethylaminopropylacrylamide, N-butoxymethylacrylamide, N-ethoxymethylacrylamide, N-n-butylacrylamide, N-tertbutylacrylamide, N-isopropylacrylamide, N-methylolacrylamide, N-methoxymethylacrylamide, N-ethylacrylamide, N-(3-methoxypropyl)acrylamide, N-n-propylacrylamide, N-trimethylbutylacrylamide, N-isooctylacrylamide, N-acetylmethacrylamide, N-butoxymethylmethacrylamide, N, N-dibutylaminopropylmethacrylamide, N,N-dimethylaminopropylmethacrylamide, N,N-diethylmethacrylamide, N-(2,2-dimethoxyethyl)methacrylamide, N,N-dimethylaminoethylmethacrylamide, N-ethylmethacrylamide, N-methoxymethylmethacrylamide, N-(3-methoxypropyl)methacrylamide, and N-methylolmethacrylamide.

Monomers of Group D are any olefinically unsaturated hydrophilic monomers that do not fall within Group A, B or C, and generally have a water solubility at 25 °C greater than about 50 g/L. These monomers can be ionic, ionogenic, or nonionic. Exemplary anionic monomers include salts of at least one of monomers with carboxylic or dicarboxylic acid groups, anhydrides, and salts of sulfonic or phosphonic acid, as well as sals of half-esters of dicarboxylic acids. Such monomers include at least one member selected from the group consisting of ammonium [meth]acrylate, sodium itaconate, sodium citraconate, sodium maleate, sodium [meth]acrylate, and ammonium acrylamidomethylpropane sulfonate, all by way of example. Anionogenic monomers include any monomer that can be base-neutralized to form anionic groups, either prior or subsequent to polymerization. Examples of anionogenic monomers include at least one member selected from the group consisting of [meth]acrylic acid, itaconic acid, crotonic acid, citraconic acid, maleic acid, methyl maleate, butyl maleate, acrylamidomethylpropane sulfonic acid, fumaric acid, mesaconic acid, glutaconic acid, maleic anhydride, and itaconic anhydride.

Zwitterionic and/or amphoteric monomers may also be employed as group D monomers. Zwitterionic monomers or repeats are those which contain both a formal positive and negative charge on different atoms; although they carry a net charge of zero, they are considered ionic monomers. Examples of zwitterionic monomers that may be used as group D monomers include those having pendant carboxybetaine, phosphobetaine, or sulfobetaine moieties; specific examples include 2-methacryloxyethyl phosphorylcholine, 2-(methacryloyloxy)ethyl-2'-(trimethylammonium)ethyl phosphate inner salt, 1(4(4'-vinylbenzyloxy)butane)-2'(trimethylammonium)ethyl phosphate salt, and [3-(methacryloylamino)propyl]dimethyl(3-sulfopropyl) ammonium hydroxide salt. Amphoteric monomers are ionogenic monomers which contain both acidic and basic groups.

Group D monomers may also include nonionic monomers with the following structure, having an optional polyethyleneglycol (PEG) component:

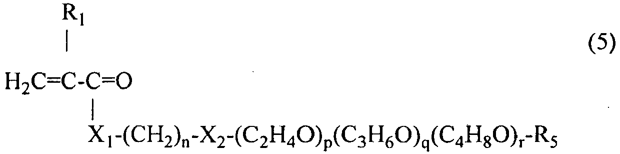

X₁ and X₂ are independently either O, S or NH; R₁ is either H or CH₃; R₅ is either H or linear or branched alkyl with 1 to 4 carbon atoms; n is between 0-6 (and X₂ is omitted when n=0); p is between 0-50, q is between 0-50, and r is between 0-50. Repeating units of p, q and r may be incorporated in random, alternating, block or gradient structures.

Examples of Group D monomers having the structure of (5) include at least one member selected from the group consisting of hydroxyethyl [meth]acrylate, hydroxypropyl [meth]acrylate, hydroxybutyl [meth]acrylate, hydroxyhexyl [meth]acrylate, hydroxyethyl [meth]acrylamide, hydroxypropyl [meth]acrylamide, hydroxybutyl [meth]acrylamide, hydroxyhexyl [meth]acrylamide and corresponding [meth]acrylamides including hydroxyethyl [meth]acrylamide, hydroxypropyl [meth]acrylamide, hydroxybutyl [meth]acrylamide, hydroxyhexyl [meth]acrylamide, poly(oxyethyl)₁₀ methacrylate, and methyl poly(oxyethyl)₈ acrylate.

Other exemplary nonionic monomers within group D include at least one of vinyl alcohol (from vinyl acetate), vinylpyrolidinone, and N-vinylformamide.

Monomers of Group E are broadly any other olefinically unsaturated monomers that do not fall into groups A through D. Exemplary monomers primarily include at least one member selected from the group consisting of mono-unsaturated monomers such as C1-C4 alkyl [meth]acrylates, C1-C4 alkyl fluoro [meth]acrylates, vinyl esters, alkylvinyl ethers, vinyl amides, styrene, and p-alkyl styrenes. However, multi-unsaturated monomers are not precluded.

In some embodiments if desired, group E may be used to provide cross-linking. Exemplary crosslinking monomers include at least one member selected from the group consisting of [meth]acrylic esters as well as allyl and vinyl ethers of di or multifunctional alcohols, such as 1,2-ethanediol, 1,2-propanediol, 1,3-propanediol, 1,2-butanediol, 1,3-butanediol, 2,3-butanediol, 1,4-butanediol, but-2-ene-1,4-diol, 1,2-pentanediol, 1,5-pentanediol, 1,2-hexanediol, 1,6-hexanediol,1,10-decanediol, 1,2-dodecanediol, 1,12-dodecanediol, neopentylglycol, 3-methylpentane-1,5-diol, 2,5-dimethyl-1,3-hexanediol, 2,2,4-trimethyl-1,3-pentanediol, 1,2-cyclohexanediol, 1,4-cyclohexanediol, 1,4-bis(hydroxymethyl)cyclohexane, diethyleneglycol, triethyleneglycol, tetraethyleneglycol, dipropyleneglycol, tripropyleneglycol, tetrapropyleneglycol, polyethyleneglycols, polypropyleneglycols, polytetrahydrofurans, trimethylolpropane, glycerol, pentaerythritol, 1,2,5-pentanetriol, 1,2,6-hexanetriol, and sorbitol.

Further suitable crosslinkers can include at least one of urethane diacrylates; straight chain or branched, linear or cyclic, aliphatic or aromatic hydrocarbons with at least 2 double bonds such as divinyl benzene; [meth]acrylamides and N-allyl amines of difunctional amines such as 1,2-diaminoethane, 1,3-diaminopropane, 1,4-diaminobutane, 1,6-diaminohexane; triallyl amine and triallylammonium salts, such as triallylmethylammonium chloride or methylsulfate; N-vinyl compounds of urea derivatives such as N,N'-divinylethyleneurea or N,N'-divinylpropyleneurea; di, tri, or tetra functional sulfhydryl compounds such as ethyleneglycol dimercaptopropionate, glycerol trimercaptoacetate, and pentaerythritol tetramercaptopropionate; alkoxy silane functional mercaptans such as 3-mecaptopropyl trimethoxy silane; and alkoxy silane functional alkyl [meth]acrylates or alkyl [meth]acrylamides such as methacryloxypropyl triethoxy silane and methacrylamidopropyl trimethoxy silane.

### Description of properties/attributes of copolymer

The total weight percent of ionic and ionogenic monomers in the cationic/cationogeniccomb copolymers described herein is in the range of about 2 to about 45%. In some embodiments, the range is between about 5 and about 35%, and in other embodiments is between about 10 and about 25%.

The weight percent of ionic monomers in the cationic/cationogenic comb copolymers described herein can be in the range of about 2 to about 45%, in some embodiments is between about 5 and about 30%, in other embodiments is between about 5 to about 25%, and in still other embodiments is between about 12 and about 22%.

The weight percent of associative moieties, Z, within the copolymer can be in the range of about 2 to about 50%, in some embodiments is between about 4 to about 40%, in other embodiments is between about 7 and about 30%, and in still other embodiments is between about 20 and about 30%.

Without wishing to be bound by any theory or explanation, it is believed that the weight ratio of associative moieties Z to the ionic and/or ionogenic monomers in the copolymer is at least partially responsible for the superior results obtained. That is, according to certain exemplary embodiments, the cationic and/or cationogenic monomers of group A, along with any other ionic monomers incorporated from groups C through E, have a relationship to the amount of associative groups Z present in the copolymer.

The ratio (wt%:wt%) of associative moieties Z to ionic and/or ionogenic monomers within the copolymer can be in the range of about 0.1 to about 5.5. In some embodiments the ratio is between about 0.25 and about 4, in another case is between about 0.4 and about 3, and in yet other cases is between about 0.7 and about 2.5. In still another embodiment the weight ratio of associative moieties Z to ionic and/or inogenic monomers within the copolymer is about 1.2 to about 2.2. The ratio (wt%:wt%) of associative moieties Z to ionic monomers within the copolymer can be in the range of about 0.15 to about 6.5. In some cases, the ratio is between about 0.3 and about 4.5, in another case the ratio is between about 0.4 and about 3.5, and in yet other embodiments is between about 0.7 and about 2.5. In still another embodiment the weight ratio of associative moieties Z to ionic monomers within the copolymer is about 1.2 to about 2.2.

The cationic/cationogenic comb copolymers may be crystalline or non-crystalline. In crystalline embodiments where the copolymer exhibits a melting temperature Tₘ with a heat of fusion greater than about 3 J/g, the melting temperature of the copolymers is typically less than about 100 °C, in some cases is less than about 50 °C, in many cases is less than about 25 °C, in other cases is less than about 15 °C. and in still other cases is less than about 0 °C.

Despite its ability to thicken personal care products containing additional ingredients, in certain exemplary embodiments, a three percent (solids) weight solution of the cationic/cationogenic comb copolymer described herein in water has a viscosity at 25 °C of less than about 0.25 Pa·s (250 cP) and in some embodiments has a viscosity at 25 °C of less than about 0.1 Pa·s (100 cP). In still other embodiments, a three percent (solids) weight solution of the cationic/cationogenic comb copolymer has a viscosity at 25 °C of less than about 0.05 Pa·s (50 cP). Thus, unlike the prior art, certain exemplary embodiments do not have to rely on thickening the water phase of personal care formulations as is common with currently-used high molecular weight and frequently cross-linked polymers, while substantial sensory and deposition benefits can be achieved over prior art cationic polymer additives.

The polymer chemistry can be tailored for compatibility and solubility in various aqueous cosmetic formulations by adjusting the influence of hydroxyl, amide and acid secondary monomers. The nitrogen content in the amide monomer, for example, can enhance deposition when lower cationic levels are desirable and contributes to both compatibility and viscosity. Limited amounts of acid monomer may reduce stickiness and can improve potential formulation compatibility. Some hydroxyl monomer content can improve polymer and formulation clarity in aqueous or alcoholic systems.

In one embodiment, cationic/cationogenic comb copolymers exhibit a range of solubility in water. In some embodiments the solubility in water at 25 °C is relatively low, for example, a water solubility of less than about 50 wt%, in other embodiments is less than about 20 wt%, and in still other embodiments is less than about 10 wt%. By "water solubility" or "water soluble" it is meant measuring the solubility of the copolymer in water as the concentration at which the turbidity of the aqueous solution first measures 250 EBC (European Brewery Convention Standard) units, with turbidity of the aqueous solution less than 250 EBC at lower concentrations.

Advantageously, certain exemplary embodiments may also include, but unlike the prior art do not require, crosslinking of the copolymer and/or the presence of sulfates which can lead to undesirable coloror odor. By "crosslinking" or "crosslinked", it is meant, the joining of adjacent chains of the copolymer by covalent bonds created through the purposeful introduction of a polyfunctional monomer. The covalent bonds that result in crosslinking may be formed through any reaction including condensation and addition reactions.

In one embodiment the cationic/cationogenic comb copolymer is comprised of about 12 to about 22 wt% of monomers of Group A, is comprised of about 25 to about 75 wt% of a monomer of Structure 2 wherein X₁=O, n=q=r=0, p is between 3 and 30 and Z is an alkyl group having in the range of 10 to 18 carbon atoms, has between about 7 and about 30 wt% of associative moieties Z, is not crosslinked, and exhibits a solution viscosity in water at three weight percent (solids) of less than about 100 cP when measured at 25 °C. In one aspect of this embodiment the amount of the monomer of Structure 2 is greater than 25wt.% and typically greater than about 30wt.% (e.g., about 30wt.% to about 75wt%).

In another embodiment the cationic/cationogeniccomb copolymer is comprised of about 12 to about 22 wt% of monomers of Group A, is comprised of about 25 to about 75 wt% of a monomer of Structure 2 wherein X₁=O, n=q=r=0, p is between 3 and 30 and Z is an alkyl group having in the range of 10 to 18 carbon atoms, is comprised of an acrylamide monomer of Group C, has between about 7 and about 30 wt% of associative moieties Z, is not crosslinked, and exhibits a solution viscosity in water at three weight percent (solids) of less than about 0.1 Pa·s (100 cP) when measured at 25 °C. In one aspect of this embodiment, wherein A was 15wt%, monomer of Structure 2 has p=4 and comprises 70wt.% of the copolymer and the Group C monomer is N,N-dimethylacrylamide, the water solubility was determined to be about 8wt.% (e.g., Example 72). In one aspect of this embodiment the amount of the monomer of Structure 2 is greater than 25wt.%, in some cases greater than about 30wt.% and typically greater than about 50wt.% (e.g., about 50wt% to about 75wt.%).

In yet another embodiment the cationic/cationogenic comb copolymer is comprised of about 12 to about 22 wt% of monomers of Group A, is comprised of about 25 to about 75 wt% of a monomer of Structure 2 wherein X₁=O, n=q=r=0, p is. between 3 and 30 and Z is an alkyl group having in the range of 10 to 18 carbon atoms, is comprised of a hydroxyalkyl [meth]acrylate monomer of Group D, has between about 7 and about 30 wt% of associative moieties Z, is not crosslinked, and exhibits a solution viscosity in water at three weight percent (solids) of less than about 100 cP when measured at 25 °C. In one aspect of this embodiment, wherein A was 15wt.%, monomer of Structure 2 has p=4 to 25 and comprises 70wt.% of the copolymer and the Group D monomer is hydroxypropyl methyacrylate, the water solubility was determined to be about 8wt.% (e.g., Example 71). In one aspect of this embodiment the amount of the monomer of Structure 2 is greater than 25wt.%, in some cases greater than about 30wt.% and typically greater than about 50wt.% (e.g., about 50wt.% to about 75wt.%).

The cationic/cationogenic comb copolymer can be manufactured according to any suitable process that results in the monomers being incorporated into the desired chain structure. One particularly suitable method of copolymer synthesis is solution polymerization in alcohol, glycol, or aqueous alcoholic mixture in the absence of any cross-linking components. The use of solution polymerization typically leads to the repeating units of the copolymer being incorporated randomly, though block, alternating or graft arrangements of repeating units are also possible.

In some embodiments, the copolymer is formulated into a personal care composition or product also including a cosmetically acceptable base material along with at least one active ingredient The use of cationic/cationogeniccomb copolymer compositions describe herein can increase the deposition of the active ingredient on keratinous substrates over a like formulation in which the copolymer is not used. The deposition of the active ingredient within a composition or product is determined by application of about 1 to about 4 mg/cm² of the composition or product to a keratinous substrate such as in-vitro synthetic skin, followed by drying of the substrate for 5 to 30 minutes, then immersion of the substrate into a petri-dish with 30 mL of deionized water under agitation with a smooth magnetic stirrer set at a speed of 300 rpm and maintained at a constant temperature between room temperature (22 °C ± 2) and body temperature (37 °C ± 2) for a test period of 30 seconds to 20 minutes. Following immersion, the deposition of the active ingredient on the substrate is determined by any suitable analytical method for quantifying the active ingredient(e.g., HPLC, energy dispersive x-ray spectrometry analysis, among other suitable methods). In cases where the active ingredient has a water solubility less than 50 g/L, the deposition is increased by about 10% or more, in some cases by about 20% or more, and in other cases the deposition is increased by about 50% or more. In embodiments in which the active ingredient has a water solubility greater than 50 g/L, the use of the copolymers can increase deposition by about 10% or more, in some cases by about 20% or more, and in other cases by about 200% or more. In some cases, the deposition of the active ingredient may be increased by about 500% or more. In some embodiments the cationic/cationogenic comb copolymer compositions described herein can increase the deposition of both an active ingredient with water solubility less than about 50 g/L and an active ingredient with water solubility greater than about 50 g/L by percentages indicated above.

In still other embodiments, the use of the cationic/cationogenic comb copolymers described herein can itself demonstrate deposition to keratinous substrates.

### Personal care products and exemplary formulations using copolymer

Thus, the cationic/cationogenic comb copolymer compositions disclosed herein may be employed as an ingredient in creating a personal care product that provides increased effectiveness in the deposition and retention of actives, has superior sensory attributes, and/or as a thickener. The cationic/cationogenic comb copolymer composition is generally present as about 0.1 to about 20% by weight in such products, in some cases between about 0.5 and about 10% by weight, and in still other cases is present between about 1% and about 5% by weight. The composition can be used with a wide range of personal care products having a base media that can comprise at least one member selected from the group consisting of cosmetic oil (i.e. oils compatible for cosmetic uses), water, alcohol and combinations thereof, all by way of example only.

The cationic/cationogenic comb copolymers can be added to personal care products as pure copolymer, or as a solution or suspension in water or any suitable organic solvent or combination of solvents and/or water. Alcohol and glycol are particularly suitable organic solvents. While any suitable glycol can be employed, examples of suitable glycols comprise at least one of propylene glycol, butylene glycol, and glycerol. For example, the cationic/cationogenic comb polymers can be provided as a solution in butylene glycol and water. The amount of glycol can range from about 10 to about 70wt.% of the solution, in some cases about 20 to about 60wt.% and in other cases about 30 to about 50wt.% of the solution.

Exemplary cosmetic, toiletry and topical health care type personal care products in which the cationic/cationogeniccomb copolymers may be employed include, without limitation, at least one of shampoos; chemical and non-chemical hair curling and hair straightening products; hair style maintaining and hair conditioning products; lotions and creams for nails, hands, feet, face, scalp and/or body, hair dye; face and body makeup; nail care products; astringents; deodorants; antiperspirants; depilatories; skin protective creams and lotions (such as sunscreens); skin and body cleansers; skin conditioners; skin toners; skin firming compositions; liquid soaps; bar soaps; bath products; shaving products; and oral hygiene products (such as toothpastes, oral suspensions, and mouth care products), any or the foregoing of which may additionally contain pharmaceutical, phytopharmaceutical and/or neutraceutical ingredients.

The form of such personal care products is not limited and may be, without limitation, a liquid, gel, spray, emulsion (such as lotions and creams), shampoo, pomade, foam, ointment, tablet, stick (such as lip care products), makeup, suppositories, among others, any of which can be applied to the skin or hair and which typically are designed to remain in contact therewith until removed, such as by rinsing with water or washing with shampoo or soap. Gels can be soft, stiff, or squeezable. Emulsions can be oil-in-water, water-in-oil, water-and-silicone, or multiphase. Sprays can be non-pressurized aerosols delivered from manually pumped finger-actuated sprayers or can be pressurized aerosols. In some embodiments, exemplary cationic/cationogenic comb copolymers are formulated in aerosol compositions such as mousse, spray, or foam forming formulation, where a chemical or gaseous propellant is used.

The foregoing personal care products in which cationic/cationogenic comb copolymers in accordance with exemplary embodiments advantageously may be used generally, but not necessarily, can be broadly categorized as anhydrous oil-base products, water-in-oil emulsions, oil-in-water emulsions, or aqueous or alcohol based systems.

The cationic/cationogenic comb copolymer composition can be effective with a broad range of cosmetic oils, such as at least one of esters (e.g., alkyl benzoates having between 12 to 15 carbons), triglycerides (e.g., Caprylic/Caprylate triglyceride), hydrocarbons (e.g., mineral oil, sunflower oil), natural oils (e.g., jojoba oil, safflower oil), and castor oil, among others. Suitable oils are also disclosed, for example, at column 3, line 37, to column 4, line 4, of U.S. Patent No. 5,736,125. Silicone oils may also be used as cosmetic oils. In general, any natural or synthetic oil for cosmetic use is suitable for the composition of present invention. Non-limiting examples of natural oils are at least one of avocado oil, coconut oil, palm oil, sesame oil, peanut oil, sunflower oil, almond oil, peach kernel oil, wheat germ oil, macadamia nut oil, night primrose oil, jojoba oil, castor oil, olive oil, soya oil and the derivatives thereof. Mineral oil such as paraffin oil and petrolatum are also suitable.

Suitable synthetic oils are at least one member selected from the group consisting of fatty alcohols; fatty acid esters such as isopropyl myristate, palmitate, stearate and isostearate; oleyl oleate; isocetyl stearate; hexyl laurate; dibutyl adipate; dioctyl adiphate; myristyl myristate; oleyl erucate; polyethylene glycol and it derivatives; polyglyceryl fatty acid esters; and cetyl palmitate, by way of example only.

Silicone oils are also suitable. Useful silicone oils are non-volatile silicone oils known by INCl names that include dimethicone or dimethiconol. Volatile silicone oils such as cyclomethicones may also be used.

The following are non-limiting examples of anhydrous cosmetic formulations containing modified oils incorporating cationic/cationogeniccomb copolymer compositions as described herein:
A) Thickened Anhydrous Oils suitable for personal care applications (e.g. hair gels):
   Oils - about 50 to about 95 wt%
   Cationic/cationogenic comb copolymer - about 0.1 to about 20 wt% Complementary polymer - up to about 5 wt%
   Other Additives or Actives - about 0.1 to about 30 wt%
B) Anhydrous Scalp Serum:
   Oils - about 50 to about 95 wt%
   Cationic/cationogenic comb copolymer - about 0.1 to about 20 wt%
   Complementary polymer - up to about 5 wt%
   Other Additives or Actives - about 0.1 to about 30 wt%
C) Anhydrous Sunscreen Stick or Gel:
   Oils - about 50 to about 95 wt%
   Cationic/cationogenic comb copolymer - about 0.1 to about 20 wt% Complementary polymer- up to about 5 wt%
   Other Additives or Actives - about 0.1 to about 30 wt%
D) Anhydrous Antiperspirant Deodorant Stick or Gel:
   Emollient- about 50 to about 95 wt%
   Cationic/cationogenic comb copolymer - about 0.1 to about 20 wt% Complementary polymer- up to about 5 wt%
   Antiperspirant deodorant (APDO) actives - about 0.1 to about 30 wt% Other Additives or Actives - about 1 to about 30 wt%
E) Color Cosmetic (e.g. blush, lipstick)
   Oil - about 50 to about 95 wt%
   Cationic/cationogenic comb copolymer - about 0.1 to about 20 wt%
   Complementary polymer - up to about 5 wt%
   Pigment- about 0.1 to about 30 wt%
   Other Additives or Actives - about 0.1 to about 10 wt %

Conventional thickeners such as waxes comprising at least one of carnauba wax, bees wax, and Candelilla wax, among others, may be employed as an additive to supplement the thickening effect of the formulations achieved by the cationic/cationogenic comb copolymer.

Water-in-oil emulsions can be prepared by mixing together (1) a heated (i.e., melted) solution of the cationic/cationogenic comb copolymer composition in any of the previously discussed oils and (2) an aqueous phase, the aqueous phase being at a temperature similar to the oil solution (typically within about 10°C); and then cooling the mixture while stirring. Alternatively, the cationic/cationogenic comb copolymer composition could instead be initially added to the aqueous phase, or it could be added after the oil and aqueous phase has been emulsified. Regardless of the manner in which the cationic/cationogenic comb copolymer composition is added, the ratio of the aqueous phaseto the oil phase can be, for example, about 0.5:1 to about 9:1.

The following are non-limiting examples of cosmetic formulations comprising water-in-oil emulsions:
A) Skin Moisturizer
   Water - about 50 to about 90 wt%
   Silicone- about 1 to about 10 wt%
   Emulsifier - about 0.5 to about 5 wt%
   Emollient- about 5 to about 20 wt%
   Cationic/cationogeniccomb copolymer- about 0.1 to about 20 wt%
   Complementary polymer- up to about 5 wt%
   Other Additives orActives - about 0.1 to about 3 wt%
B) Sunscreen
   Water - about 50 to about 90 wt%
   Silicone- about 1 to about 10 wt%
   Emulsifier - about 0.5 to about 5 wt%
   Emollient- about 5 to about 20 wt%
   Cationic/cationogenic comb copolymer- about 0.1 to about 20 wt%
   Complementary polymer - up to about 5 wt%
   Sunscreen Active - about 1 to about 25 wt%
   Other Additives orActives - about 0.1 to about 3 wt%
C) Antiperspirant Deodorant
Water - about 50 to about 90 wt%
Silicone- about 1 to about 10 wt%
Emulsifier - about 0.5 to about 5 wt%
Emollient- about 1 to about 20 wt%
Cationic/cationogenic comb copolymer- about 0.1 to about 20 wt%
Complementary polymer- up to about 5 wt%
APDO actives - about 0.1 to about 30 wt%
Other Additives orActives - about 0.1 to about 5 wt%

Oil-in-water emulsions are prepared by mixing together (1) a heated (i.e., melted) solution of the cationic/cationogenic comb copolymer composition in the oil phase and (2) an aqueous phase, the aqueous phase beingat a temperature similar to the emollient solution (typically within about 10°C); and then cooling the mixture while stirring. However, as with the water-in-oil emulsions, the cationic/cationogenic comb copolymer composition may initially be added to the aqueous phaseor added post-emulsification. The ratio of the oil phase to the water phase can be, for example, about 0.1:1 to about 1:1. The following are non-limiting examples of cosmetic formulations comprising oil-in-water emulsions:
A) Skin Moisturizer
   Water - about 50 to about 90 wt%
   Emulsifier - about 0.5 to about 5 wt%
   Emollient- about 1 to about 20 wt%
   Cationic/cationogenic comb copolymer- about 0.1 to about 20 wt%
   Complementary polymer- up to about 5 wt%
   Other Additives or Actives - about 0.1 to about 3 wt%
B) Sunscreen
   Water - about 50 to about 90 wt%
   Emulsifier - about 0.5 to about 5 wt%
   Emollient- about 1 to about 20 wt%
   Cationic/cationogenic comb copolymer- about 0.1 to about 20 wt%
   Complementary polymer- up to about 5 wt%
   Sunscreen Active - about 1 to about 25 wt%
   Other Additives or Actives - about 0.1 to about 3 wt%
C) Mousse or other hair styling product
   Water - about 50 to about 90 wt%
   Emulsifier - about 0.5 to about 1 wt%
   Surfactant - about 0.1 to about 2 wt%
   Cationic/cationogenic comb copolymer- about 0.1 to about 20 wt%
   Complementary polymer - up to about 5 wt%
   Other Additives or Actives - about 0.1 to about 2 wt%
   Solvent- about 1 to about 25 wt%
   Propellant- up to about 10 wt%

The following are non-limiting examples of cosmetic formulations comprising alcohol or aqueous systems.
A) Coloring Shampoo
   Water - about 50 to about 90 wt %
   Surfactant - about 2 to about 20 wt%
   Foam booster-up to about 20 wt%
   Cationic/cationogenic comb copolymer- about 0.1 to about 20 wt%
   Complementary polymer- up to about 5 wt%
   Other Additives or Actives - about 0.1 to about 10 wt%
B) Hair Spray (aerosol and non-aerosol)
   Water - about 10 to about 90 wt%
   Cationic/cationogenic comb copolymer- about 0.1 to about 20 wt%
   Complementary polymer- up to about 5 wt%
   Ethanol or other solvents - about 33 to about 90 wt%
   Optional Propellant for an aerosol - about 0 to about 50 wt%
   Other Additives orActives - about 0.1 to about 2 wt%
C) Shampoo
   Water - about 50 to about 90 wt%
   Surfactant- up to about 20 wt%
   Foam booster - about 2 to about 20 wt%
   Cationic/cationogenic comb copolymer- about 0.1 to about 20 wt%
   Complementary polymer- up to about 5 wt%
   Other Additives or Actives - about 0.1 to about 10 wt%
D) Hair Styling products
   Water - about 10 to about 90 wt%
   Cationic/cationogenic comb copolymer- about 0.1 to about 20 wt%
   Complementary polymer- up to about 5 wt%
   Ethanol or other solvents- about 0 to about 10 wt%
   Other Additives orActives - about 0.1 to about 10 wt%
E) Body cleansing products
Water - about 50 to about 90 wt%
Surfactant - about 2 to about 20 wt%
Cationic/cationogenic comb copolymer- about 0.1 to about 20 wt%
Complementary polymer- up to about 5 wt%
Foam booster - up to about 20 wt%
Other Additives or Actives - about 0.1 to about 10 wt%.

In products in which emollients are employed, any suitable emollient for use in cosmetic compositions can be used. Examples of suitable emollients include at least one of esters (e.g., C12-15 alkyl benzoate) and triglycerides (e.g., Caprylic/caprylate triglyceride); hydrocarbon oils (e.g., mineral oil), natural oil (e.g., Jojoba oil, safflower oil), tridecyl trimellitate, sunflower oil, castor oil, among other compounds used to impart desired or improved sensory or aesthetic properties of a personal care composition.

In products in which emulsifiers are employed, any suitable cosmetic emulsifier having a hydrophilic-lipophilic balance (HLB) in the range of about 1 to about 20 can be used. The emulsifier can be nonionic, cationic, anionic, or amphoteric or a combination of such emulsifiers can be used.

Examples of nonionic emulsifiers are at least one of laureths, e.g. laureth-4; ceteths, e.g. ceteths-1; polyethylene glycol cetyl ether; ceteareths, e.g. ceteareth-25; polyglycol fatty acid glycerides; hydroxylated lecithin; lactyl esters of fatty acids; and alkyl polyglycosides.

Examples of cationic emulsifiers are at least one of cetyldimethyl-2-hydroxyethylammonium dihydrogenphosphate;cetyltrimonium chloride; cetyltrimonium bromide; cocotrimonium methosulfate; as well as emulsifiers that contain a quaternized nitrogen.

Anionic emulsifiers include, for example, at least one of alkyl sulfates; alkyl ether sulfates; alkylsulfonates; alkylarylsulfonates; alkyl succinates; alkyl sulfosuccinates; N-alkylsarcosinates; acyl taurates; acyl isethionates; alkyl phosphates; alkyl ether phosphates; alkyl ether carboxylates; alpha-olefinsulfonates, and the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Surfactants and/or foam boosters may also be employed and like the emulsifiers can be nonionic, cationic, anionic, or amphoteric or a combination of such surfactants can be used.

Suitable anionic surfactants are for example, at least one of alkyl sulfates; alkyl ether sulfates; alkylsulfonates; alkylarylsulfonates; alkyl succinates; N-alkylsarcosinates; acyl taurates; acyl isethionates; alkyl phosphates; alkyl ether phosphates; alkyl ether carboxylates; alpha olefinsulfonates, and may include the alkali metal and alkaline earth metal salts of such materials (e.g. sodium, potassium, magnesium, calcium) as well as ammonium, trialkylamine, trialkanol amine, and alkylalkanol amine salts. The alkyl ether sulfates, alkyl ether phosphates and alkyl ether carboxylates may include ethylene oxide or propylene oxide units.

Suitable amphoteric surfactants are, for example, at least one of alkylbetaines; alkylamidopropylbetaines; alkylsulfobetaines; alkyl glycinates; alkylcarboxyglycinates; alkyl amphoacetates or amphopropionates; and alkyl amphodiacetates or amphodipropionates. For example, it is possible to use cocodimethylsulfopropylbetaine, laurylbetaine, and cocamidopropylbetaine or sodium cocamphopropionate as surfactants.

Examples of nonionic surfactants are the reaction products of aliphatic alcohols having between 6 and 20 carbon atoms in the alkyl chains, which can be linear or branched with ethylene oxide and/or propylene oxide. Also suitable are at least one of alkylamine oxides; mono- or dialkylakanolamides; fatty acid esters of polyethylene glycols; alkyl polyglycosides and sorbitan ether esters.

Examples of cationic surfactant are quaternary ammonium compounds, for example, cetyltrimethylammonium chloride, as well as other surfactants that contain a quaternized nitrogen.

In products in which propellant or solvents are employed, those may include at least one of isobutane, butane, dimethyl ether, and ethanol, among others.

Examples of other additive compounds include one or more members selected from the group consisting of silicone based plasticizers, natural or synthetic compounds (e.g., polysaccharides, natural or synthetic gums, stabilizers, anionic and nonionic associative thickener or rheology modifiers soluble in oil or water phase), among other compounds. The additives may include at least one compound selected from the group consisting of preservatives, stabilizers (e.g., Xanthan Gum), humectants (e.g., MP Diol, Sorbitol, and Hexylene Glycol), antioxidant (e.g., Vitamins), rheology modifiers, fragrances, and pigments, among other additives.

In some personal care products, active compounds that interact with or protect skin or hair can be included. Examples of such active compounds include at least one of sunscreen compounds (e.g. zinc oxide, titanium dioxide, octinoxate, octocrylene, ethylhexyl salicylate, oxybenzone); skin whiteners (e.g. salicylic acid); anti-cellulite compounds; anti-aging compounds (e.g., polypeptides such as Argininie/Lysine, Argininie PCA, Aspergillus/Aspidosperma Quebracho Ferment, Avena Sativa (Oat) Kernel Protein, and Avocado Sterols, proteins, peptides, copper peptides, fermented biopolymers, beta-glucan, botanical actives, Bifida Ferment Lysate, Calophylum Inorhylum seed oil, camellia sinensis extract, ceramides, chlorella vulgaris extract, coriolus versicolor extract, corylus avellana (hazel) seed extract, Hyaluronic acid, erythorbic acid, hydrolyzed elastins, hydrolyzed proteins, hydrolyzed soy flour, hydrolyzed peptides, and Vitamins A, E, C, K, and B5 as wel as Niacinamide); anti-dandruff compounds (e.g., zinc pyrithione); APDO compounds (e.g., aluminum chlorohydrate, aluminum zirconium tetra chlorohydrex); vitamins (e.g., Tocopherol natural, synthetic Tocopherol, synthetic tocopheryl acetate, Retinol, Retinyl palmitate, Retinyl acetate, Provitamin B-5, ascorbic acid, sodium ascorbyl phosphate, Ascorbyl glucoside, Magnesium ascorbyl phosphate); Polysaccharides (e.g., Hyaluronic acid, B-1,3-glucans, Chitosan); Botanicals (e.g., Aloe vera, Green tea extract, Grape seed extract, Isoflavones, Chamomille/bisabolol, Fennel, Ginko, Ginseng, Guava); Alpha Hydroxy Acids (e.g., Citric acid, Glycolic acid, Lactic acid); Sugar cane extracts; insect repellents; and Coenzymes and Enzymes (e.g., Ubiquinone, Coenzyme Q10), all by way of example only. It will be appreciated that certain active compounds may fall into more than one category and/or be used to accomplish more than one result.

For purposes of this invention, silicone oils and additives are considered active ingredients. Non-limiting examples of silicone oils or additives that may be incorporated in personal care products are known by INCI names that include dimethicone, dimethiconol, siloxane and cyclomethicones. In certain embodiments, the inventive cationic comb copolymer can improve deposition of actives including silicone oils.

### Discussion of optional "other polymer" in conjunction with cationic comb copolymer in personal care formulations

Personal care products may employ the cationic/cationogenic comb copolymer compositions alone as a deposition, sensory and/or thickening agent, while in other embodiments, the compositions may optionally employ up to about 5% by weight of one or more additional, complementary polymers. The complementary polymer(s) can be any type of polymer, including those which are at least one of nonionic, amphoteric or zwitterionic, anionic, cationic or mixtures of such types of polymers.

Exemplary synthetic, nonionic complementary polymers include at least one of vinyl pyrrolidone homopolymer and copolymers, including those which have a vinyl acetate group, such as, for example, those under the trade name "Luviskol" including the homopolymers Luviskol® K30, K60, K90 as well the copolymers Luviskol® VA 55 and VA 64 Plus, all available from BASF AG, in addition to Advantage® LS-E from ISP, all by way of example only. Natural non-ionic polymers suitable for the composition of the present invention can comprise at least one of cellulose, starches, chitosan, xanthan gum, guar gum, neutralized shellac and their derivatives.

Exemplary amphoteric polymers that may be incorporated into the personal care compositions with the cationic/cationogenic comb copolymers include at least one polymer and copolymer that are derived from acrylamides, [meth]acrylic acid, and tert-butyl amino ethylmethacrylate, such as the octylacrylamide/acrylate/butylaminoethyl [meth]acrylate copolymer available under the tradename Amphomer®; methacryloyl ethyl betaine and alkyl [meth]acrylate copolymer, such as those commercially available under the tradename Yukaformer, including Yukaformer® AM75; copolymers derived from monomers containing carboxyl groups and/orsulfonic groups (e.g. [meth]acrylic acid and itaconic acid) copolymerized with monomers such as mono or dialkylaminoalkyl[meth]acrylates or mono or diallylaminoalkyl[meth]acrylamides; as well as copolymers derived from N-octyl acrylamide, methyl [meth]acrylate, hydroxypropyl [meth]acrylate, n-tert-butylaminoethyl [meth]acrylate and/or acrylic acid, all by way of example only.

Suitable complementary anionic polymers include at least one of homopolymers and copolymers of [meth]acrylic acid or salts thereof; copolymers of [meth]acrylic acid and acrylamide or salts thereof; sodium salt of polyhydroxycarboxylic acids; water soluble or water dispersible polyester, polyurethanes (Luviset® P.U.R.) and polyureas; and copolymers of t-butyl acrylate, ethyl acrylate, methacrylic acid (e.g. Luvimer® 100P).

Other suitable complementary anionic polymers are at least one vinyl alkyl ether copolymer, such as methyl vinyl ether/maleic acid copolymer, obtained by hydrolysis of vinyl ether/maleic anhydride copolymer and available under the trade name "Gantrez® AN or ES". These polymers may also be partially esterified, as for example, "Gantrez® ES 225" or "ES 435." Ethyl, butyl and isobutyl esters of ethyl vinyl ether/maleic acid copolymer are also useful.

Further exemplary complementary anionic polymers include at least one of Balance® CR (acrylate copolymer), Balance® 47 (Octylacrylamide/acrylate/butylaminoethyl methacrylate copolymer), Balance® 0/55 (acrylate copolymer), Aquaflex® FX 64 (ISP; isobutylene/ethylmaleimide/hydroxyethylmaleimide copolymer), Aquaflex® SF-40 (ISP; vinylpyrollidone/vinylcaprolactam/dimethylaminopropylamine acrylate copolymer), Alliance® LT-120 (ISP/Rohm & Hass; acrylate/C1-2 succinate/hydroxyacrylate copolymer), Aquarez® HS (Eastman; polyester-1), Diaformer® Z-400 (Clariant; methacryloylethylbetaine/methacrylate copolymer), Diaformer® Z-712 or Z-711 (Clariant; methacryloylethyl N-oxide/methacrylate copolymer), Omnirez® 200 (ISP; monoethyl ester of poly(methyl vinylether/maleic acid), Amphomer® HC (acrylate/octylacrylamide copolymer) Amphomer® 28-4910 (octylacrylamide/acrylate/butylaminoethyl methacrylate copolymer), Advantage® HC 37 (ISP terpolymer of vinylcaprolactam/vinylpyrrolidone/dimethylaminoetyl methacrylate), Acudyne® 258(Rohm & Haas; acrylate/hydroxyl ester acrylate copolymer), Luviset® PUR (BASF, polyurethane-1), and Eastman® A48 (Eastman).

Still further useful anionic polymers are at least one vinyl acetate/crotonic acid or vinyl acetate/vinyl neodecanoate/crotonic acid copolymers available under the trade name "Resyn®"; sodium acrylate/vinyl alcohol copolymer available under the trade name "Hydagen® F"; sodium polystyrene sulfonate, e.g. "Flexan® 140"; ethyl acrylate/acrylic acid/N-tert-butyl acrylamide copolymers available under the trade name "Ultrahold®"; vinyl pyrrolidone/vinyl acetate/itaconic acid co-polymer; and acrylic acid/acrylamide copolymer or the sodium salts thereof available under the trade name "Reten®;" acrylate copolymer available under trade name Salcare® SC 81; PEG/PPG 25/25 dimethicone/acrylate copolymer available underthe trade name Luviflex Silk from BASF; acrylate/t-butylacrylamide copolymer available under the trade name Ultrahold Strong; vinyl caprolactam/PVP/dimethylaminoethylmethacrylate copolymer available as Advantage LC-E; and vinyl acetate/crotonates copolymer available undertrade name Luviset® C 66, all by way of example only.

Cationic polymers that may be used in personal care products in addition to the cationic/cationogenic comb copolymers described herein include the cationic cellulose type polymer available under the trade name JR from Amerchol®, such as polyquaternium 10 and cationic guar gums, such as guar hydroxypropyltrimonium chloride, including those available under the tradename Jaguar®. Furthermore, chitosan and chitin can also be included as cationic natural polymers, along with cationic derivatives of natural polymers such as starches, celluloses, and xanthan gum.

Other suitable cationic polymers include any of the polyquaternium polymers, such as polyquaternium 6, polyquaternium 7, polyquaternium 11, polyquaternium 16, polyquaternium 22, polyquaternium 24, polyquaternium 28, polyquaternium 30, polyquaternium 36, polyquaternium 37, polyquaternium 46, polyquaternium 67 and polyquaternium 72, and mixtures thereof, all by way of example only.

Other exemplary complementary cationic polymers include at least one of salts of vinylpyrrolidone/N-vinylimidazolium copolymer (e.g., Luviquat® FC, Luviquat HM, Luviquat MS, and Luviquat Care); N-vinylpyrrolidone/dinethylaminoethyl methacrylate copolymer quaternized with diethyl sulfate (e.g., Luviquat PQ-11); salts of N-vinylcaprolactum N-vinylpyrrolidone/N-vinylimidazolium copolymer (e.g., Luviquat Hold); cationic cellulose derivatives (e.g., polyquaternium-4 and -10); and acrylamide and dimethyldiallylammonium chloride copolymer (e.g., polyquaternium-7).

The complementary polymer may also include silicone compounds, such as, for example, at least one of polydiorganosiloxanes, polydialkylsiloxanes, polyalkylsiloxanes, polyarylsiloxane, silicone resins, silicone gums or dimethicone copolyols and amino-functional silicone compounds such as amodimethicone. Other silicone compounds include graft polymers of organosiloxane and polyethyloxazolines compounds known with the INCI name Polysilicone-9. Any polymeric compound having an INCl name including silicone, methicone, dimethicone, or siloxane as part of its name may be used.

### Experimental examples of created cationic comb polymers

### EXAMPLES

The invention is further described by way of the following examples, which are presented by way of illustration, not of limitation of the claims attached hereto.

In the following examples, various combinations of up to eight out of a total of fourteen acrylic monomers (designated below as M1 to M14) selected from both hydrophilic and hydrophobic esters, amides, alcohols, acids and cations were used in forming cationic/cationogenic comb copolymer compositions in accordance with exemplary embodiments.

In the examples, the composition M1 refers to the monomer BX-CSEM-25/80, a mixture of approximately 75% wt. cetyl/stearyl polyethoxy (25) methacrylate, about 5% wt. methacrylic acid and about 20% wt. water, commercially available from Bimax, Inc. M1 was used to provide group B repeating units.

M2 is a group E monomer and refers to n-Butyl acrylate, which was obtained from various commercial sources at 99% purity.

M3 refers to the monomer CD9075, a mixed commercial grade of approximately 98% wt. lauryl polyethoxy (4) acrylate with about 2% wt. lauryl alcohol and ethoxylates of lauryl alcohol availablefrom Sartomer Co; M3 was used to provide group B repeating units.

M4 refers to the monomer diacetone acrylamide, commercially obtained at 98% purity from Kyowa Hakko Chemical Co. M4 was used to provide group C repeating units.

M5 refers to the monomer dimethyl acrylamide, commercially obtained at 98% purity from Kowa American Corp. M5 was used to provide group C repeating units.

M6 refers to the monomer HPMA 97, a 97-98% purity mixture of approximately 75% wt. hydroxypropyl and 25% wt. hydroxyisopropyl methacrylates commercially available from Evonik Industries. M6 was used to provide group D repeating units.

M7 refers to the monomer methacrylic acid, obtained from various commercial sources at 99+% purity. M7 was used to provide group D repeating units.

M8 refers to the monomer Ageflex FA1 Q80MC*500, an 80% wt. solution of 2-acryloyloxyethyl trimethylammonium chloride in water, commercially available from Ciba Specialty Chemicals. M8 was used to provide groupA repeating units.

M9 refers to the monomer BX-LEM-23/100, a mixture of approximately 94% wt. lauryl polyethoxy (23) methacrylate, 5% wt. methacrylic acid and 1% wt. water, commercially available from Bimax, Inc. M9 was used to provide group B repeating units.

M10 refers to the monomer methyl acrylate, obtained from various commercial sources at 99+% purity. M10 was used to provide group E repeating units.

M11 refers to the monomer [2-(Methacryloyloxy)ethyl]trimethylammonium methyl sulfate solution, 80% wt. in water, commercially available from Sigma-Aldrich. M11 was used to provide group A repeating units.

M12 refers to the monomer n-butyl methacrylate, obtained from various commercial sources at 99+% purity. M12 was used to provide group E repeating units.

M13 refers to the monomer Mhoromer BM613, a 50% wt. solution of trimethylaminopropyl methacrylamide chloride in water, commercially available from Evonik Industries. M13 was used to provide group A repeating units.

M14 refers to the monomer methacrylamide, commercially obtained as 98% purity from Sigma-Aldrich. M14 was used to provide group C repeating units.

Unless otherwise indicated, the initiator in each case was 97% wt. 2,2'-azobis-2-methylpropionamidine dihydrochloride (also known as V50), commercially available from Wako Chemicals USA.

### Example 1

A monomer mixture was prepared by stirring together 121.0 grams M1, 96.3 grams M3, 41.25 grams M6 and 51.6 grams M8 with 220 grams 2-propanol (IPA).

The monomer mixture was warmed to 50 °C and 212 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.5 grams additional IPA were added to the remaining 318 grams of monomer mixture, sparged with nitrogen and poured into a 500 ml Pyrex addition funnel for delayed addition to the reactor with a FMI QG-50-1CKC metering pump. An initiator mixture, consisting of 1.1 grams V50 dissolved in 6.1 grams of deionized (DI) water and 6.1 grams IPA, was added to a 50 ml syringe barrel for delayed addition to the reactor with a FMI QG-6-0SSY metering pump.

At 65 °C, polymerization was initiated by adding a mixture of 1.0 gram V50 in 5.0 grams of DI water all at once to the stirred reactor. Within 1 minute, the contents of the reactor simultaneously began to lose transparency and rise in temperature. After 5 minutes, the contents had changed to a translucent white mixture and the temperature had risen to 70 °C. After 6 minutes, pumps were turned on for both delayed addition initiator and monomer mixtures. The monomer mixture had already been adjusted to deliver in 60 minutes and the initiator mixture had been adjusted to deliver in 90 minutes. The water bath temperature was increased stepwise to 80 °C during the first 30 minutes of delayed addition while the reactor temperature increased slowly to 73 °C. By the time all the monomer mixture had been delivered b the reactor, the temperature had increased to 81 °C and the contents were viscous and white in color. After all the initiator mixture had been delivered, the reactor was heated further and the process held at 85 °C for an additional 2 hours.

Nitrogen sparging was then started and a water aspirator attached to apply partial vacuum and remove volatile solvent with additional heat from the water bath to keep the reactor contents at 86-87 °C. After 15 minutes vacuum sparging, 150 grams of hot (70-80 °C) DI water was added which turned the reactor contents completely transparent. As 173 more grams of hot water were added, the contents turned translucent but not opaque. The viscosity of the polymer solution increased as solvent was removed while vacuum sparging was continued for another 3 hours. Finally, the polymer solution was cooled to room temperature and 3.0 grams of 10% aqueous hydrogen peroxide was added as preservative. The final appearance was a translucent viscous liquid.

### Example 2

A monomer mixture was prepared bystirring together 41.25 grams M5, 192.5 grams M3 and 51.6 grams M8 with 220 grams IPA.

The monomer mixture was warmed to 50 °C and 202 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.8 grams additional IPA were added to the remaining 303 grams of monomer mixture, sparged with nitrogen and poured into a 500 ml Pyrex addition funnel for delayed addition to the reactor with a FMI QG-50-1 CKC metering pump. An initiator mixture, consisting of 1.4 grams V50 dissolved in 8.0 grams of DI water and 7.7 grams IPA, was added to a 50 ml syringe barrel for delayed addition to the reactor with a FMI QG-6-0SSY metering pump.

At 65 °C, polymerization was initiated by adding a mixture of 1.4 gram V50 in 7.1 grams of DI water all at once to the stirred reactor. Within 1 minute, the contents of the reactor turned cloudy and rose very quickly in temperature to 74 °C. An additional 7.5 grams of IPA were added and the temperature subsequently decreased. After 4 minutes, pumps were turned on for both delayed addition initiator and monomer mixtures. The monomer mixture had been adjusted to deliver in 60 minutes and the initiator mixture had been adjusted to deliver in 90 minutes. The water bath temperature was increased stepwise to 75 °C during the delayed addition while the reactor temperature increased slowly back to 73 °C. After all the initiator mixture had been delivered, the reactor was heated further and the process held at 75 °C for an additional 2 hours.

Nitrogen sparging was then started and a water aspirator attached to apply partial vacuum and remove volatile solvent with additional heat from the water bath to keep the reactor contents at 82-87 °C. After 1 hour vacuum sparging, 324 grams of hot (70-80 °C) DI water was added to reduce viscosity enough to maintain circulation. Vacuum sparging was continued for another 2 hours. Finally, the polymer solution was cooled to room temperature and 3.0 grams of 10% aqueous hydrogen peroxide was added as preservative. The final appearance was an opaque liquid settling slowly to two transparent layers

### Example 3

A monomer mixture was prepared bystirring together 55.0 grams M4, 82.5 grams M5, 61.9 grams M3, 61.9 grams M2 and 17.2 grams M8 with 220 grams IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 62 °C by immersion in a 70 °C water bath. 27.5 grams additional IPA were added to the remaining 299 grams of monomer mixture, sparged with nitrogen and poured into a 500 ml Pyrex addition funnel for delayed addition to the reactor with a FMI QG-50-1CKC metering pump. An initiator mixture, consisting of 1.7 grams V50 dissolved in 9.1 grams of DI water and 9.1 grams IPA, was added to a 50 ml syringe barrel for delayed addition to the reactor with a FMI QG-6-0SSY metering pump.

At 62 °C, polymerization was initiated by adding a mixture of 1.6 gram V50 in 8.3 grams of DI water all at once to the stirred reactor. Within 1 minute, the contents of the reactor simultaneously began to lose transparency and rise in temperature. After 17 minutes, the contents had changed to a translucent white mixture and the temperature had risen to 80 °C. After 6 minutes, pumps were turned on for both delayed addition initiator and monomer mixtures. The monomer mixture had been adjusted to deliver in 60 minutes and the initiator mixture had been adjusted to deliver in 90 minutes. The water bath temperature was increased stepwise to 80 °C during the first 20 minutes of delayed addition during which time the reactor temperature first dropped and then leveled off near 72 °C. By the time all the monomer mixture had been delivered to the reactor, the temperature had increased to 81 °C and the contents were viscous and white in color. After all the initiator mixture had been delivered, thereactor was heated further and the process held at 85 °C for an additional 2 hours.

Nitrogen sparging was then started and a water aspirator attached to apply partial vacuum and remove volatile solvent with additional heat from the water bath to keep the reactor contents at 74-78 °C. After 10 minutes vacuum sparging, 250 grams of hot (70-80 °C) DI water was added which turned the reactor contents completely transparent. An additional 173 grams of hot water were added and the contents remained transparent. The viscosity of the polymer solution increased as solvent was removed while vacuum sparging was continued for another 3 hours. Finally, the polymer solution was cooled to room temperature and 3.0 grams of 10% aqueous hydrogen peroxide was added as preservative. The final appearance was a transparent viscous liquid.

### Example 4

A starting monomer mixture was prepared by stirring together 75.6 grams M3 and 55.0 grams M4 with 121 grams IPA.

The mixture was charged to a 1 L glass resin reactor fitted with a stainless steel stirrer and reflux condenser, and then sparged with dry nitrogen for 20 minutes while heating to 66 °C by immersion in a 71 °C water bath.

A second monomer mixture was prepared by stirring together 75.6 grams M3, 55.0 grams M4 and 8.6 grams M8 with 82.5 grams IPA. This was sparged with nitrogen, warmed to 50 °C and poured into a 500 ml Pyrex addition funnel for delayed addition to the reactor with a FMI QG-50-1 CKC metering pump.

A starting initiator mixture was prepared, consisting of 1.1 grams V50 dissolved in 8.8 grams of DI water and 8.6 grams M8.

A delayed addition initiator mixture was prepared, consisting of 1.6 grams V50 dissolved in 9.1 grams of DI water and 9.1 grams IPA. The mixture was added to a 50 ml syringe barrel for delayed addition to the reactor with a FMI QG-6-0SSY metering pump.

Polymerization was started by adding the starting mixture all at once to the reactor.

After 20 minutes, at 69 °C, the delayed feed pumps were started for monomer and initiator feeds adjusted to finish in 60 minutes. The process temperature held steady at 71-72 °C during feeds with minimal heat evolved. The temperature was increased to 73-74 °C and held 30 minutes more after feeds were done. Then a mixture of 0.5 grams V50 in 4.4 grams DI water was added and the temperature increased to 81 °C for 50 minutes. Another mixture of 0.6 grams V50 in 5.4 grams DI water was added and the temperature held for 20 minutes more. 79 grams of hot DI water was added turning the polymer mixture transparent. Nitrogen sparging, accompanied by a partial vacuum, was used to remove solvent over a period of 2 ½ hours during which time a total of 190 grams more hot DI water was added and the polymer mixture became pearlescent and more viscous.

The finished viscous polymer liquid was kept in a 50 °C oven overnight whereupon a separate low viscosity transparent layer had separated and 140 grams of this was decanted leaving about 480grams polymer mixture. The final appearance at 20 °C was a pearlescent/translucent, highly viscous liquid.

### Example 5

A monomer mixture was prepared by stirring together 121.0 grams of M1, 96.3 grams of M3, 41.25 grams of M6 and 51.6 grams of M8 with 220 grams 2-propanol (IPA).

The monomer mixture was warmed to 50 °C and 212 grams (40%) were charged to a 1 L glass resin reactor fitted with a stainless steel stirrer and reflux condenser, and then sparged with dry nitrogen for 30 minutes while heating to 67 °C by immersion in a >67 °C water bath. 27.5 grams additional IPA were added to the remaining 318 grams of monomer mixture and poured into a 500 ml Pyrex addition funnel for delayed addition to the reactor with a FMI QG-50-1CKC metering pump. An initiator mixture, consisting of 1.1 grams V50 dissolved in 3.3 grams of DI water and 8.8 grams methanol (MeOH), was added to a 50 ml syringe barrel for delayed addition to the reactor with a FMI QG-6-0SSY metering pump.

At 67 °C, polymerization was initiated by adding a mixture of 0.99 gram V50 in 2.48 grams of DI water and 2.48 grams of MeOH all at once to the stirred reactor. Within 1 minute, the contents of the reactor began to lose transparency. By 4 minutes, the batch temperature began to rise. After 9 minutes, the temperature had risen to 67 °C and the pumps were turned on for both the delayed addition initiator and monomer mixtures. The monomer mixture had already been adjusted to deliver in 60 minutes and the initiator mixture had been adjusted to deliver in 90 minutes. The water bath temperature was increased to a setting of 90 °C at 23 minutes. By the time all the monomer mixture had been delivered to the reactor, the batch temperature had increased to 76 °C and the contents were viscous and white in color. After all the initiator mixture had been delivered, the reactor was still at 76 °C and the process was held for a further 2 hours and 20 minutes.

Nitrogen sparging was then started and a water aspirator attached to apply partial vacuum and remove volatile solvent with additional heat from the water bath to keep the reactor contents at 68-70 °C. After 15 minutes vacuum sparging, 321 grams of hot (70-80 °C)-DI water was added. Afurther 139.5 more grams of hot water were added 1 hour and 15 minutes later, and the viscosity of the polymer solution increased as solvent was removed while vacuum sparging was continued for another 1 hour 15 minutes. Finally, the polymer solution was cooled to room temperature and 3.0 grams of 10% aqueous hydrogen peroxide was added as preservative. The final appearance was an opaque viscous liquid.

### Example 6

A monomer mixture was prepared by stirring together 41.25 grams of M5, 192.5 grams of M3 and 51.6 grams of M8 with 220 grams IPA.

The monomer mixture was warmed to 50 °C and 202 grams (40%) were charged to a 1 L glass resin reactor fitted with a stainless steel stirrer and reflux condenser, and then sparged with dry nitrogen for 30 minutes while heating to 65 °C by immersion in a 68 °C water bath. 27.5 grams additional IPA were added to the remaining 303 grams of monomer mixture and poured into a 500 ml Pyrex addition funnel for delayed addition to the reactor with a FMI QG-50-1 CKC metering pump. An initiator mixture, consisting of 1.4 grams V50 dissolved in 4.3 grams of DI water and 11.4 grams IPA, was added to a 50 ml syringe barrel for delayed addition to the reactor with a FMI QG-6-0SSY metering pump.

At 65 °C, polymerization was initiated by adding a mixture of 1.4 gram V50 in 3.6 grams of DI water and 3.6 grams of MeOH all at once to the stirred reactor. Within 1 minute, the contents of the reactor turned cloudy and rose very quickly in temperature to 73 °C. An additional 2.3 grams of IPA were added and the temperature leveled off. After 3 minutes, pumps were turned on for both delayed addition initiator and monomer mixtures. The monomer mixture had been adjusted to deliver in 60 minutes and the initiator mixture had been adjusted to deliver in 90 minutes. The water bath temperature was increased stepwise to 79 °C during the delayed addition while the reactor temperature increased slowly to 77 °C. After all the initiator mixture had been delivered, the process was held at 75 °C for an additional 2 hours.

Nitrogen sparging was then started and a water aspirator attached to apply partial vacuum and remove volatile solvent with additional heat from the water bath to keep the reactor contents at >66 °C, After 15 minutes vacuum sparging, 123 grams of hot (70-80 °C) DI water was added which turned the batch clear. Further water addition to 321 grams turned the batch cloudy/opaque. Vacuum sparging was continued for another 3.5 hours. Finally, the polymer solution was cooled to room temperature and 3.0 grams of 10% aqueous hydrogen peroxide was added as preservative. The final appearance was a translucent liquid.

### Example 7

A monomer mixture was prepared bystirring together 144.4 grams of M3, 82.5 grams of M5, 34.4 grams of M4, and 17.2 grams of M8 with 220 grams IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 64 °C by immersion in a 66 °C water bath. 27.5 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 64 °C, polymerization was initiated by adding a mixture of 1.4 gram V50 in 3.6 grams of DI water and 3.6 grams of MeOH all at once to the stirred reactor. Within 1 minute, the contents of the reactor began to turn cloudy. With little thermal response the temperature setting was increased to 82 °C after 3 minutes. With the batch temperature continuing to parallel the bath temperature, the initiator feed was started at 16 minutes. At 24 minutes the temperature was raised again to a setting of 90.7 °C and the monomer feed was started. The monomer mixture had been adjusted to deliver in 60 minutes and the initiator mixture had been adjusted to deliver in 90 minutes. An additional 0.5 gram of the initiator mixture was added at 30 minutes to instigate an exotherm. The water bath temperature was maintained between 70-80 °C during the delayed addition. By the time all the monomer and initiator mixtures had been delivered to the reactor, the temperature was at 77 °C and the contents were cloudy/milky. The process was then held for 2 hours 10 minutes.

Nitrogen sparging was then started and a water aspirator attached to apply partial vacuum and remove volatile solvent with additional heat from the water bath to keep the reactor contents between 70-80 °C. After 15 minutes vacuum sparging, 89 grams of hot (70-80 °C) DI water was added which turned the reactor contents completely transparent. An additional 232 grams of hot water were added and the contents turned white. The viscosity of the polymer solution increased as solvent was removed while vacuum sparging was continued for another 2 hours and 20 minutes. Finally, the polymer solution was cooled to room temperature and 3.0 grams of 10% aqueous hydrogen peroxide was added as preservative. The final appearance was a transparent viscous liquid.

### Example 8

A monomer mixture was prepared bystirring together 29.6 grams of M1, 39.8 grams of M7, 22.5 grams of M2, 82.9 grams of M6, 38.5 grams of M3, 13.8 grams of M4 and 68.8 grams of M8 with 220 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 65°C by immersion in a 68 °C water bath. 27.5 grams additional IPA were added to the remaining 309 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 65 °C, polymerization was initiated by adding a mixture of 1.4 grams V50 in 2.5 grams of DI water and 2.5 grams of MeOH all at once to the stirred reactor. Within 3 minutes, the contents of the reactor began rise in temperature and turned cloudy. After 6 minutes, the batch was white and the temperature was raised to a setting of 90 °C. At 8 minutes the batch exhibited two different phases while the viscosity had climbed. At 9 minutes the contents had risen to 82 °C and pumps were turned on for both delayed addition initiator and monomer mixtures. The monomer mixture had been adjusted to deliver in 60 minutes and the initiator mixture had been adjusted to deliver in 90 minutes. The temperature was at 81 °C by the time all the monomer mixture had been delivered to the reactor and 76 °C by the end of the initiator feed addiion. The contents were viscous and white and the process held at 73-76 °C for an additional 2 hours and 20 minutes.

At this point, the temperature setting was increased to 100 °C and nitrogen sparging was started and a water aspirator attached to apply partial vacuum to remove volatile solvent. After 10 minutes sparging, 176 grams of hot (70-80 °C) DI water was added which turned the mixture transparent. The addition of another 159 grams of water resulted in a somewhat clear batch. Sparging continued for another 2 hours and 10 minutes. Finally, the polymer solution was cooled to room temperature and 3.0 grams of 10% aqueous hydrogen peroxide was added as preservative. The final appearance was a translucent viscous liquid.

### Example 9

A monomer mixture was prepared by stirring together 122.7 grams M3, 20.4 grams M4, 20.4 grams M5, 10.2 grams M7 and 38.3 grams M8 with 128.3grams IPA.

The monomer mixture was warmed to 50 °C and 170 grams (50%) were charged to a 1 L glass resin reactor fitted with a stainless steel stirrer and reflux condenser, and then sparged with dry nitrogen for 20 minutes while heating to 76 °C by immersion in a 88 °C water bath. 29.0 grams additional IPA were added to the remaining 169 grams of monomer mixture, sparged with nitrogen and poured into a 500 ml Pyrex addition funnel for delayed addition to the reactor with a FMI QG-50-1CKC metering pump. An initiator mixture, consisting of 0.79 grams V50 dissolved in 4.0 grams of DI water and 4.0 grams IPA, was added to a 50 ml syringe barrel for delayed addition to the reactor with a FMI QG-6-0SSY metering pump.

Polymerization was started as an initiator mixture, consisting of 0.79 grams V50 dissolved in 4.0 grams of DI water and 4.0 grams IPA, was added all at once to the reactor.

Almost immediately, the contents became turbid and white and the temperature rose rapidly. After 3 minutes, the temperature had stabilized at 83 °C and pumps were turned on for both delayed addition initiator and monomer mixtures. The monomer and initiator mixtures had been adjusted to deliver in 60 minutes. The water bath temperature was increased stepwise to 88 °C during the first 20 minutes of delayed addition. Five minutes after both monomer and initiator feeds had run out, the bath temperature was increased and the batch held one hour at 83 °C. When 85 grams DI water was added to the turbid solution, it became transparent. 118 grams additional water was added and it became more viscous and milky. Nitrogen sparging, accompanied by a partial vacuum was started to remove solvent vapor for 4 hours at 90 °C. Vacuum and sparging was terminated followed by cooling to room temperature. The final appearance was a translucent, highly viscous liquid.

### Example 10

A monomer mixture was prepared bystirring together 112.38 of M1, 37.81 grams of M2, 41.26 grams of M6, 51.57 grams of M3 and 68.75 grams of M8 with 220 grams of IPA.

The monomer mixture was warmed to 50 °C and 213 grams (40%) were charged to a 1 L glass resin reactor fitted with a stainless steel stirrer and reflux condenser, and then sparged with dry nitrogen for 30 minutes while heating to 66 °C by immersion in a 68 °C water bath. 27.5 grams additional IPA were added to the remaining 319 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 66 °C, polymerization was initiated by adding a mixture of 1.4 grams V50 in 2.5 grams of DI water and 2.5 grams of MeOH all at once to the stirred reactor. Within 1 minute, the contents of the reactor began rise in temperature and turned white. After 4 minutes, the batch was 70 °C and the temperature was raised to a setting of 88 °C. At 6 minutes the contents had risen to 71 °C and pumps were turned on for both delayed addition initiator and monomer mixtures. The monomer mixture had been adjusted to deliver in 60 minutes and the initiator mixture had been adjusted to deliver in 90 minutes. The temperature was at 73-77 °C by the time all the monomer and initiator mixtures had been delivered to the reactor. The contents were viscous and white and the process held above 71 °C for an additional 2 hours.

At this point, the temperature was at 71 °C and the setting increased to 100 °C and nitrogen sparging was started and a water aspirator attached to apply partial vacuum to remove volatile solvent. After 40 minutes sparging, 160 grams of hot (70-80 °C) DI water was added which turned the mixture somewhat clear. The addition of another 172 grams of water turned the batch white again. Sparging continued for another 2 hours and 10 minutes. Finally, the polymer solution was cooled to room temperature and 3.0 grams of 10% aqueous hydrogen peroxide was added as preservative. The final appearance was an opaqueviscous liquid.

### Example 11

A monomer mixture was prepared bystirring together 120.66 grams of M1, 7.23 grams of M7, 103.15 grams of M2, 41.27 grams of M5, 13.76 grams of M3 and 17.21 grams of M8 with 220 grams of IPA.

The monomer mixture was warmed to 50 °C and 209 grams (40%) were charged to a 1 L glass resin reactor fitted with a stainless steel stirrer and reflux condenser, and then sparged with dry nitrogen for 30 minutes while heating to 67 °C by immersion in a 68 °C water bath. 27.6 grams additional IPA were added to the remaining 314 grams of monomer mixture and poured into a 500 ml Pyrex addition funnel for delayed addition to the reactor with a FMI QG-50-1 CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 67 °C, polymerization was initiated by adding a mixture of 1.4 grams V50 in 2.5 grams of DI water and 2.5 grams of MeOH all at once to the stirred reactor. Within 1 minute, the contents of the reactor had turned white. After 8 minutes, with the batch hovering near 67 °C, the temperature was raised to a setting of 89 °C. At 23 minutes the contents was at 71 °C and pump was turned on for the initiator addition feed. At 25 minutes with little thermal response an additional 5.4 grams of initiator feed was placed directly into the charge. The monomer mixture had been adjusted to deliver in 60 minutes and the initiator mixture had been adjusted to deliver in 90 minutes. The temperature was at 79 °C by the time all the monomer and initiator mixtures had been delivered to the reactor. The contents were viscous and cloudy and the process held above 74 °C for an additional 2 hours.

At this point the temperature was at 74 °C and the setting increased to 100 °C and nitrogen sparging was started and a water aspirator attached to apply partial vacuum to remove volatile solvent. The batch was clear with a reddish hue. After 40 minutes sparging, 25 grams of hot (70-80 °C) DI water was added which turned the mixture somewhat cloudy. The addition of another 182 grams of water turned the batch opaque. A final addition of 124 grams was added with the batch still opaque. Sparging coninued for another 2 hours and 10 minutes. Finally, the polymer solution was cooled to room temperature and 3.0 grams of 10% aqueous hydrogen peroxide was added as preservative. The final appearance was an opaque viscous liquid.

### Example 12

A monomer mixture was prepared by stirring together 129.67 grams of M1, 29.77 grams of M2, 41.29 grams of M6, 73.35 grams of M3 and 34.90 grams of M8 with 220 grams of IPA.

The monomer mixture was warmed to 50 °C and 211 grams (40%) were charged to a 1 L glass resin reactor fitted with a stainless steel stirrer and reflux condenser, and then sparged with dry nitrogen for 30 minutes while heating to 63 °C by immersion in a 67 °C water bath. 27.5 grams additional IPA were added to the remaining 317 grams of monomer mixture and poured info a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 63 °C, polymerization was initiated by adding a mixture of 1.4 grams V50 in 2.5 grams of DI water and 2.5 grams of MeOH all at once to the stirred reactor. Within 1 minute, the contents of the reactor had turned white and the temperature began to rise. After 5 minutes, with the batch at 66 °C, pumps were turned on for both delayed addition initiator and monomer mixtures. The monomer mixture had been adjusted to deliver in 60 minutes and the initiator mixture had been adjusted to deliver in 90 minutes. The temperature was at 81 °C by the time all the monomer and initiator mixtures had been delivered to the reactor. The contents had a bluish, cloudy/translucent quality and the process held above 67 °C for an additional 3 hours 20minutes.

At this point, the temperature of the reaction mixture was at 68 °C and the heat setting on the water bath was increased to 101 °C. Nitrogen sparging was started and a water aspirator attached to apply partial vacuum to remove volatile solvent. The batch was brown in hue and cloudy. After 1 hour and 20 minutes sparging, 142 grams of hot (70-80 °C) DI water was added which turned the mixture tan and opaque. The final addition of 180 grams was added with the batch still opaque. Sparging coninued for another 30 minutes. Finally, the polymer solution was cooled to room temperature and 3.0 grams of 10% aqueous hydrogen peroxide was added as preservative. The final appearance was an opaque viscous liquid.

### Example 13

A monomer mixture was prepared by stirring together 96.75 grams of M9, 8.49 grams of M7, 103.15 grams of M2, 41.27 grams of M5, 13.80 grams of M3 and 17.20 grams of M8 with 220 grams of IPA.

The monomer mixture was warmed to 50 °C and 200 grams (40%) were charged to a 1 L glass resin reactor fitted with a stainless steel stirrer and reflux condenser, and then sparged with dry nitrogen for 30 minutes while heating to 67 °C by immersion in a 70 °C water bath. 27.5 grams additional IPA were added to the remaining 300 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 67 °C, polymerization was initiated by adding a mixture of 1.4 grams V50 in 2.5 grams of DI water and 2.5 grams of MeOH all at once to the stirred reactor. Within 1.5 minutes, the contents of the reactor had turned cloudy and the temperature began to rise. After 6 minutes, with the batch at 75 °C, pumps were turned on for both delayed addition initiator and monomer mixtures. The monomer mixture had been adjusted to deliver in 60 minutes and the initiator mixture had been adjusted to deliver in 90 minutes. The temperature was at 77 °C by the time all the monomer and initiator mixtures had been delivered to the reactor. The contents were opaque and the process held above 75 °C for an additional 1 hours 50 minutes.

At this point, the temperature was at 75 °C and the heat setting was increased to 101 °C. Nitrogen sparging was started and a water aspirator attached to apply partial vacuum to remove volatile solvent The batch was red in hue and opaque. After 1 hour and 11 minutes sparging, 142 grams of hot (70-80 °C) DI water was added which turned the clearer. The final addition of 205 grams was added with the batch returning to a reddish opacity, but perhaps were air bubbles. Sparging continued for another 1 hour and 45 minutes. Finally, the polymer solution was cooled to room temperature and 3.0 grams of 10% aqueous hydrogen peroxide was added as preservative. The final appearance was a translucent viscous liquid.

### Example 14

A monomer mixture was prepared by stirring together 84.33 grams of M9, 68.75 grams of M10, 55.0 grams of M6, 41.25 grams of M3, and 34.38 grams of M8 with 220 grams of IPA.

The monomer mixture was warmed to 50 °C and 201 grams (40%) were charged to a 1 L glass resin reactor fitted with a stainless steel stirrer and reflux condenser, and then sparged with dry nitrogen for 30 minutes while heating to 64 °C by immersion in a 66 °C water bath. 27.9 grams additional IPA were added to the remaining 300 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 64 °C, polymerization was initiated by adding a mixture of 1.4 grams V50 in 2.5 grams of DI water and 2.5 grams of MeOH all at once to the stirred reactor. Within 1 minute, the contents of the reactor had turned cloudy and the temperature began to rise slightly. After 3 minutes, the temperature setting was raised to 85 °C. At 5 minutes, with the batch at 66 °C, pumps were turned on for both delayed addition initiator and monomer mixtures. The monomer mixture had been adjusted to deliver in 60 minutes and the initiator mixture had been adjusted to deliver in 90 minutes. The temperature was at 75 °C by the time all the monomer and initiator mixtures had been delivered to the reactor. The contents were pearlescent in appearance and the process held above 71 °C for an additional 2 hours.

At this point, the temperature was at 71 °C and the heat setting increased to 101 °C. After 30 minutes, nitrogen sparging was started and a water aspirator attached to apply partial vacuum to remove volatile solvent The batch was pearlescent. After 45 minutes sparging, 323 grams of hot (70-80 °C) DI water was added with no change in appearance throughout the addition. Sparging continued for another 1 hour and 37 minutes. Finally, the polymer solution was cooled to room temperature and 3.0 grams of 10% aqueous hydrogen peroxide was added as preservative. The final appearance was a translucent viscous liquid.

### Example 15

A monomer mixture was prepared by stirring together 112.45 grams of M9, 82.52 grams of M6, 55.01 grams of M3, and 34.38 grams of M8 with 220 grams of IPA.

The monomer mixture was warmed to 50 °C and 202 grams (40%) were charged to a 1 L glass resin reactor fitted with a stainless steel stirrer and reflux condenser, and then sparged with dry nitrogen for 30 minutes while heating to 67 °C by immersion in a 68 °C water bath. 27.5 grams additional IPA were added to the remaining 300 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 67 °C, polymerization was initiated by adding a mixture of 1.4 grams V50 in 5 grams of DI water all at once to the stirred reactor. Within 1.5 minutes, the contents of the reactor had turned cloudy and the temperature began to rise slightly; the heat setting was increased to 85 °C. After 6 minutes, with the batch at 70 °C, pumps were turned on for both delayed addition initiator and monomer mixtures. The monomer mixture had been adjusted to deliver in 60 minutes and the initiator mixture had been adjusted to deliver in 90 minutes. The temperature was at67 °C when the setting was increased to 90 °C at 20 minutes. By the time all the monomer and initiator mixtures had been delivered to the reactor, the temperature was 78.5 °C and the contents were cloudy. The process was held above 69 °C for an additional 2 hours and 20 minutes.

At this point, the temperature was at 69 °C and the setting increased to 101 °C. The nitrogen sparging was started with a water aspirator attached to apply partial vacuum to remove volatile solvent. After a 1 hour sparge, 325 grams of hot (70-80 °C) DI water was added with no change in appearance throughout the addition with a perhaps slight drop in viscosity. Sparging continued for another 1 hour and 50 minutes. Finally, the polymer solution was cooled b room temperature and 3.0 grams of 10% aqueous hydrogen peroxide was added as preservative. The final appearance was a translucent viscous liquid.

### Example 16

A monomer mixture was prepared bystirring together 139.18 grams of M1, 33.69 grams of M7, 96.27 grams of M2, and 42.05 grams of M8 with 220 grams of I PA.

The monomer mixture was warmed to 50 °C and 213 grams (40%) were charged to a 1 L glass resin reactor fitted with a stainless steel stirrer and reflux condenser, and then sparged with dry nitrogen for 30 minutes while heating to 65 °C by immersion in a 67 °C water bath. 27.5 grams additional IPA were added to the remaining 319 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 65 °C, polymerization was initiated by adding a mixture of 1.4 grams V50 in 5 grams of DI water all at once to the stirred reactor. In less than one minute, the contents of the reactor had turned cloudy and the temperature began to rise slightly. At one minute, the contents were white and the heat setting was increased to 90 °C. After 4 minutes, with the batch at 65 °C after a very slight thermal response, the feed pump was turned on for the delayed addition initiator mixture. At 9 minutes with the temperature at 66 °C, the monomer feed pump was started. The monomer mixture had been adjusted to deliver in 60 minutes and the initiator mixture had been adjusted to deliver in 90 minutes. By the time all the monomer and initiator mixtures had been delivered to the reactor, the temperature was 76 °C and the contents were white. The process was held above 71 °C for an additional 2 hours and 30 minutes.

At this point, the temperature was at 71 °C and the heat setting was increased to 101 °C. Nitrogen sparging was started with a water aspirator attached to apply partial vacuum to remove volatile solvent After a 1 hour sparge, 61 grams of hot (70-80 °C) DI water was added with the appearance changing to a pearlescent color. The final addition of 283 grams of water returned the batch to a white color. Sparging continued for another 1 hour and 20 minutes. Finally, the polymer solution was cooled to room temperature and 3.0 grams of 10% aqueous hydrogen peroxide was added as preservative. The final appearance was an opaque viscous liquid.

### Example 17

A monomer mixture was prepared by stirring together 112.1 grams M3, 64.1 grams M5, 24.0 grams M14 and 20.0 grams M8 with 120 grams IPA and 4.8 grams DI water.

The monomer mixture was charged to a 1 L glass resin reactor fitted with a stainless steel stirrer and reflux condenser, and then sparged with dry nitrogen for 15 minutes while heating to 71 °C by immersion in a 86 °C water bath. An initiator mixture, consisting of 0.91 grams V50 dissolved in 3.6 grams of DI water and 1.0 grams IPA, was added all at once to the reactor.

After one minute, the contents became increasingly cloudy and in 2 minutes, the temperature was rising rapidly at 83 °C. After 12 minutes, the turbid solution had increased substantially in viscosity and reflux had stopped. The batch was held at 85 °C for an additional 58 minutes at which time 90 grams of hot DI water was added which turned the contents transparent. 150 grams more DI water was added which turned the contents turbid again. Nitrogen sparging, accompanied by a partial vacuum, was used to remove solvent vapor over a period of 90 minutes followed by cooling to room temperature. The final appearance was an opaque, highly viscous liquid.

### Example 18

A monomer mixture was prepared by stirring together 140.55 grams of M9, 82.50 grams of M6, 27.52 grams of M5, and 34.45 grams of M8 with 220 grams of IPA.

The monomer mixture was warmed to 50 °C and 202 grams (40%) were charged to a 1 L glass resin reactor fitted with a stainless steel stirrer and reflux condenser, and then sparged with dry nitrogen for 30 minutes while heating to 66 °C by immersion in a 69 °C water bath. 27.5 grams additional IPA were added to the remaining 303 grams of monomer mixture and poured into a 500 ml Pyrex addition funnel for delayed addition to the reactor with a FMI QG-50-1 CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 65 °C, polymerization was initiated by adding a mixture of 1.4 grams V50 in 5 grams of DI water all at once to the stirred reactor. In less than 2 minutes, the contents of the reactor had turned cloudy and the temperature began to rise slightly. The temperature setting was adjusted to 90 °C at that time. After 5 minutes, with the batch at 70 °C and the exotherm had reached a plateau, the feed pump was turned on for the delayed addition initiator mixture. At 7 minutes with the temperature still at 70 °C, the monomer feed pump was started. The monomer mixture had been adjusted to deliver in 60 minutes and the initiator mixture had been adjusted to deliver in 90 minutes. By the time all the monomer and initiator mixtures had been delivered to the reactor, the temperature was 81 °C and the content was translucent. The process was held above 77 °C for an additional 3 hours and 20 minutes.

At this point, the temperature was at 77 °C and nitrogen sparging was started with a water aspirator attached to apply partial vacuum to remove volatile solvent. After a 30 minute sparge, 131 grams of hot (70-80 °C) DI water was added with the appearance changing to somewhat clear. The final addition of 194 grams of water left the appearance clear. Sparging continued for another 1 hour and 30 minutes. Finally, the polymer solution was cooled to room temperature and 3.0 grams of 10% aqueous hydrogen peroxide was added as preservative. The final appearance was a clear viscous liquid.

### Example 19

A monomer mixture was prepared by stirring together 121.1 grams of M1, 68.79 grams of M10, 96.28 grams of M5, and 17.20 grams of M8 with 220 grams of IPA.

The monomer mixture was warmed to 50 °C and 209 grams (40%) were charged to a 1 L glass resin reactor fitted with a stainless steel stirrer and reflux condenser, and then sparged with dry nitrogen for 30 minutes while heating to 66 °C by immersion in a 67 °C water bath. 27.5 grams additional IPA were added to the remaining 314 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 66 °C, polymerization was initiated by adding a mixture of 1.4 grams V50 in 5 grams of DI water all at once to the stirred reactor. In 2 minutes, the contents of the reactor had turned cloudy and the temperature began to rise quickly. The temperature setting was adjusted to 90 °C and the monomer feed started to dampen the exotherm. After 3 minutes, with the temperature continuing to rise to 82 °C, 15.48 g of IPA was added to cool the reaction. At 7 minutes, the bath temperature was lowered with cold water in a further attempt to reduce heat, and the feed pump was turned on for the delayed addition initiator mixture. The monomer mixture had been adjusted to deliver in 60 minutes and the initiator mixture had been adjusted to deliver in 90 minutes. At 39 minutes, with the temperature at 76 °C, an additional 3.06 g of IPA was added in another attempt to cool the reaction. By the time all the monomer and initiator mixtures had been delivered to the reactor, the temperature was 76 °C and the content was transparent with a pinkish hue. The process was held above 73 °C for an additional 2 hours and 30 minutes.

At this point, the temperature was at 74 °C and nitrogen sparging was started with a water aspirator attached to apply partial vacuum to remove volatile solvent. After a 50 minutes sparge, 106 grams of hot (70-80 °C) DI water was added with the appearance changing to somewhat clear. The final addition of 220 grams of water left the appearance clear. Sparging continued for another 1 hour and 30 minutes. Finally, the polymer solution was cooled to room temperature and 3.1 grams of 10% aqueous hydrogen peroxide was added as preservative. The final appearance was a clear viscous liquid.

### Example 20

A monomer mixture was prepared bystirring together 69.17 grams of M1, 55.01 grams of M12, 82.57 grams of M3, 68.75 grams of M5 and 17.21 grams of M8 with 220 grams of IPA.

The monomer mixture was warmed to 50 °C and 209 grams (40%) were charged to a 1 L glass resin reactor fitted with a stainless steel stirrer and reflux condenser, and then sparged with dry nitrogen for 30 minutes while heating to 66 °C by immersion in a 68 °C water bath. 27.5 grams additional IPA were added to the remaining 308 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 66 °C, polymerization was initiated by adding a mixture of 1.4 grams V50 in 5 grams of DI water all at once to the stirred reactor. In less than one minute, the contents of the reactor had turned cloudy. At 2.5 minutes, the temperature setting was adjusted to 90 °C and 3.42 grams of the initiator feed solution was added directly to the reactor. After 4.5 minutes, with the temperature still at 66 °C, the initiator feed was started. At 10 minutes, the temperature was at 67 °C and the feed pump was turned on for the delayed addition monomer mixture. The monomer mixture had been adjusted to deliver in 60 minutes and the initiator mixture had been adjusted to deliver in 90 minutes. By the time all the monomer and initiator mixtures had been delivered to the reactor, the temperature was 81 °C and the content was opaque with a pinkish hue. The process was held above 72 °C for an additional 2 hours.

At this point, the temperature was at 72 °C and nitrogen sparging was started with a water aspirator attached to apply partial vacuum to remove volatile solvent. After a 1 hour sparge, 70 grams of hot (70-80 °C) DI water was added with the appearance being white. The final addition of 262 grams of water left the appearance white. Sparging continued for another 1 hour and 20 minutes. Finally, the polymer solution was cooled to room temperature and 3.2 grams of 10% aqueous hydrogen peroxide was added as preservative. The final appearance was a clear viscous liquid.

### Example 21

A monomer mixture was prepared bystirring together 121.11 grams of M1, 41.30 grams of M6, 96.25 grams of M3, and 51.57 grams of M8 with 55 grams of IPA and 165 grams of propylene glycol.

The monomer mixture was warmed to 50 °C and 212 grams (40%) were charged to a 1 L glass resin reactor fitted with a stainless steel stirrer and reflux condenser, and then sparged with dry nitrogen for 30 minutes while heating to 65 °C by immersion in a 67 °C water bath. 27.5 grams additional IPA were added to the remaining 318 grams of monomer mixture and poured into a 500 ml Pyrex addition funnel for delayed addition to the reactor with a FMI QG-50-1CKC metering pump. An initiator mixture, consisting of 1.4 grams V50 dissolved in 12.1 grams of DI water, was added to a 50 ml syringe barrel for delayed addition to the reactor with a FMI QG-6-0SSY metering pump.

At 65 °C, polymerization was initiated by adding a mixture of 1.4 grams V50 in 7 grams of DI water all at once to the stirred reactor. In less than one minute, the reactor temperature had begun to rise. At 3 minutes, the temperature setting was adjusted to 90 °C. After 7 minutes, the temperature was at 80.5 °C and the feed pump was turned on for the delayed addition monomer mixture to cool the reaction. At 8 minutes, with the temperature continuing to rise to 82.7 °C, 5.20 g of IPA was added to dampen the exotherm and the initiator feed was started. The monomer mixture had been adjusted to deliver in 60 minutes and the initiator mixture had been adjusted to deliver in 90 minutes. By the time all the monomer and initiator mixtures had been delivered to the reactor, the temperature was 73 °C and the content was clear. The process was held at around 75 °C for an additional 1 hour and 40 minutes, after which the reactor was stopped to continue the next day.

On day two, the reactor temperature was at 18 °C and the heat was turned on to 90 °C. After 3 hours and 11 minutes, the temperature reached 72 °C and nitrogen sparging was started with a water aspirator attached to apply partial vacuum to remove volatile solvent. After a 25 minute sparge, a chaser of 1.65 grams of V50 and 19.80 grams of propylene glycol was added directly to the reactor. At 4 hours and 27 minutes, the temperature setting was raised to 110.7 °C. Finally, at 7 hours, the polymer solution was cooled to room temperature and 3.2 grams of 10% aqueous hydrogen peroxide was added as preservative. The final appearance was a clear viscous liquid.

### Example 22

A starting monomer mixture was prepared by stirring together 74.3 grams M3 and 82.5 grams M5 with 100 grams IPA.

The mixture was charged to a 1 L glass resin reactor fitted with a stainless steel stirrer and reflux condenser, and then sparged with dry nitrogen for 20 minutes while heating to 69 °C by immersion in a 72 °C water bath.

A second monomer mixture was prepared by stirring together 49.6 grams M3, 55.0 grams M5 and 6.9 grams M8 with 99.1 grams IPA. This was sparged with nitrogen, warmed to 50 °C and poured into a 500 ml Pyrex addition funnel for delayed addition to the reactor with a FMI QG-50-1 CKC metering pump.

A starting initiator mixture was prepared, consisting of 1.3 grams V50 dissolved in 9.0 grams of DI water and 10.36 grams M8.

A delayed addition initiator mixture was prepared, consisting of 1.3 grams V50 dissolved in 6.9 grams of DI water and 6.9 grams IPA. The mixture was added to a 50 ml syringe barrel for delayed addition to the reactor with a FMI QG-6-0SSY metering pump.

Polymerization was started by adding the starting mixture all at once to the reactor.

The temperature rose very quickly to reflux at 92 °C in 2 minutes with the heated water bath removed. Delay monomer and initiator feed pumps were started, adjusted to finish in 30 minutes, having lowered the temperature to 65 deg. Nitrogen sparging, accompanied by a partial vacuum, was used to remove solvent over a period of 3 hours during which time a total of 407 grams of DI water was pumped in during the first hour. The polymer mixture became extremely viscous but remained transparent throughout

The finished viscous polymer liquid was kept in a 50 °C oven overnight to release many small nitrogen bubbles. The final appearance at 20 °C was a transparent, extremely viscous gel.

### Example 23

A monomer reactor charge mixture was prepared by stirring together 25.94 grams of M1, 20.67 grams of M7, 13.80 grams of M2, 6.92 grams of M3, 48.14 grams of M4 and 34.37 grams of M8 with 121.25 grams of IPA.

A monomer feed mixture was prepared by stirring together 26.02 grams of M1, 20.66 grams of M7, 13.74 grams of M2, 6.87 grams of M3, 48.13 grams of M4 and 34.36 grams of M8 with 132.58 grams of IPA.

The monomer mixtures were warmed to 50 °C and the reactor charge mixture was charged to a 1 L glass resin reactor fitted with a stainless steel stirrer and reflux condenser, and then sparged with dry nitrogen for 30 minutes while heating to 58 °C by immersion in a 66 °C water bath. The feed mixture was poured into a 500 ml Pyrex addition funnel for delayed addition to the reactor with a FMI QG-50-1CKC metering pump. An initiator mixture, consisting of 1.4 grams V50 dissolved in 8 grams of DI water and 8 grams of IPA, was added to a 50 ml syringe barrel for delayed addition to the reactor with a FMI QG-6-0SSY metering pump.

At 58 °C, polymerization was initiated by adding a mixture of 1.6 grams V50 in 7.7 grams of Dl water all at once to the stirred reactor. The contents of the reactor had turned turbid in 7 minutes and white in 10 minutes, with the temperature rising steadily during that time. At 14.5 minutes, the temperature was at 72 °C and the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 70 °C and the process was held above 72 °C for an additional 2 hours 30 minutes.

At this point, the temperature was at 80 °C and 170 grams of hot (70-80 °C) DI water was added turning the contents transparent. The final addition of 153 grams of water left the appearance mostly transparent. At 3 hours and 54 minutes, the nitrogen sparging was started with a water aspirator attached to apply partial vacuum to remove volatile solvent. Sparging was stopped then resumed at 21 hours and 48 minutes, when another 183 grams of hot DI water was added which increased the viscosity. Sparging continued for another 0.5 hour and the polymer solution was cooled to room temperature and 3.0 grams of 10% aqueous hydrogen peroxide was added as preservative several weeks later during transfer to a new container. The final appearance was a pearlescent viscous liquid.

### Example 24

A monomer reactor charge mixture was prepared by stirring together 56.19 grams of M1, 37.81 grams of M2, 6.88 grams of M3, 20.63 grams of M5 and 34.38 grams of M8 with 137.50 grams of IPA.

A monomer feed mixture was prepared by stirring together 56.20 grams of M1, 37.82 grams of M2, 6.89 grams of M3, 20.64 grams of M5 and 34.45 grams of M8 with 104.50 grams of IPA.

The monomer mixtures were warmed to 50 °C and the reactor charge mixture was charged to a 1 L glass resin reactor fitted with a stainless steel stirrer and reflux condenser, and then sparged with dry nitrogen for 30 minutes while heating to 61 °C by immersion in a 68 °C water bath. The feed mixture was poured into a 500 ml Pyrex addition funnel for delayed addition to the reactor with a FMI QG-50-1CKC metering pump. An initiator mixture, consisting of 1.3 grams V50 dissolved in 7 grams of DI water and 7 grams of IPA, was added to a 50 ml syringe barrel for delayed addition to the reactor with a FMI QG-6-0SSY metering pump.

At 61 °C, polymerization was initiated by adding a mixture of 1.1 grams V50 in 5.5 grams of Dl water all at once to the stirred reactor. The contents were cloudy and 66 °C by 7 minutes, when the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 71 °C and the process was held for 1 hour.

At this point, the temperature was at 75 °C and ∼100 grams of hot (70-80 °C) Dl water was added turning the contents clearer. The addition of 224 grams of water left the appearance cloudier and translucent. At 3 hours and 58 minutes, the nitrogen sparging was started with a water aspirator attached to apply partial vacuum to remove volatile solvent and 240 additional grams of hot DI water was added which lowered the viscosity. Sparging continued for another 1 hour and 5 minutes. Finally, the polymer solution was cooled to room temperature and 3.0 grams of 10% aqueous hydrogen peroxide was added as preservative several weeks after during transfer to a new container. The final appearance was an opaque viscous liquid.

### Example 25

A monomer reactor charge mixture was prepared by stirring together 31.13 grams of M1, 19.29 grams of M2, 5.50 grams of M3, 55.06 grams of M5 and 6.88 grams of M8 with 110.01 grams of IPA.

A monomer feed mixture was prepared by stirring together 46.68 grams of M 1, 28.88 grams of M2, 8.25 grams of M3, 82.50 grams of M5 and 10.40 grams of M8 with 132.00 grams of IPA.

The monomer mixtures were warmed to 50 °C and the reactor charge mixture was charged to a 1 L glass resin reactor fitted with a stainless steel stirrer and reflux condenser, and then sparged with dry nitrogen for 30 minutes while heating to 55 °C by immersion in a 75 °C water bath. The feed mixture was poured into a 500 ml Pyrex addition funnel for delayed addition to the reactor with a FMI QG-50-1CKC metering pump. An initiator mixture, consisting of 1.7 grams V50 dissolved in 9 grams of DI water and 9 grams of IPA, was added to a 50 ml syringe barrel for delayed addition to the reactor with a FMI QG-6-0SSY metering pump.

At 55 °C, polymerization was initiated by adding a mixture of 1.4 grams V50 in 7.2 grams of DI water all at once to the stirred reactor. By 2 minutes, the temperature of the reactor was 56.8 °C and steadily rose to 76 °C at 12.5 minutes, when the feed pump was turned on for the delayed addition initiator and mixture. The monomer feed pump was turned on at 14 minutes, when cold water was added to the bath to dampen the exotherm. The monomer mixture had been adjusted to deliver in 60 minutes and the initiator mixture had been adjusted to deliver in 90 minutes. By the time all the monomer and initiator mixtures had been delivered to the reactor, 3.2 grams of the initiator feed mixture was unused and the temperature was 70 °C. The process was held for 1 hour 25 minutes.

At this point, the temperature was at 65 °C and nitrogen sparging was started with a water aspirator attached to apply partial vacuum to remove volatile solvent. At 4 hours 2 minutes, 362.96 grams of hot (70-80 °C) DI water was added which turned the contents milky. Sparging continued for another 1 hour and 30 minutes. Finally, the polymer solution was cooled to room temperature and 3.0 grams of 10% aqueous hydrogen peroxide was added as preservative several weeks after during transfer to a new container. The final appearance was an opaqueviscous liquid.

### Example 26

A monomer reactor charge mixture was prepared by stirring together 52.00 grams of M1, 35.72 grams of M2, 5.50 grams of M3, 22.03 grams of M4 and 6.90 grams of M8 with 110.01 grams of IPA.

A monomer feed mixture was prepared by stirring together 77.80 grams of M1, 53.64 grams of M2, 8.25 grams of M3, 33.00 grams of M4 and 10.33 grams of M8 with 132.02 grams of IPA.

The monomer mixtures were warmed to 50 °C and the reactor charge mixture was charged to a 1 L glass resin reactor fitted with a stainless steel stirrer and reflux condenser, and then sparged with dry nitrogen for 30 minutes while heating to 64 °C by immersion in a 70 °C water bath. The feed mixture was poured into a 500 ml Pyrex addition funnel for delayed addition to the reactor with a FMI QG-50-1CKC metering pump. An initiator mixture, consisting of 1.1 grams V50 dissolved in 6 grams of DI water and 6 grams of IPA, was added to a 50 ml syringe barrel for delayed addition to the reactor with a FMI QG-6-0SSY metering pump.

At 64 °C, polymerization was initiated by adding a mixture of 1.1 grams V50 in 5.6 grams of DI water all at once to the stirred reactor. The temperature setting was turned up stepwise to 90 °C by 19.5 minutes, when the batch was 69.4 °C. At 23 minutes, when the batch was 70.5 °C, the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 81 °C and the process was held for 1 hour 10 minutes.

At this point, the temperature was at 80 °C and nitrogen sparging was started with a water aspirator attached to apply partial vacuum to remove volatile solvent. At 3 hours 52 minutes, 119 grams of hot (70-80 °C) DI water was added which turned the contents white and the viscosity increased dramatically. An additional 199.57grams of water and 155 grams of IPA were added b reduce the viscosity, which caused the stirrer motor to stop at 4 hours and 19 min. Viscosity continued to climb and another 180 grams of water was added at 5.5 hours and sparging continued for another 30 minutes. Finally, the polymer solution was cooled to room temperature and 3.0 grams of 10% aqueous hydrogen peroxidewas added as preservative several weeks after during transfer to a new container. The final appearance was a pearlescent viscous liquid.

### Example 27

A monomer mixture was prepared by stirring together 62.73 grams of M1, 26.69 grams of M4, 17.60 grams of M5, 23.82 grams of M6, 15.05 grams of M7, 68.50 grams of M3, 46.12 grams of M2 and 38.96 grams of M8 with 110.05 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 63 °C by immersion in a 70 °C water bath. 27.5 grams additional IPA were added to the remaining 309 grams of monomer mixture and poured into a 500 ml Pyrex addition funnel for delayed addition to the reactor with a FMI QG-50-1CKC metering pump. An initiator mixture, consisting of 1.4 grams V50 dissolved in 8 grams of water and 8 grams of IPA, was added to a 50 ml syringe barrel for delayed addition to the reactor with a FMI QG-6-0SSY metering pump.

At 63 °C, polymerization was initiated by adding a mixture of 1.4 grams V50 in 7 grams of Dl water all at once to the stirred reactor. The mixture turned cloudy in 1.5 minutes. At 10.8 minutes, the temperature setting was adjusted to 87 °C and 2.5 minutes later, the feed pump was turned on for the delayed addition initiator mixture. The heat setting was raised again to 90 °C at 20 min, and was followed by a direct addition of initiator/water 8.5 minutes later. The monomer feed was started at 36 minutes at a temperature of 67 °C. The monomer mixture had been adjusted to deliver in 60 minutes and the initiator mixture had been adjusted to deliver in 90 minutes. By the time all the monomer and initiator mixtures had been delivered to the reactor, the temperature was 75 °C. The process was held above 80 °C for an additional 2 hours and 15 minutes.

At this point, the temperature was at 76 °C and nitrogen sparging was started with a water aspirator attached to apply partial vacuum to remove volatile solvent. After a 25 minute sparge, 301.8 grams of hot (70-80 °C) DI water was added directly to the reactor and sparging continued. Finally, the polymer solution was cooled to room temperature and 3.0 grams of 10% aqueous hydrogen peroxide was added as preservative several weeks later during transfer to a new container. The final appearance was a translucent viscous liquid.

### Example 28

A monomer mixture was prepared by stirring together 55.0 grams of M5, 82.5 grams of M6, 92.81 grams of M3, 30.94 grams of M2, and 17.19 grams of M8 with 220 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 60 °C by immersion in a 66 °C water bath. 27.5 grams additional IPA were added to the remaining 309 grams of monomer mixture and poured into a 500 ml Pyrex addition funnel for delayed addition to the reactor with a FMI QG-50-1 CKC metering pump. An initiator mixture, consisting of 1.5 grams V50 dissolved in 8.5 grams of water and 8.5 grams of IPA, was added to a 50 ml syringe barrel for delayed addition to the reactor with a FMI QG-6-0SSY metering pump.

At 60 °C, polymerization was initiated by adding a mixture of 1.5 grams V50 in 7.7 grams of Dl water all at once to the stirred reactor. The mixture turned cloudy less than 1 minute. By 8 minutes, with the temperature steadily rising to 62.1 °C, the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 84 °C and the temperature setting had been increased to 100 °C. The process was held above 77 °C for an additional 1 hour and 45 minutes.

At this point, the temperature was at 78 °C, and 68.8 grams of water was added directly to the reactor which increased the viscosity. After a total of 321.88 grams of hot (70-80 °C) DI water was added, the contents had turned more opaque. Directly after, the nitrogen sparging was started with a water aspirator attached to apply partial vacuum to remove volatile solvent. Sparging continued for 3.5 hours with the temperature increased stepwise to a setting of 108 °C. Finally, the polymer solution was cooled to room temperature and 3.0 grams of 10% aqueous hydrogen peroxide was added as preservative. The final appearance was a translucent viscous liquid.

### Example 29

A monomer mixture was prepared by stirring together 55.0 grams of M5, 82.5 grams of M6, 61.92 grams of M3, 61.88 grams of M2, and 17.23 grams of M8 with 220 grams of IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 62 °C by immersion in a 70 °C water bath. 27.5 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 1.65 grams V50 dissolved in 9 grams of water and 9 grams of IPA, was added to a 50 ml syringe barrel for delayed addition to the reactor with a FMI QG-6-0SSY metering pump.

At 62 °C, polymerization was initiated by adding a mixture of 1.65 grams V50 in 8.4 grams of DI water all at once to the stirred reactor. The mixture turned cloudy in less than 1 minute. By 18.4 minutes, with the temperature steadily rising to 82 °C after the setting was incrementally raised to 90 °C, the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 75 °C and the temperature setting had been increased to 100 °C. The process was held above 75 °C for an additional 1 hourand 50 minutes.

At this point, the temperature was at 75 °C, and nitrogen sparging was started with a water aspirator attached to apply partial vacuum to remove volatile solvent. After 12 minutes sparging, 250 grams of hot (70-80 °C) DI water was added, the contents turned clear. An additional 76 grams of water turned the mixture white again. Sparging continued for 2 hours and 40 minutes, with the temperature increased to a setting of 104 °C. Finally, the polymer solution was cooled to room temperature and 3.05 grams of 10% aqueous hydrogen peroxide was added as preservative. The final appearance was a clear viscous liquid.

### Example 30

A monomer mixture was prepared bystirring together 110.06 grams of M4, 13.77 grams of M5, 13.79 grams of M6, 82.57 grams of M3, and 68.75 grams of M8 with 220 grams of IPA.

The monomer mixture was warmed to 50 °C and 204 grams (40%) were charged to a 1 L glass resin reactor fitted with a stainless steel stirrer and reflux condenser, and then sparged with dry nitrogen for 30 minutes while heating to 62 °C by immersion in a 68 °C water bath. 27.5 grams additional IPA were added to the remaining 305 grams of monomer mixture and poured into a 500 ml Pyrex addition funnel for delayed addition to the reactor with a FMI QG-50-1CKC metering pump. An initiator mixture, consisting of 1.27 grams V50 dissolved in 6.9 grams of water and 6.9 grams of IPA, was added to a 50 ml syringe barrel for delayed addition to the reactor with a FMI QG-6-0SSY metering pump. At 62 °C, polymerization was initiated by adding a mixture of 1.27 grams V50 in 6.6 grams of DI water all at once to the stirred reactor. The contents reached 94 °C in less than 2.5 minutes, and the feed pumps were turned on for the delayed addition initiator and monomer mixtures.

The monomer mixture had been adjusted to deliver in 60 minutes and the initiator mixture had been adjusted to deliver in 90 minutes. By 4.5 minutes, the contents were boiling slightly and the viscosity had increased noticeably. By the time all the monomer and initiator mixtures had been delivered to the reactor, the temperature was 73 °C and the temperature setting had been increased incrementally to 92 °C. The process was held for an additional 1 hour and 45 minutes.

At this point, the temperature was at 71 °C, and nitrogen sparging was started with a water aspirator attached to apply partial vacuum to remove volatile solvent. After 30 minutes sparging, 322 grams of hot (70-80 °C) DI water was added, and the temperature setting was increased to 98 °C 14 minutes later. Sparging continued for another 2 hours and 40 minutes and the polymer solution was cooled to room temperature and 3.06 grams of 10% aqueous hydrogen peroxide was added as preservative. The final appearance was a clear viscous liquid.

### Example 31

A monomer mixture was prepared bystirring together 103.73 grams of M1, 82.50 grams of M2, 82.50 grams of M6, 137.5 grams of M3, and 17.25 grams of M8 with 220 grams of IPA.

The monomer mixture was warmed to 50 °C and 208 grams (40%) were charged to a 1 L glass resin reactor fitted with a stainless steel stirrer and reflux condenser, and then sparged with dry nitrogen for 30 minutes while heating to 63 °C by immersion in a 70 °C water bath. 27.5 grams additional IPA were added to the remaining 312 grams of monomer mixture and poured into a 500 ml Pyrex addition funnel for delayed addition to the reactor with a FMI QG-50-1CKC metering pump. An initiator mixture, consisting of 1.49 grams V50 dissolved in 8.25 grams of water and 8.25 grams of IPA, was added to a 50 ml syringe barrel for delayed addition to the reactor with a FMI QG-6-0SSY metering pump.

At 63 °C, polymerization was initiated by adding a mixture of 1.50 grams V50 in 7.43 grams of DI water all at once to the stirred reactor. The contents had turned cloudy and white in less than 2 minutes. By 7 minutes and 40 seconds, as the temperature slowly rose to 65 °C, the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 79 °C and the temperature setting had been increased incrementally to 98 °C. The process was held for an additional 1 hour and 30 minutes.

At this point, the temperature was at 73 °C, and nitrogen sparging was started with a water aspirator attached to apply partial vacuum to remove volatile solvent. After 10 minutes sparging, 327 grams of hot (70-80 °C) DI water was added, and the temperature setting was increased to 102 °C. Finally, the polymer solution was cooled to room temperature and 3.06 grams of 10% aqueous hydrogen peroxide was added as preservative. The final appearance was a translucent viscous liquid.

### Example 32

A monomer mixture was prepared bystirring together 51.86 grams of M1, 55.01 grams of M5, 82.50 grams of M6, 41.28 grams of M3, and 68.75 grams of M8 with 220 grams of IPA.

The monomer mixture was warmed to 50 °C and 208 grams (40%) were charged to a 1 L glass resin reactor fitted with a stainless steel stirrer and reflux condenser, and then sparged with dry nitrogen for 30 minutes while heating to 64 °C by immersion in a 69 °C water bath. 27.5 grams additional IPA were added to the remaining 312 grams of monomer mixture and poured into a 500 ml Pyrex addition funnel for delayed addition to the reactor with a FMI QG-50-1CKC metering pump. An initiator mixture, consisting of 1.5 grams V50 dissolved in 8.5 grams of water and 8.5 grams of IPA, was added to a 50 ml syringe barrel for delayed addition to the reactor with a FMI QG-6-0SSY metering pump.

At 64 °C, polymerization was initiated by adding a mixture of 1.5 grams V50 in 7.7 grams of DI water all at once to the stirred reactor. The viscosity of the contents had increased in less than 4 minutes. By 8 minutes and 40 seconds, as the temperature slowly rose to 71 °C, the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 78 °C and the temperature setting had been increased incrementally to 102 °C. The process was held for an additional 1 hour and 30 minutes.

At this point, the temperature was at 77 °C, and nitrogen sparging was started with a water aspirator attached to apply partial vacuum to remove volatile solvent. 325 grams of hot (70-80 °C) DI water was added, and the temperature setting was increased to 106 °C 30 minutes later. Sparging continued for another 2 hours and 45 minutes and the polymer solution was cooled to room temperature and 3.03 grams of 10% aqueous hydrogen peroxide was added as preservative. The final appearance was a clear viscous liquid.

### Example 33

A monomer mixture was prepared by stirring together 96.25 grams of M5, 82.50 grams of M2, 82.55 grams of M3, and 17.20 grams of M8 with 220 grams of IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 65 °C by immersion in a 75 °C water bath. 27.5 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 1.8 grams V50 dissolved in 9.8 grams of water and 9.7 grams of IPA, was added to a 50 ml syringe barrel for delayed addition to the reactor with a FMI QG-6-0SSY metering pump.

At 65 °C, polymerization was initiated by adding a mixture of 1.8 grams V50 in 8.9 grams of Dl water all at once to the stirred reactor. The contents had turned cloudy and turbid within 1 minute, and the temperature had increased rapidly to 73 °C. At 3 minutes, 14 grams of IPA was added to dampen the exotherm as the temperature had climbed to 79 °C. At 7 minutes, the feed pumps were turned on for the delayed addition initiator and monomer mixtures as the temperature dropped slightly to 77 °C. The monomer mixture had been adjusted to deliver in 60 minutes and the initiator mixture had been adjusted to deliver in 90 minutes. By the time all the monomer and initiator mixtures had been delivered to the reactor, the temperature was 77 °C and the temperature setting had been increased incrementally to 98 °C. The process was held for an additional 2 hours and 12 minutes.

At this point, the temperature was at 79 °C, and nitrogen sparging was started with a water aspirator attached to apply partial vacuum to remove volatile solvent. After 26 minutes sparging, 322 grams of hot (70-80 °C) DI water was added, and the temperature setting had been increased to 104 °C 10 minutes before. Sparging continued for another 2 hours and 45 minutes and the polymer solution was cooled to room temperature and 3.03 grams of 10% aqueous hydrogen peroxide was added as preservative. The final appearance was a clear viscous liquid.

### Example 34

A monomer mixture was prepared bystirring together 29.04 grams of M1, 13.35 grams of M4, 8.80 grams of M5, 30.41 grams of M6, 85.86 grams of M3, 74.58 grams of M2 and 36.38 grams of M8 with 220 grams of IPA.

The monomer mixture was warmed to 50 °C and 203 grams (40%) were charged to a 1 L glass resin reactor fitted with a stainless steel stirrer and reflux condenser, and then sparged with dry nitrogen for 30 minutes while heating to 64 °C by immersion in a 73 °C water bath. 27.5 grams additional IPA were added to the remaining 305 grams of monomer mixture and poured into a 500 ml Pyrex addition funnel for delayed addition to the reactor with a FMI QG-50-1CKC metering pump. An initiator mixture, consisting of 1.4 grams V50 dissolved in 8.0 grams of water and 7.7 grams of IPA, was added to a 50 ml syringe barrel for delayed addition to the reactor with a FMI QG-6-0SSY metering pump.

At 64 °C, polymerization was initiated by adding a mixture of 1.4 grams V50 in 7.2 grams of Dl water all at once to the stirred reactor. The contents had turned white within 1.5 minute. At 11 minutes, with the temperature at 65 °C, the temp setting was increased to 88 °C. By 13 minutes, setting was increased again to 94 °C, and the feed pumps were turned on for the delayed addition initiator and monomer mixtures. The monomer mixture had been adjusted to deliver in 60 minutes and the initiator mixture had been adjusted to deliver in 90 minutes. By the time all the monomer and initiator mixtures had been delivered to the reactor, the temperature was 76 °C. The process was held for an additional 1 hour and 45 minutes.

At this point, the temperature was at 71 °C and nitrogen sparging was started with a water aspirator attached to apply partial vacuum to remove volatile solvent. After a nearly 1 hour sparge, 50 grams of hot (70-80 °C) DI water was added, turning the contents clear. The final addition of another 277 grams returned the contents to a white color, and the temperature setting had been increased to 104 °C 45 minutes before. Sparging continued for another 2 hours and the polymer solution was cooled to room temperature and 3.05 grams of 10% aqueous hydrogen peroxide was added as preservative. The final appearance was a translucent viscous liquid.

### Example 35

A monomer mixture was prepared bystirring together 86.46 grams of M1, 41.35 grams of M7, 82.50 grams of M6, 68.78 grams of M3, and 17.21 grams of M8 with 220 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 63 °C by immersion in a 70 °C water bath. 27.5 grams additional IPA were added to the remaining 310 grams of monomer mixture and poured into a 500 ml Pyrex addition funnel for delayed addition to the reactor with a FMI QG-50-1CKC metering pump. An initiator mixture, consisting of 1.4 grams V50 dissolved in 8.0 grams of water and 7.7 grams of IPA, was added to a 50 ml syringe barrel for delayed addition to the reactor with a FMI QG-6-0SSY metering pump.

At 63 °C, polymerization was initiated by adding a mixture of 1.4 grams V50 in 7.2 grams of DI water all at once to the stirred reactor. At 4.5 minutes, the temperature was at 63 °C and the setting was increased to 88 °C. At 15 minutes, with the temperature at 67 °C, the delayed initiator mixture was started to induce an exotherm. By 27 minutes, with the temperature still hovering at 67 C, a second initiator mixture of 0.7 grams of V50 and 3.7 grams of DI water was added directly to the reactor charge. At 30.5 minutes, the monomer addition feed was turned on and the temperature was 69 °C. The monomer mixture had been adjusted to deliver in 60 minutes and the initiator mixture had been adjusted to deliver in 90 minutes. By the time all the monomer and initiator mixtures had been delivered to the reactor, the temperature was 79 °C and the temperature setting had been increased stepwise to 98 °C. The process was held for an additional 2 hours and 7 minutes.

At this point, the temperature was at 74 °C and nitrogen sparging was started with a water aspirator attached to apply partial vacuum to remove volatile solvent. After a 1 hour and 20 minute sparge, 328 grams of hot (70-80 °C) DI water was added, and the temperature setting had been increased to 106 °C. Sparging continued for another 2 hours and 40 minutes and the polymer solution was cooled to room temperature and 3.06 grams of 10% aqueous hydrogen peroxidewas added as preservative. The final appearance was a translucent viscous liquid.

### Example 36

A monomer mixture was prepared by stirring together 109.98 grams of M4, 75.63 grams of M3, 75.64 grams of M2, and 17.35 grams of M8 with 220 grams of IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 64 °C by immersion in a 69 °C water bath. 27.5 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 1.4 grams V50 dissolved in 8.0 grams of water and 7.7 grams of IPA, was added to a 50 ml syringe barrel for delayed addition to the reactor with a FMI QG-6-0SSY metering pump.

At 64 °C, polymerization was initiated by adding a mixture of 1.4 grams V50 in 7.2 grams of DI water all at once to the stirred reactor. At 5 minutes, the temperature was at 67 °C and the setting was increased to 92 °C. The delayed initiator and monomer addition mixtures were started as well. The monomer mixture had been adjusted to deliver in 60 minutes and the initiator mixture had been adjusted to deliver in 90 minutes. By the time all the monomer and initiator mixtures had been delivered to the reactor, the temperature was 73 °C and the process was held for an addtional 2 hours and 43 minutes.

At this point, the temperature was at 72 °C and nitrogen sparging was started with a water aspirator attached to apply partial vacuum to remove volatile solvent. The temperature setting was increased to 98 °C. After a 39 minute sparge, 326 grams of hot (70-80 °C) DI water was added, turningthe contents white. Sparging continued for another 2 hours and 23 minutes and the polymer solution was cooled to room temperature and 3.07 grams of 10% aqueous hydrogen peroxide was added as preservative. The final appearance was a translucent viscous liquid.

### Example 37

A monomer mixture was prepared by stirring together 41.26 grams of M7, 89.45 grams of M3, 89.48 grams of M2, and 68.82 grams of M8 with 220 grams of IPA.

The monomer mixture was warmed to 50 °C and 204 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.5 grams additional IPA were added to the remaining 305 grams of monomer mixture and poured into a 500 ml Pyrex addition funnel for delayed addition to the reactor with a FMI QG-50-1 CKC metering pump. An initiator mixture, consisting of 1.5 grams V50 dissolved in 8.5 grams of water and 8.5 grams of IPA, was added to a 50 ml syringe barrel for delayed addition to the reactor with a FMI QG-6-0SSY metering pump.

At 65 °C, polymerization was initiated by adding a mixture of 1.5 grams V50 in 7.7 grams of Dl water all at once to the stirred reactor. At around 2 minutes, the contents had turned cloudy. At 8 minutes, with the temperature at 66 °C, the heat setting was increased to 92 °C and the delayed initiator and monomer addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 79 °C and the process was held for an additional 1 hour and 45 minutes.

At this point, the temperature was at 77 °C and nitrogen sparging was started with a water aspirator attached to apply partial vacuum to remove volatile solvent and 323 grams of hot (70-80 °C) Dl water was added. The temperature setting was increased to 104 °C 14 minutes later. Sparging continued for another 2 hours and 2 minutes and the polymer solution was cooled to room temperature and 3.03 grams of 10% aqueous hydrogen peroxide was added as preservative. The final appearance was an opaque viscous liquid.

### Example 38

A monomer mixture was prepared by stirring together 47.56 grams of M1, 110.05 grams of M4, 27.50 grams of M5, 13.76 grams of M3, 51.52 grams of M2 and 42.97 grams of M8 with 220 grams of IPA.

The monomer mixture was warmed to 50 °C and 205 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.5 grams additional IPA were added to the remaining 308 grams of monomer mixture and poured into a 500 ml Pyrex addition funnel for delayed addition to the reactor with a FMI QG-50-1 CKC metering pump. An initiator mixture, consisting of 1.4 grams V50 dissolved in 7.7 grams of water and 7.4 grams of IPA, was added to a 50 ml syringe barrel for delayed addition to the reactor with a FMI QG-6-0SSY metering pump.

At 65 °C, polymerization was initiated by adding a mixture of 1.4 grams V50 in 7.0 grams of DI water all at once to the stirred reactor. At around 2 minutes, the temperature had soared to 88 °C, and 12.17 g of IPA was added to the contents to cool the reaction. At 4 minutes, with the temperature at 84 °C, the delayed monomer addition mixture was started. Two minutes later, at 79 °C, the initiator feed pump was started. The monomer mixture had been adjusted to deliver in 60 minutes and the initiator mixture had been adjusted to deliver in 90 minutes. By the time all the monomer and initiator mixtures had been delivered to the reactor, the temperature was 75 °C and the process was held for an additional 1 hour and 45 minutes.

At this point, the temperature was at 75 °C and nitrogen sparging was started with a water aspirator attached to apply partial vacuum to remove volatile solvent. The temperature setting was increased to 104 °C 16 minutes later. After a 55 minute sparge, 323 grams of hot (70-80 °C) DI water was added. Sparging continued for another 2 hours and 8 minutes and the polymer solution was cooled to room temperature and 3.06 grams of 10% aqueous hydrogen peroxide was added as preservative. The final appearance was a clear viscous liquid.

### Example 39

A monomer mixture was prepared by stirring together 29.00 grams of M1, 21.27 grams of M7, 20.20 grams of M4, 8.81 grams of M5, 11.95 grams of M6, 85.80 grams of M3, 74.58 grams of M2 and 34.72 grams of M8 with 220 grams of IPA.

The monomer mixture was warmed to 50 °C and 203 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.5 grams additional IPA were added to the remaining 305 grams of monomer mixture and poured into a 500 ml Pyrex addition funnel for delayed addition to the reactor with a FMI QG-50-1 CKC metering pump. An initiator mixture, consisting of 1.4 grams V50 dissolved in 8.0 grams of water and 7.7 grams of IPA, was added to a 50 ml syringe barrel for delayed addition to the reactor with a FMI OG-6-0SSY metering pump.

At 65 °C, polymerization was initiated by adding a mixture of 1.4 grams V50 in 7.2 grams of Dl water all at once to the stirred reactor. At around 1 minute, the contents had turned white. By 12 minutes, with little heat generated, the temperature setting was increased to 92 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 79 °C, the setting was raised to 98 °C a minute later, and the process was held for an additional 2 hours and 32 minutes.

At this point, the temperature was at 72 °C and nitrogen sparging was started with a water aspirator attached to apply partial vacuum to remove volatile solvent. The temperature setting was increased to 108 °C incrementally. After a 13 minute sparge, 322 grams of hot (70-80 °C) DI water was added. Sparging continued for another 2 hours and 15 minutes and the polymer solution was cooled to room temperature and 3.04 grams of 10% aqueous hydrogen peroxide was added as preservative. The final appearance was a pearlescent viscous liquid.

### Example 40

A monomer mixture was prepared by stirring together 20.61 grams of M7, 82.50 grams of M6,158.13 grams of M3 and 17.60 grams of M8 with 220 grams of IPA.

The monomer mixture was warmed to 50 °C and 209 grams (40%) were charged to a 1 L glass resin reactor fitted with a stainless steel stirrer and reflux condenser, and then sparged with dry nitrogen for 30 minutes while heating to 66 °C by immersion in a 71 °C water bath. 27.7 grams additional IPA were added to the remaining 300 grams of monomer mixture and poured into a 500 ml Pyrex addition funnel for delayed addition to the reactor with a FMI QG-50-1CKC metering pump. An initiator mixture, consisting of 1.4 grams V50 dissolved in 8.0 grams of water and 7.7 grams of IPA, was added to a 50 ml syringe barrel for delayed addition to the reactor with a FMI QG-6-0SSY metering pump.

At 66 °C, polymerization was initiated by adding a mixture of 1.4 grams V50 in 7.2 grams of DI water all at once to the stirred reactor. At around 6 minutes after that addition, at the plateau of a mild exotherm, the delayed addition monomer and initiator mixtures were started and the temperature setting was raised to 94 °C. The monomer mixture had been adjusted to deliver in 60 minutes and the initiator mixture had been adjusted to deliver in 90 minutes. By the time all the monomer and initiator mixtures had been delivered to the reactor, the temperature was 78 °C and the contents had a tan/beige color. The process was held for an additional 1 hours and 27 minutes.

At this point, the temperature was at 78 °C and nitrogen sparging was started with a water aspirator attached to apply partial vacuum to remove volatile solvent. The temperature setting was increased to 104 °C incrementally 19 minutes later. After a 43 minute sparge, 323 grams of hot (70-80 °C) DI water was added. Sparging continued for another 2 hours and 24 minutes and the polymer solution was cooled to room temperature and 3.05 grams of 10% aqueous hydrogen peroxide was added as preservative. The final appearance was an opaqueviscous liquid.

### Example 41

A monomer mixture was prepared by stirring together 41.36 grams of M7, 96.25 grams of M4, 103.13 grams of M3 and 43.06 grams of M8 with 220 grams of IPA.

The monomer mixture was warmed to 50 °C and 201 grams (40%) were charged to a 1 L glass resin reactor fitted with a stainless steel stirrer and reflux condenser, and then sparged with dry nitrogen for 30 minutes while heating to 66 °C by immersion in a 77 °C water bath. 27.5 grams additional IPA were added to the remaining 302 grams of monomer mixture and poured into a 500 ml Pyrex addition funnel for delayed addition to the reactor with a FMI QG-50-1CKC metering pump. An initiator mixture, consisting of 1.4 grams V50 dissolved in 8.0 grams of water and 7.7 grams of IPA, was added to a 50 ml syringe barrel for delayed addition to the reactor with a FMI QG-6-0SSY metering pump.

At 66 °C, polymerization was initiated by adding a mixture of 1.4 grams V50 in 7.2 grams of DI water all at once to the stirred reactor. Between 2-3 minutes after addition, the contents had turned white/turbid. By 11 minutes, with the temperature at 76 °C, the temperature setting was increased to 94 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 76 °C, and the process was held for an addtional 1 hour and 44 minutes.

At this point the temperature was at 76 °C and nitrogen sparging was started with a water aspirator attached to apply partial vacuum to remove volatile solvent. The temperature setting was increased to 104 °C 13 minutes later. After a 41 minute sparge, 322 grams of hot (70-80 °C) Dl water was added. Sparging continued for another 2 hours and 17 minutes and the polymer solution was cooled to room temperature and 3.07 grams of 10% aqueous hydrogen peroxide was added as preservative. The final appearance was a pearlescent viscous liquid.

### Example 42

A monomer mixture was prepared by stirring together 82.50 grams of M6, 103.14 grams of M3, 34.39 grams of M2 and 68.79 grams of M8 with 220 grams of IPA.

The monomer mixture was warmed to 50 °C and 204 grams (40%) were charged to a 1 L glass resin reactor fitted with a stainless steel stirrer and reflux condenser, and then sparged with dry nitrogen for 30 minutes while heating to 65 °C by immersion in a 69 °C water bath. 27.6 grams additional IPA were added to the remaining 305 grams of monomer mixture and poured into a 500 ml Pyrex addition funnel for delayed addition to the reactor with a FMI QG-50-1CKC metering pump. An initiator mixture, consisting of 1.5 grams V50 dissolved in 8.0 grams of water and 8.0 grams of IPA, was added to a 50 ml syringe barrel for delayed addition to the reactor with a FMI QG-6-0SSY metering pump.

At 65 °C, polymerization was initiated by adding a mixture of 1.5 grams V50 in 7.3 grams of DI water all at once to the stirred reactor. Between 1-2 minutes, the contents had become turbid. By 6 minutes, with the temperature at 66 °C, the temperature setting was increased to 96 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 77 °C, and the process was held for an additional 1 hour and 18 minutes.

At this point, the temperature was at 75 °C and nitrogen sparging was started with a water aspirator attached to apply partial vacuum to remove volatile solvent. After a 1 hour and 7 minute sparge, 323 grams of hot (70-80 °C) Dl water was added and the temperature setting was increased to 104 °C. Sparging continued for another 2 hours and 25 minutes and the polymer solution was cooled to room temperature and 3.04 grams of 10% aqueous hydrogen peroxide was added as preservative. The final appearance was a translucent viscous liquid.

### Example 43

A monomer mixture was prepared by stirring together 17.29 grams of M 1, 41.25 grams of M7, 6.87 grams of M4, 6.88 grams of M5, 82.50 grams of M6, 27.50 grams of M3, 41.40 grams of M2 and 68.75 grams of M8 with 220 grams of IPA.

The monomer mixture was warmed to 50 °C and 205 grams (40%) were charged to a 1 L glass resin reactor fitted with a stainless steel stirrer and reflux condenser, and then sparged with dry nitrogen for 30 minutes while heating to 64 °C by.immersion in a 69 °C water bath. 27.6 grams additional IPA were added to the remaining 307 grams of monomer mixture and poured into a 500 ml Pyrex addition funnel for delayed addition to the reactor with a FMI QG-50-1CKC metering pump. An initiator mixture, consisting of 1.7 grams V50 dissolved in 9.3 grams of water and 8.8 grams of IPA, was added to a 50 ml syringe barrel for delayed addition to the reactor with a FMI QG-6-0SSY metering pump.

At 64 °C, polymerization was initiated by adding a mixture of 1.7 grams V50 in 8.3 grams of DI water all at once to the stirred reactor. Between 2-3 minutes, the contents had become turbid. By 10 minutes, with the temperature at 67 °C, the temperature setting was increased to 95 °C and the delayed monomer and initiator addition mixtures were started. The monomer mixture had been adjusted to deliver in 60 minutes and the initiator mixture had been adjusted to deliver in 90 minutes. At 1 hour and 4 minutes, the monomer feed had ended and the heater was turned off as the contents began to boil. 6 minutes later the heat was turned back on at a setting of 84 °C. By the time the initiator mixture had been delivered to the reactor, the temperature was 66 °C, the setting was increased to 89 °C and the process was held for an additional 1 hour and 56 minutes.

At this point, the temperature was at 73 °C and nitrogen sparging was started with a water aspirator attached to apply partial vacuum to remove volatile solvent. After a 38 minute sparge, 322 grams of hot (70-80 °C) DI water was added and the temperature setting was increased to 104 °C. The viscosity has also increased. Sparging continued for another 2 hours and 27 minutes and the polymer solution was cooled to room temperature and 3.05 grams of 10% aqueous hydrogen peroxide was added as preservative. The final appearance was a translucent viscous liquid.

### Example 44

A monomer mixture was prepared by stirring together 77.80 grams of M1, 13.74 grams of M7, 109.98 grams of M4, 13.75 grams of M6, 13.76 grams of M3, 48.11 grams of M2 and 17.25 grams of M8 with 220 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 62 °C by immersion in a 73 °C water bath. 27.5 grams additional IPA were added to the remaining 309 grams of monomer mixture and poured into a 500 ml Pyrex addition funnel for delayed addition to the reactor with a FMI QG-50-1CKC metering pump. An initiator mixture, consisting of 1.3 grams V50 dissolved in 7.4 grams of water and 7.2 grams of IPA, was added to a 50 ml syringe barrel for delayed addition to the reactor with a FMI QG-6-0SSY metering pump.

At 62 °C, polymerization was initiated by adding a mixture of 1.3 grams V50 in 6.9 grams of DI water all at once to the stirred reactor. By 8 minutes, when the temperature had climbed to 67 °C, the temperature setting was increased to 92 °C and the delayed monomer and initiator addition mixtures were started. The monomer mixture had been adjusted to deliver in 60 minutes and the initiator mixture had been adjusted to deliver in 90 minutes. At 30 minutes the temperature setting was increased to 96 °C and 29 minutes later, white particles were observed floating in the batch. By the time all the monomer and initiator mixtures had been delivered to the reactor, the temperature was 70 °C, and the process was held for an additional 1 hour and 47 minutes.

At this point, the temperature was at 72 °C and nitrogen sparging was started with a water aspirator attached to apply partial vacuum to remove volatile solvent. The temperature setting was increased to 104 °C. After a 1 hour and 29 minute sparge, 320 grams of hot (70-80 °C) DI water was added. Sparging continued for another 1 hour and 28 minutes and the polymer solution was cooled to room temperature and 3.03 grams of 10% aqueous hydrogen peroxide was added as preservative. The final appearance was a pearlescent viscous liquid.

### Example 45

A monomer mixture was prepared by stirring together 87.31 grams of M1, 16.50 grams of M7, 6.87 grams of M6, 58.44 grams of M3, 103.16 grams of M2 and 25.78 grams of M8 with 220 grams of IPA.

The monomer mixture was warmed to 50 °C and 207 grams (40%) were charged to a 1 L glass resin reactor fitted with a stainless steel stirrer and reflux condenser, and then sparged with dry nitrogen for 30 minutes while heating to 64 °C by immersion in a 70 °C water bath. 27.7 grams additional IPA were added to the remaining 311 grams of monomer mixture and poured into a 500 ml Pyrex addition funnel for delayed addition to the reactor with a FMI QG-50-1CKC metering pump. An initiator mixture, consisting of 1.4 grams V50 dissolved in 7.4 grams of water and 7.4 grams of IPA, was added to a 50 ml syringe barrel for delayed addition to the reactor with a FMI QG-6-0SSY metering pump.

At 64 °C, polymerization was initiated by adding a mixture of 1.4 grams V50 in 7.1 grams of DI water all at once to the stirred reactor. In less than 1 minute, the contents had become turbid. By 11 minutes, with the temperature at 66 °C, the temperature setting was increased to 96 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 77 °C, and the process was held for an additional 1 hour and 38 minutes.

At this point, the temperature was at 74 °C and nitrogen sparging was started with a water aspirator attached to apply partial vacuum to remove volatile solvent. The temperature setting was raised to 104 °C a minute later. After a 1 hour and 47 minute sparge, 306 grams of hot (70-80 °C) DI water was added. The heater, which had stopped functioning, was set at 102 °C. Sparging continued for another 1 hour and 30 minutes and the polymer solution was cooled to room temperature and 3.05 grams of 10% aqueous hydrogen peroxide was added as preservative. The final appearance was an opaque viscous liquid.

### Example 46

A monomer mixture was prepared by stirring together 82.12 grams of M1, 41.27 grams of M7, 48.30 grams of M5, 65.30 grams of M3, and 68.72 grams of M8 with 220 grams of IPA.

The monomer mixture was warmed to 50 °C and 210 grams (40%) were charged to a 1 L glass resin reactor fitted with a stainless steel stirrer and reflux condenser, and then sparged with dry nitrogen for 30 minutes while heating to 64 °C by immersion in a 69 °C water bath. 27.5 grams additional IPA were added to the remaining 315 grams of monomer mixture and poured into a 500 ml Pyrex addition funnel for delayed addition to the reactor with a FMI QG-50-1CKC metering pump. An initiator mixture, consisting of 1.8 grams V50 dissolved in 9.7 grams of water and 9.7 grams of IPA, was added to a 50 ml syringe barrel for delayed addition to the reactor with a FMI QG-6-0SSY metering pump.

At 64 °C, polymerization was initiated by adding a mixture of 1.8 grams V50 in 8.8 grams of DI water all at once to the stirred reactor. Within about 2 minutes, the contents had turned white. By 7 minutes, with the temperature having increased to 73 °C, the temperature setting was increased to 94 °C and the delayed monomer addition mixture was started. One minute later, the delayed initiator addition mixture was started. The monomer mixture had been adjusted to deliver in 60 minutes and the initiator mixture had been adjusted to deliver in 90 minutes. By the time all the monomer and initiator mixtures had been delivered to the reactor, the temperature was 76 °C, and the temperature setting was increased to 96 °C 1.5 hours before. The process was held for an additional 1 hourand 55 minutes.

At this point the temperature was at 74 °C and nitrogen sparging was started with a water aspirator attached to apply partial vacuum to remove volatile solvent. The temperature setting was raised to 104 °C 43 minutes later and the contents had a pinkish hue. After a 1 hour and 3 minute sparge, 303 grams of hot (70-80 °C) DI water was added. Sparging continued for another 2 hours and 1 minute and the polymer solution was cooled to room temperature and 3.03 grams of 10% aqueous hydrogen peroxide was added as preservative. The final appearance was a pearlescent viscous liquid.

### Example 47

A monomer mixture was prepared by stirring together 56.30 grams of M1, 41.26 grams of M7, 134.06 grams of M3 and 68.77 grams of M8 with 220 grams of IPA.

The monomer mixture was warmed to 50 °C and 208 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.6 grams additional IPA were added to the remaining 312 grams of monomer mixture and poured into a 500 ml Pyrex addition funnel for delayed addition to the reactor with a FMI QG-50-1CKC metering pump. An initiator mixture, consisting of 1.4 grams V50 dissolved in 7.7 grams of water and 7.7 grams of IPA, was added to a 50 ml syringe barrel for delayed addition to the reactor with a FMI QG-6-0SSY metering pump.

At 65 °C, polymerization was initiated by adding a mixture of 1.4 grams V50 in 7.2 grams of DI water all at once to the stirred reactor. Between 6-7 minutes, the contents had turned white. By 9 minutes, with the temperature at 71 °C, the temperature setting was increased to 98 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 73 °C. The process was held for an additional 1 hour and 45 minutes.

At this point, the temperature was at 74 °C and nitrogen sparging was started with a water aspirator attached to apply partial vacuum to remove volatile solvent. The temperature setting was raised to 104 °C 18 minutes later. At this time, 321 grams of hot (70-80 °C) DI water was added. Sparging continued for another 3 hours and 5 minutes and the polymer solution was cooled to room temperature and 3.07 grams of 10% aqueous hydrogen peroxide was added as preservative. The final appearance was a pearlescent viscous liquid.

### Example 48

A monomer mixture was prepared by stirring together 41.26 grams of M7, 96.26 grams of M5, 92.82 grams of M3, 30.90 grams of M2 and 17.30 grams of M8 with 220 grams of IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.8 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 2.2 grams V50 dissolved in 12.1 grams of water and 12.1 grams of IPA, was added to a 50 ml syringe barrel for delayed addition to the reactor with a FMI QG-6-0SSY metering pump.

At 65 °C, polymerization was initiated by adding a mixture of 2.2 grams V50 in 11.0 grams of DI water all at once to the stirred reactor. At 9 minutes, with the temperature slightly higher at 66 °C, the temperature setting was increased to 96 °C and the delayed monomer and initiator addition mixtures were started. The monomer mixture had been adjusted to deliver in 60 minutes and the initiator mixture had been adjusted to deliver in 90 minutes. At around 1 hour, with the temperature at 74 degrees, the contents had taken on an orange hue. By the time all the monomer and initiator mixtures had been delivered to the reactor, the temperature was 81 °C. The process was held for an additional 1 hourand 38 minutes.

At this point, the temperature was at 75 °C and nitrogen sparging was started with a water aspirator attached to apply partial vacuum to remove volatile solvent. The temperature setting was raised to 104 °C. After a 1 hour and 24 minute sparge, 326 grams of hot (70-80 °C) DI water was added. Sparging continued for another 2 hours and 9 minutes and the polymer solution was cooled to room temperature and 3.12 grams of 10% aqueous hydrogen peroxide was added as preservative. The final appearance was a clear viscous liquid.

### Example 49

A monomer mixture was prepared bystirring together 42.00 grams of M7, 55.00 grams of M4,41.25 grams of M6, 61.88 grams of M3, 61.91 grams of M2 and 18.19 grams of M8 with 220 grams of IPA.

The monomer mixture was warmed to 50 °C and 199 grams (40%) were charged to a 1 L glass resin reactor fitted with a stainless steel stirrer and reflux condenser, and then sparged with dry nitrogen for 30 minutes while heating to 63 °C by immersion in a 70 °C water bath. 27.6 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 1.7 grams V50 dissolved in 9.4 grams of water and 8.8 grams of IPA, was added to a 50 ml syringe barrel for delayed addition to the reactor with a FMI QG-6-0SSY metering pump.

At 63 °C, polymerization was initiated by adding a mixture of 1.7 grams V50 in 8.3 grams of DI water all at once to the stirred reactor. At 7 minutes, with the temperature slightly higher at 65 °C, the temperature setting was increased to 96 °C and the delayed monomer and initiator addition mixtures were started. The monomer mixture had been adjusted to deliver in 60 minutes and the initiator mixture had been adjusted to deliver in 90 minutes. At around 1 hour, with the temperature at 74 degrees, the contents had taken on an orange hue, which turned to yellow about 40 minutes later. By the time all the monomer and initiator mixtures had been delivered to the reactor, the temperature was 75 °C. The process was held for an additional 1 hour and 23 minutes.

At this point, the temperature was at 74 °C and nitrogen sparging was started with a water aspirator attached to apply partial vacuum to remove volatile solvent. The temperature setting was raised to 104 °C. After a 41 minute sparge, 322 grams of hot (70-80 °C) DI water was added. Sparging continued for another 2 hours and 3 minutes and the polymer solution was cooled to room temperature and 3.07 grams of 10% aqueous hydrogen peroxide was added as preservative. The final appearance was a translucent viscous liquid.

### Example 50

A monomer mixture was prepared by stirring together 41.25 grams of M7, 154.69 grams of M3, 51.58 grams of M2, and 34.41 grams of M8 with 220 grams of IPA.

The monomer mixture was warmed to 50 °C and 201 grams (40%) were charged to a 1 L glass resin reactor fitted with a stainless steel stirrer and reflux condenser, and then sparged with dry nitrogen for 30 minutes while heating to 65 °C by immersion in a 70 °C water bath. 27.8 grams additional IPA were added to the remaining 299 grams of monomer mixture and poured into a 500 ml Pyrex addition funnel for delayed addition to the reactor with a FMI QG-50-1CKC metering pump. An initiator mixture, consisting of 1.5 grams V50 dissolved in 8.5 grams of water and 8.5 grams of IPA, was added to a 50 ml syringe barrel for delayed addition to the reactor with a FMI QG-6-0SSY metering pump.

At 65 °C, polymerization was initiated by adding a mixture of 1.5 grams V50 in 7.7 grams of DI water all at once to the stirred reactor. At 9 minutes, with the temperature slightly higher at 67 °C, the temperature setting was increased to 96 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 73 °C. The process was held for an additional 1 hour and 23 minutes.

At this point, the temperature was at 74 °C and nitrogen sparging was started with a water aspirator attached to apply partial vacuum to remove volatile solvent. The temperature setting was raised to 104 °C. After a 42 minute sparge, 322 grams of hot (70-80 °C) DI water was added. Sparging continued for another 2 hours and 47 minutes and the polymer solution was cooled to room temperature and 3.04 grams of 10% aqueous hydrogen peroxide was added as preservative. The final appearance was an opaque viscous liquid.

### Example 51

A monomer mixture was prepared bystirring together 57.99 grams of M1, 15.19 grams of M7, 26.69 grams of M4, 17.60 grams of M5, 23.85 grams of M6, 68.63 grams of M3, 46.06 grams of M2 and 38.98 grams of M8 with 220 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 64 °C by immersion in a 70 °C water bath. 28.8 grams additional IPA were added to the remaining 309 grams of monomer mixture and poured into a 500 ml Pyrex addition funnel for delayed addition to the reactor with a FMI QG-50-1CKC metering pump. An initiator mixture, consisting of 1.4 grams V50 dissolved in 7.7 grams of water and 7.7 grams of IPA, was added to a 50 ml syringe barrel for delayed addition to the reactor with a FMI QG-6-0SSY metering pump.

At 64 °C, polymerization was initiated by adding a mixture of 1.4 grams V50 in 8.3 grams of DI water all at once to the stirred reactor. In 1 minute, the contents had turned white. At 5 minutes, with the temperature at 66 °C, the temperature setting was raised to 96 °C. At 7 minutes, with the temperature slightly higher at 67 °C, the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 70 °C. The process was held for an additional 1 hour and 23 minutes.

At this point, the temperature was at 74 °C and nitrogen sparging was started with a water aspirator attached to apply partial vacuum to remove volatile solvent. The temperature setting was raised to 104 °C. After a 41 minute sparge, 322 grams of hot (70-80 °C) DI water was added. Sparging contnued for another 2 hours and 48 minutes and the polymer solution was cooled to room temperature and 3.08 grams of 10% aqueous hydrogen peroxide was added as preservative. The final appearance was an opaque viscous liquid.

### Example 52

A monomer mixture was prepared by stirring together 91.25 grams of M1, 75.65 grams of M4, 72.19 grams of M3, and 68.75 grams of M8 with 220 grams of IPA.

The monomer mixture was warmed to 50 °C and 211 grams (40%) were charged to a 1 L glass resin reactor fitted with a stainless steel stirrer and reflux condenser, and then sparged with dry nitrogen for 30 minutes while heating to 63 °C by immersion in a 70 °C water bath. 28.3 grams additional IPA were added to the remaining 316 grams of monomer mixture and poured into a 500 ml Pyrex addition funnel for delayed addition to the reactor with a FMI QG-50-1CKC metering pump. An initiator mixture, consisting of 1.0 grams V50 dissolved in 5.5 grams of water and 5.5 grams of IPA, was added to a 50 ml syringe barrel for delayed addition to the reactor with a FMI QG-6-0SSY metering pump.

At 63 °C, polymerization was initiated by adding a mixture of 1.0 grams V50 in 5.5 grams of DI water all at once to the stirred reactor. In 1 minute, the contents had turned cloudy, and white in 2 minutes. At 5.5 minutes, with the temperature at 71 °C, the temperature setting was raised to 96 °C. At 7 minutes, the temperature had risen to 71 °C, and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 74 °C. The process was held for an additional 2 hours and 2 minutes.

At this point, the temperature was at 73 °C and nitrogen sparging was started with a water aspirator attached to apply partial vacuum to remove volatile solvent. The temperature setting was raised to 104 °C. After a 49 minute sparge, 322 grams of hot (70-80 °C) DI water was added. Sparging continued for another 2 hours and 14 minutes and the polymer solution was cooled to room temperature and 3.30 grams of 10% aqueous hydrogen peroxide was added as preservative. The final appearance was a pearlescent viscous liquid.

### Example 53

A monomer mixture was prepared bystirring together 104.38 grams of M9, 55.01 grams of M5, 103.18 grams of M3, and 17.48 grams of M8 with 220 grams of IPA.

The monomer mixture was warmed to 50 °C and 200 grams (40%) were charged to a 1 L glass resin reactor fitted with a stainless steel stirrer and reflux condenser, and then sparged with dry nitrogen for 30 minutes while heating to 64 °C by immersion in a 70 °C water bath. 27.6 grams additional IPA were added to the remaining 300 grams of monomer mixture and poured into a 500 ml Pyrex addition funnel for delayed addition to the reactor with a FMI QG-50-1CKC metering pump. An initiator mixture, consisting of 1.3 grams V50 dissolved in 7.3 grams of water and 7.3 grams of IPA, was added to a 50 ml syringe barrel for delayed addition to the reactor with a FMI QG-6-0SSY metering pump.

At 64 °C, polymerization was initiated by adding a mixture of 1.3 grams V50 in 7.7 grams of DI water all at once to the stirred reactor. In 4 minutes, the contents had become turbid. At 5 minutes, with the temperature at 67 °C, the temperature setting was raised to 96 °C and the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 74 °C. The process was held for an additional 1 hour and 57 minutes.

At this point, the temperature was at 73 °C and nitrogen sparging was started with a water aspirator attached to apply partial vacuum to remove volatile solvent. The temperature setting was raised to 104 °C. After a 52 minute sparge, 322 grams of hot (70-80 °C) DI water was added. Sparging continued for another 2 hours and 13 minutes and the polymer solution was cooled to room temperature and 3.03 grams of 10% aqueous hydrogen peroxide was added as preservative. The final appearance was a pearlescent viscous liquid.

### Example 54

A monomer mixture was prepared by stirring together 55.26 grams of M1, 41.26 grams of M7, 6.89 grams of M6, 58.49 grams of M3, 103.15 grams of M2 and 25.80 grams of M8 with 220 grams of IPA.

The monomer mixture was warmed to 50 °C and 205 grams (40%) were charged to a 1 L glass resin reactor fitted with a stainless steel stirrer and reflux condenser, and then sparged with dry nitrogen for 30 minutes while heating to 63 °C by immersion in a 67 °C water bath. 27.8 grams additional IPA were added to the remaining 307 grams of monomer mixture and poured into a 500 ml Pyrex addition funnel for delayed addition to the reactor with a FMI QG-50-1CKC metering pump. An initiator mixture, consisting of 1.5 grams V50 dissolved in 8.5 grams of water and 8.5 grams of IPA, was added to a 50 ml syringe barrel for delayed addition to the reactor with a FMI QG-6-0SSY metering pump.

At 63 °C, polymerization was initiated by adding a mixture of 1.5 grams V50 in 7.7 grams of DI water all at once to the stirred reactor. In 1 minute, the contents had turned cloudy. At 6.5 minutes, with the temperature at 65 °C, the temperature setting was raised to 96 °C. At 9 minutes and 65 °C, the delayed monomer and initiator addition mixtures were started. The monomer mixture had been adjusted to deliver in 60 minutes and the initiator mixture had been adjusted to deliver in 90 minutes. By the time all the monomer and initiator mixtures had been delivered to the reactor, the temperature was 77 °C. The process was held for an additional 1 hourand 40 minutes.

At this point, the temperature was at 75 °C and nitrogen sparging was started with a water aspirator attached to apply partial vacuum to remove volatile solvent. The temperature setting was raised to 104 °C one minute later. After a 41 minute sparge, 322 grams of hot (70-80 °C) DI water was added. Sparging continued for another 2 hours and 18 minutes and the polymer solution was cooled to room temperature and 3.03 grams of 10% aqueous hydrogen peroxide was added as preservative. The final appearance was an opaque viscous liquid.

### Example 55

A monomer mixture was prepared by stirring together 52.5 grams M4, 45.0 grams M3 and 12.1 grams M8 with 91.6 grams IPA.

The monomer mixture was charged to a 1 L glass resin reactor fitted with a stainless steel stirrer and reflux condenser, and then sparged with dry nitrogen for 15 minutes while heating to 68 °C by immersion in a 73 °C water bath. An initiator mixture, consisting of 0.25 grams V50 dissolved in 2.25 grams of DI water and 2.5 grams IPA, was added all at once to the reactor.

After 2 minutes of temperature rise to 84 °C, 15 grams more isopropanol was added and the temperature dropped to 80 deg, matching the bath temperature.

After an additional 13 minutes, a mixture, consisting of 0.21 grams V50 dissolved in 1.88 grams of DI water and 2.1 grams IPA, was added to the reactor. The reactor contents were then held at 73-81 °C for an additional 100 minutes with an 88 deg bath temperature.

Finally, the batch was cooled to room temperature yielding 172 grams of clear, alcoholic polymer solution where the viscosity measured 1,470 centipoise @60 rpm Brookfield LVT spindle #4. The mixture was easily diluted with 61 grams DI water to yield a more viscous clear polymer solution. The final appearance was a clear viscous liquid.

### Example 56

A monomer mixture was prepared bystirring together 78.0 grams M3, 60.0 grams M5 and 15.1 grams M8 with 220 grams IPA.

The monomer mixture was charged to a 1 L glass resin reactor fitted with a stainless steel stirrer and reflux condenser, and then sparged with dry nitrogen for 15 minutes while heating to 76 °C by immersion in a 86 °C water bath. An initiator mixture, consisting of 0.86 grams V50 dissolved in 3.2 grams of DI water and 0.9 grams IPA, was added all at once to the reactor.

After 2 minutes, the temperature had risen to reflux at 91 °C. After 12 minutes, the clear solution had increased substantially in viscosity and reflux had stopped. The batch was held at 78 °C for an additional 40 minutes followed by a nitrogen sparge under aspirator vacuum. Intermittent additions of DI water totaling 106 grams accompanied solvent removal over a period of 90 minutes. 227 grams of viscous polymer solution was removed at 60 °C which, combined with 75 grams more DI water used to rinse the reactor, yielded about 350 grams of clear liquid. The final appearance was a transparent, highly viscous liquid.

### Example 67

A monomer mixture was prepared by stirring together 86.45 grams of M1, 138.50 grams of M3, 55.00 grams of M5, and 17.29 grams of M8 with 220 grams of IPA.

The monomer mixture was warmed to 50 °C and 206 grams (40%) were charged to a 1 L glass resin reactor fitted with a stainless steel stirrer and reflux condenser, and then sparged with dry nitrogen for 30 minutes while heating to 65 °C by immersion in a 68 °C water bath. 27.5 grams additional IPA were added to the remaining 310 grams of monomer mixture and poured into a 500 ml Pyrex addition funnel for delayed addition to the reactor with a FMI QG-50-1CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 65 °C, polymerization was initiated by adding a mixture of 1.4 grams V50 in 5 grams of DI water all at once to the stirred reactor. In less than 1.5 minutes, the contents of the reactor had turned cloudy and the temperature began to rise slightly. The temperature setting was adjusted to 90 °C at that time. After 3 minutes, with the batch at 68 °C and the exotherm had reached a plateau, the feed pump was turned on for the delayed addition initiator mixture. At 5 minutes with the temperature still at 68 °C, the monomer feed pump was started. The monomer mixture had been adjusted to deliver in 60 minutes and the initiator mixture had been adjusted to deliver in 90 minutes. By the time all the monomer and initiator mixtures had been delivered to the reactor, the temperature was 76 °C and the contents were clear with a pinkish hue. The process was held above 66 °C for an additional 2 hours and 30 minutes.

At this point, the temperature was at 66 °C and the setting increased to 101 °C. The nitrogen sparging was started with a water aspirator attached to apply partial vacuum to remove volatile solvent. After a 1 hour and 10 minutes sparge, 87 grams of hot (70-80 °C) DI water was added with the appearance changing to opaque. The final addition of 238 grams of water left the appearance white/opaque. Sparging continued for another 1 hour and 15 minutes. Finally, the polymer solution was cooled to room temperature and 3.0 grams of 10% aqueous hydrogen peroxide was added as preservative. The final appearance was a translucent viscous liquid.

### Example 58

A monomer mixture was prepared by stirring together 132.6 grams M3, 26.9 grams M4, 16.3 grams M5 and 42.0 grams M8 with 135 grams IPA.

The monomer mixture was warmed to 50 °C and 158 grams (50%) were charged to a 1 L glass resin reactor fitted with a stainless steel stirrer and reflux condenser, and then sparged with dry nitrogen for 20 minutes while heating to 78 °C by immersion in a 83 °C water bath. 30.6 grams additional IPA were added to the remaining 160 grams of monomer mixture, sparged with nitrogen and poured into a 500 ml Pyrex addition funnel for delayed addition to the reactor with a FMI QG-50-1CKC metering pump. An initiator mixture, consisting of 0.8 grams V50 dissolved in 4.1 grams of DI water and 4.1 grams IPA, was added to a 50 ml syringe barrel for delayed addition to the reactor with a FMI QG-6-0SSY metering pump.

Polymerization was started as an initiator mixture, consisting of 0.68 grams V50 dissolved in 3.5 grams of DI water and 3.4 grams IPA, was added all at once to the reactor.

Almost immediately, the contents became first bluish and then turbid white and the temperature was rising rapidly. After 1 minute, the pumps were turned on for both delayed addition initiator and monomer mixtures. The monomer and initiator mixtures had been adjusted to deliver in 60 minutes. The water bath temperature was increased stepwise to 93 °C during the first 20 minutes of delayed addition during which time the reactor temperature leveled off near 82 °C. Ten minutes after both monomer and initiator feeds had run out, 80 grams of hot DI water was added to the turbid solution, turning it transparent. We added 140 grams additional hot water, turning the batch hazy. Then 150 grams more DI water was added which turned the contents turbid again. At 77 °C, nitrogen sparging, accompanied by a partial vacuum, was used to remove solvent vapor over a period of 2 hours followed by cooling to room temperature. The final appearance wasan opaque, viscous liquid.

### Example 59

A monomer mixture was prepared bystirring together 122.4 grams M3, 30.6 grams M4, 21.0 grams M5 and 38.3 grams M8 with 127.5 grams IPA.

The monomer mixture was warmed to 50 °C and 170 grams (50%) were charged to a 1 L glass resin reactor fitted with a stainless steel stirrer and reflux condenser, and then sparged with dry nitrogen for 20 minutes while heating to 76 °C by immersion in a 87 °C water bath. 29.1 grams additional IPA were added to the remaining 170 grams of monomer mixture, sparged with nitrogen and poured into a 500 ml Pyrex addition funnel for delayed addition to the reactor with a FMI QG-50-1 CKC metering pump. An initiator mixture, consisting of 1.1 grams V50 dissolved in 5.5 grams of DI water and 5.5 grams IPA, was added to a 50 ml syringe barrel for delayed addition to the reactor with a FMI QG-6-0SSY metering pump.

Polymerization was started as an initiator mixture, consisting of 0.73 grams V50 dissolved in 3.7 grams of DI water and 3.7 grams IPA, was added all at once to the reactor.

Almost immediately, the contents became first bluish and then turbid white and the temperature was rising rapidly. After 4 minutes, the temperature had stabilized at 84 °C and pumps were turned on for both delayed addition initiator and monomer mixtures. The monomer and initiator mixtures had been adjusted to deliver in 60 minutes. The water bath temperature was increased stepwise to 95 °C during the first 20 minutes of delayed addition. Five minutes after both monomer and initiator feeds had run out, at 77 °C, nitrogen sparging, accompanied by a partial vacuum was started to remove solvent vapor. More initiator solution, 0.2 grams V50 in 5.8 grams water, was added at once followed by a total of 324 grams DI water pumped in over a period of 2 ½ hours while continuing to remove solvent at 75-76 °C. Vacuum and sparging was terminated followed by cooling to room temperature. The final appearance was a translucent, viscous liquid.

### Example 60

A monomer mixture was prepared by stirring together 74.22 grams of M1, 37.22 grams of M7, 41.26 grams of M2, 82.52 grams of M6 and 68.75 grams of M8 with 220 grams of IPA.

The monomer mixture was warmed to 50 °C and 210 grams (40%) were charged to a 1 L glass resin reactor fitted with a stainless steel stirrer and reflux condenser, and then sparged with dry nitrogen for 30 minutes while heating to 66 °C by immersion in a 69 °C water bath. 27.5 grams additional IPA were added to the remaining 314 grams of monomer mixture and poured into a 500 ml Pyrex addition funnel for delayed addition to the reactor with a FMI QG-50-1 CKC metering pump. An initiator mixture, consisting of 1.4 grams V50 dissolved in 4.3 grams of DI water and 11.4 grams MeOH, was added to a 50 ml syringe barrel for delayed addition to the reactor with a FMI QG-6-0SSY metering pump.

At 66 °C, polymerization was initiated by adding a mixture of 1.4 grams V50 in 2.5 grams of DI water and 2.5 grams of MeOH all at once to the stirred reactor. Within 1 minute, the contents of the reactor began rise in temperature and turned cloudy. After 5 minutes, the batch was white and the temperature was raised to a setting of 90 °C. At 8 minutes the contents had risen to 79 °C and pumps were turned on for both delayed addition initiator and monomer mixtures. The monomer mixture had been adjusted to deliver in 60 minutes and the initiator mixture had been adjusted to deliver in 90 minutes. The temperature was at 76-78 °C by the time all the monomer and initiator mixtures had been delivered to the reactor. The contents were viscous and white and the process held above 70 °C for an additional 3 hours.

At this point, the temperature was at 70 °C and the setting increased to 100 °C and nitrogen sparging was started and a water aspirator attached to apply partial vacuum to remove volatile solvent. After 30 minutes sparging, 111 grams of hot (70-80 °C) DI water was added which turned the mixture slightly transparent. The addition of another 227 grams of water turned the batch cloudy/opaque again. Sparging continued for another 1 hour and 40 minutes. Finally, the polymer solution was cooled to room temperature and 3.0 grams of 10% aqueous hydrogen peroxide was added as preservative. The final appearance was a translucent viscous liquid.

### Example 61

A monomer mixture was prepared by stirring together 132.8 grams M3, 40.8 grams M4, and 38.6 grams M8 with 127.5 grams IPA.

The monomer mixture was warmed to 50 °C and 170 grams (50%) were charged to a 1 L glass resin reactor fitted with a stainless steel stirrer and reflux condenser, and then sparged with dry nitrogen for 20 minutes while heating to 71 °C by immersion in a 80 °C water bath. 28.9 grams additional IPA were added to the remaining 169 grams of monomer mixture, sparged with nitrogen and poured into a 500 ml Pyrex addition funnel for delayed addition to the reactor with a FMI QG-50-1 CKC metering pump. An initiator mixture, consisting of 1.2 grams V50 dissolved in 12.0 grams of DI water, was added to a 50 ml syringe barrel for delayed addition to the reactor with a FMI QG-6-0SSY metering pump.

Polymerization was started as an initiator mixture, consisting of 0.6 grams V50 dissolved in 6.0 grams of DI water, was added all at once to the reactor.

Almost immediately, the contents became turbid and white and the temperature rose rapidly. After 3 minutes, the temperature had stabilized at 86 °C and pumps were turned on for both delayed addition initiator and monomer mixtures. The monomer and initiator mixtures had been adjusted to deliver in 60 minutes. The water bath temperature was increased stepwise to 90 °C during the first 20 minutes of delayed addition. Eight minutes after both monomer and initiator feeds had run out, a mixture of 0.34 grams V50 dissolved in 3.1 grams of water was added and the temperature held for 25 minutes at 81 °C. 65 grams of hot DI water was then added to the turbid solution, making it transparent. An additional 321 grams of DI water was added and it became more viscous and milky. Nitrogen sparging, accompanied by a partial vacuum was started to remove solvent vapor for 3 hours at 85 °C. Vacuum and sparging was terminated followed by cooling to room temperature. The final appearance was a translucent, highly viscous liquid.

### Example 62

A monomer mixture was prepared by stirring together 132.7 grams M3, 40.8 grams M5, and 38.3 grams M8 with 140.2 grams IPA.

The monomer mixture was charged to a 1 L glass resin reactor fitted with a stainless steel stirrer and reflux condenser, and then sparged with dry nitrogen for 30 minutes while heating to 65 °C by immersion in a 70 °C water bath. An initiator mixture was prepared consisting of 2.14 grams V50 dissolved in 11.4 grams of DI water and 11.4 grams IPA was prepared. Polymerization was started by adding 13.1 of the initiator mixture, all at once, to the reactor.

Almost immediately, the contents became bluish-white and turbid as the temperature rose rapidly. After 3 minutes, with solvent reflux, the temperature had stabilized at 86 °C and the remainder of the initiator mixture was added all at once. The water bath temperature was increased stepwise to 90 °C during the next 20 minutes. 87 grams of hot DI water was then added to the turbid solution, making it transparent. An additional 215 grams of DI water was added and it became more viscous and milky. The product was poured into an open pan and kept in a vacuum oven at 60 °C for 14 hours. The final appearance was a translucent, highly viscous liquid.

### Example 63

A monomer mixture was prepared by stirring together 134.0 grams M3, 41.2 grams M5, and 38.6 grams M8 with 128.8 grams IPA.

The monomer mixture was warmed to 50 °C and 170 grams (50%) were charged to a 1 L glass resin reactor fitted with a stainless steel stirrer and reflux condenser, and then sparged with dry nitrogen for 20 minutes while heating to 66 °C by immersion in a 70 °C water bath. The remaining 169 grams of monomer mixture was diluted with 15.9 grams IPA and 16.7 grams DI water, sparged with nitrogen and poured into a 500 ml Pyrex addition funnel for delayed addition to the reactor with a FMI QG-50-1CKC metering pump. An initiator mixture, consisting of 1.28 grams V50 dissolved in 6.8 grams of IPA and 6.8 grams of DI water, was added to a 50 ml syringe barrel for delayed addition to the reactor with a FMI QG-6-0SSY metering pump.

Polymerization was started as an initiator mixture, consisting of 0.64 grams V50 dissolved in 3.4 grams of IPA and 3.4 grams of DI water, was added all at once to the reactor.

Within 1 minute, the contents started turning bluish and turbid and the temperature started rising. After 3 minutes, the temperature was 65 °C and pumps were turned on for both delayed addition initiator and monomer mixtures. The monomer and initiator mixtures had been adjusted to deliver in 60 minutes. The water bath temperature was increased stepwise to reach 85 °C during the monomer delayed addition. 30 minutes after both monomer and initiator feeds had run out, 79 grams of hot DI water was then added to the bluish white solution, turning it transparent. An additional 219 grams of DI water was added and it became more viscous and milky. Nitrogen sparging, accompanied by a partial vacuum was started to remove solvent vapor for 1 hour at 75 °C. Vacuum and sparging was terminated followed by cooling to room temperature. After sitting for 2 days, a nearly transparent aqueous layer settled out and 99 grams was removed.

The remaining viscous polymer solution was reheated and vacuum stripped at 55 °C for 90 minutes, and cooled again yielding 337 grams. The final appearance was a translucent, viscous fluid.

### Example 64

A monomer mixture was prepared by stirring together 103.1 grams M3, 9.2 grams M4, 6.3 grams M7, and 129.1 grams M1 with 165 grams IPA.

To half (206 grams) of the monomer mixture, 17.2 grams M8 was added, sparged with nitrogen, warmed to 50 °C and poured into a 500 ml Pyrex addition funnel for delayed addition to the reactor with a FMI QG-50-1CKC metering pump. The remaining half of the monomer mixture was diuted further with 38.5 grams IPA and charged to a 1 L glass resin reactor fitted with a stainless steel stirrer and reflux condenser, and then sparged with dry nitrogen for 20 minutes while heating to 63 °C by immersion in a 72 °C water bath. A starting mixture was prepared, consisting of 1.1 grams V50 dissolved in 8.9 grams of DI water and 17.4 grams M8.

A delayed addition initiator mixture was prepared, consisting of 1.50 grams V50 dissolved in 8.25 grams of DI water and 8.25 grams IPA. The mixture was added to a 50 ml syringe barrel for delayed addition to the reactor with a FMI QG-6-0SSY metering pump, adjusted to deliver it in 40 minutes.

Polymerization was started by adding the starting mixture all at once to the reactor. Within 2 minutes, the contents became first bluish and then turbid white and the temperature rose to 64 °C. After 4 minutes, the pumps were turned on for both delayed addition initiator and monomer mixtures. The monomer mixture pump had been adjusted to deliver in 40 minutes. The water bath temperature was increased stepwise to 84 °C during the first 20 minutes of delayed addition during which time the reactor temperature leveled off near 75 °C. 15 minutes after both monomer and initiator feeds had run out, at 79 °C, nitrogen sparging, accompanied by a partial vacuum, was used to remove solvent over a period of 80 minutes during which time a total of 383 grams of hot DI water was added. While the first 60 grams of water added turned the batch transparent, the remaining water made it translucent and then quite turbid. After cooling overnight, the viscous and uniformly turbid liquid was re-heated and vacuum with nitrogen sparge was continued at 75-78 °C for an additional 5 hours during which time an additional 480 grams of hot DI water was added to reduce viscosity. The final appearance at 20 °C was an opaque, non-flowing paste.

### Example 65

A monomer mixture was prepared bystirring together 103.2 grams M3, 103.2 grams M2, and 55.0 grams M6 with 165 grams IPA.

To half (213 grams) of the monomer mixture, 8.6 grams M8 was added, sparged with nitrogen, warmed to 50 °C and poured into a 500 ml Pyrex addition funnel for delayed addition to the reactor with a FMI QG-50-1CKC metering pump. The remaining half of the monomer mixture was diluted further with 28.6 grams IPA and charged to a 1 L glass resin reactor fitted with a stainless steel stirrer and reflux condenser, and then sparged with dry nitrogen for 20 minutes while heating to 63 °C by immersion in a 73 °C water bath. A starting initiator mixture was prepared, consisting of 1.24 grams V50 dissolved in 8.8 grams of DI water and 8.6 grams M8.

A delayed addition initiator mixture was prepared, consisting of 1.9 grams V50 dissolved in 10.3 grams of DI water and 10.3 grams IPA. The mixture was added to a 50 ml syringe barrel for delayed addition to the reactor with a FMI QG-6-0SSY metering pump, adjusted to deliver it in 60 minutes.

Polymerization was started by adding the starting mixture all at once to the reactor. The contents soon became turbid and the temperature rose slowly over 30 minutes increasing in viscosity, to 71 °C. The water bath temperature was increased stepwise to 80 °C while 6.4 grams more DI water was added to the batch. 80 minutes after starting, 0.25 grams V50 dissolved in 2.5 grams DI water was added to the batch to re-start polymerization and the pumps were turned on for both delayed addition initiator and monomer mixtures. The monomer mixture pump had been adjusted to deliver in 60 minutes. 15 minutes after both monomer and initiator feeds had run out, at 81 °C, nitrogen sparging, accompanied by a partial vacuum, was used to remove solvent over a period of 90 minutes during which time a total of 477 grams of hot DI water was added to the initially translucent polymer mixture. The water additions only made the mixture more turbid and opaque. After setting for 2 days at 50 °C, the mixture had partially separated with 211 grams transparent aqueous layer at the bottom which was discarded after measuring only 1.7% NV solids. The cooled polymer mixture was too slippery to measure effectively with the Brookfield viscometer. The final appearance at 20 °C was an opaque, slippery viscous fluid.

### Example 66

A monomer mixture was prepared by stirring together 206.3 grams M3, 41.3 grams M7, and 13.8 grams M6 with 165 grams IPA.

To half (213 grams) of the monomer mixture, 8.6 grams M8 was added, sparged with nitrogen, warmed to 50 °C and poured into a 500 ml Pyrex addition funnel for delayed addition to the reactor with a FMI QG-50-1 CKC metering pump. The remaining half of the monomer mixture was diluted further with 28.7 grams IPA and charged to a 1 L glass resin reactor fitted with a stainless steel stirrer and reflux condenser, and then sparged with dry nitrogen for 20 minutes while heating to 63 °C by immersion in a 76 °C water bath. A starting initiator mixture was prepared, consisting of 1.24 grams V50 dissolved in 9.0 grams of DI water and 8.6 grams M8.

A delayed addition initiator mixture was prepared, consisting of 1.24 grams V50 dissolved in 6.9 grams of DI water and 6.8 grams IPA. The mixture was added to a 50 ml syringe barrel for delayed addition to the reactor with a FMI QG-6-0SSY metering pump, adjusted to deliver it in 85 minutes.

Polymerization was started by adding the starting mixture all at once to the reactor.

The contents soon became turbid and slightly bluish as the temperature slowly rose slightly. After 4 minutes, the pumps were turned on for both delayed addition initiator and monomer mixtures. The water bath was increased stepwise to 80 °C during delayed feeds with the process reaching 76 °C when monomer feed was completed in 42 minutes. 30 minutes after the initiator feed had run out, at 76 °C, nitrogen sparging, accompanied by a partial vacuum, was used to remove solvent over a period of 3 hours during whichtime a total of 539 grams of hot DI water was added to the slightly tan colored opaque polymer mixture. The final appearance at 20 °C was an opaque, slippery viscous fluid.

### Example 67

A starting monomer mixture was prepared by stirring together 20.6 grams M3, 68.8 grams M5, and 20.6 grams M2 with 79.8 grams IPA.

The mixture was charged to a 1 L glass resin reactor fitted with a stainless steel stirrer and reflux condenser, and then sparged with dry nitrogen for 20 minutes while heating to 66 °C by immersion in a 71 °C water bath.

A second monomer mixture was prepared by stirring together 22.2 grams M3, 73.3 grams M5, 22.3 grams M2 and 36.9 grams M8 with 169 grams IPA. This was sparged with nitrogen, warmed to 50 °C and poured into a 500 ml Pyrex addition funnel for delayed addition to the reactor with a FMI QG-50-1CKC metering pump.

A starting initiator mixture was prepared, consisting of 1.66 grams V50 dissolved in 11.9 grams of DI water and 34.4 grams M8.

A delayed addition initiator mixture was prepared, consisting of 2.24 grams V50 dissolved in 12.4 grams of DI water and 12.4 grams IPA. The mixture was added to a 50 ml syringe barrel for delayed addition to the reactor with a FMI QG-6-0SSY metering pump, adjusted to deliver it in 51 minutes.

Polymerization was started by adding the starting mixture all at once to the reactor. The temperature rose very quickly to 90 °C in 3 minutes and 15 grams of cold IPA was added to control reflux. The pump was started for delayed monomer feed adjusted to finish in 60 minutes. After 3 more minutes, at 84 °C, the delayed addition initiator feed was started. The water bath was increased stepwise to 80 °C during delayed feeds to hold the process temperature around 72 to 73 °C. The monomer mixture pump had been adjusted to deliver in 42 minutes. 20 minutes after the monomer and initiator feeds had run out, at 75 °C, 0.26 grams V50 dissolved in 2.6 grams DI water was added and the process held at 75 degrees for another 35 minutes. 268 grams of hot DI water were added making the translation solution somewhat more transparent. Nitrogen sparging, accompanied by a partial vacuum, was used to remove solvent over a period of 3 hours during which time a total of 351 more grams of hot DI water was added to the nearly transparent polymer mixture.

The finished viscous polymer liquid was kept in a 50 °C oven overnight. The final appearance at 20 °C was a translucent, highly viscous liquid.

### Example 68

A starting monomer mixture was prepared by stirring together 64.8 grams M1, 27.6 grams M5, and 51.6 grams M3 with 137.3 grams IPA.

The mixture was charged to a 1 L glass resin reactor fitted with a stainless steel stirrer and reflux condenser, and then sparged with dry nitrogen for 20 minutes while heating to 66 °C by immersion in a 71 °C water bath.

A second monomer mixture was prepared by stirring together 64.9 grams M1, 27.5 grams M5, 51.6 grams M3 and 8.6 grams M8 with 83 grams IPA. This was sparged with nitrogen, warmed to 50 °C and poured into a 500 ml Pyrex addition funnel for delayed addition to the reactor with a FMI QG-50-1CKC metering pump.

A starting initiator mixture was prepared, consisting of 1.1 grams V50 dissolved in 8.9 grams of DI water and 8.6 grams M8.

A delayed addition initiator mixture was prepared, consisting of 1.6 grams V50 dissolved in 9.1 grams of DI water and 9.1 grams IPA. The mixture was added to a 50 ml syringe barrel for delayed addition to the reactor with a FMI QG-6-0SSY metering pump.

Polymerization was started by adding the starting mixture all at once to the reactor. The temperature rose to 69 °C over 10 minutes and the pumps were started for delayed monomer and initiator feeds adjusted to finish in 50 minutes. The process temperature held steady at 68-71 °C during feeds and then the temperature was increased to 80 °C for the next hour. Then 302 grams of DI water was pumped in slowly over 18 minutes making the solution more viscous and transparent Nitrogen sparging, accompanied by a partial vacuum, was used to remove solvent over a period of 2 hours during which time a total of 185 grams of hot DI water was added and the polymer mixture became opaque and much more viscous.

The finished viscous polymer liquid was kept in a 50 °C oven overnight. The final appearance at 20 °C was an opaque white, extremely viscous paste.

### Example 69

A monomer mixture was prepared by stirring together 398.6 grams of M3 and 165.8 grams of M8 with 217 grams of IPA and 272 grams of 1,3-butanediol (BD).

Part of the monomer mixture, 263.1 grams (25%), were charged b a 1 L jacketed glass resin reactor fitted with a stainless steel stirrer and a Friedrichs reflux condenser, and then sparged with dry nitrogen for 60 minutes while heating to 60 °C by control of the jacket temperature. The remaining 790.3 grams of monomer mixture was poured into a 1000 ml Pyrex addition funnel for delayed addition to the reactor with a FMI QG-20-2CSC metering pump. The monomer addition funnel was wrapped in electrical heat tape and the monomer mixture was heated to 35-40 °C for addition to the reactor. An initiator mixture, consisting of 6.25 grams V50 dissolved in 48.0 grams of DI water, was added to a 125 ml addition funnel for delayed addition to the reactor with a FMI QG-20-0SSY metering pump.

At 60 °C, polymerization was initiated by adding a mixture of 1.7 grams V50 in 8.8 grams of DI water all at once to the stirred reactor. Within 30 seconds, the contents of the reactor began rising in temperature, but retained its transparency. Within 1 minute, the temperature of the contents had risen to 70 °C and the monomer delay feed pump was turned on. The initiator delay feed pump was turned on when the reactor contents reached a peak exotherm at 3 minutes. The monomer mixture had been adjusted to deliver in 62 minutes and the initiator mixture had been adjusted to deliver in 90 minutes. The monomer mixture within the Pyrex funnel was stirred periodically to maintain a homogenous appearance. The reactor jacket temperature was increased stepwise to 77 °C while the reactor temperature decreased slowly to 75 °C by the time 15% of the monomer mixture had been delivered to the reactor. The contents were viscous and remained nearly transparent After all the initiator mixture had been delivered, the reactor was held at 75 °C for an additional 2 hours.

After the 2 hour hold, a chaser of 5.9 grams of t-butyl hydroperoxide (70 wt% in water) was added to the reactor, then 3.0 grams of the monosodium salt of hydroxy methanesulfinic acid dehydrate (SFS) and 27.5 grams of water were added to the reactor over a 20 minute period. The chasing continued for 30 minutes and the polymer solution was cooled to 35 °C for transfer to a rotary evaporator. Volatile solvents were removed from the polymer solution over a 5 hour period by maintaining the temperature of the rotary evaporator at 65 °C and slowly increasing the vacuum from 250 mbar to 20 mbar. A final polymer solution was obtained as a clear viscous liquid.

### Example 70

A monomer mixture was prepared by stirring together 408.1 grams of M1 and 50.2 grams of M8 with 329 grams of IPA and 167 grams of deionized water.

Part of the monomer mixture, 264.7 grams (28%), were charged to a 1 L jacketed glass resin reactor fitted with a stainless steel stirrer and a Friedrichs reflux condenser, and then sparged with dry nitrogen for 60 minutes while heating to 65 °C by control of the jacket temperature. The remaining 690.6 grams of monomer mixture was poured into a 1000 ml Pyrex addition funnel for delayed addition to the reactor with a FMI QG-20-2CSC metering pump. The monomer addition funnel was wrapped in electrical heat tape and the monomer mixture was heated to 35-40 °C for addition to the reactor. An initiator mixture, consisting of 4.2 grams V50 dissolved in 37.0 grams of DI water, was added to a 125 ml addition funnel for delayed addition to the reactor with a FMI QG-20-0SSY metering pump.

At 65 °C, polymerization was initiated by adding a mixture of 1.1 grams V50 in 7.0 grams of DI water all at once to the stirred reactor. Within 2 minutes, the contents of the reactor began rising in temperature, but retained its transparency. After 9 minutes, the temperature of the contents had risen to 69 °C and pumps were turned on for both delayed addition initiator and monomer mixtures. The monomer mixture had been adjusted to deliver in 53 minutes and the initiator mixture had been adjusted to deliver in 93 minutes. The reactor jacket temperature was increased stepwise to 84 °C while the reactor temperature increased slowly to 75 °C by the time all the monomer mixture had been delivered to the reactor. The contents were viscous and remained nearly transparent. After all the initiator mixture had been delivered, the reactor was held at 75 °C for an additional 2 hours.

After the 2 hour hold, a chaser of 5.8 grams of t-butyl hydroperoxide (70 wt% in water) was charged to the reactor, then 3.0 grams of the SFS and 27.5 grams of water were added to the reactor over a 20 minute period. The chasing continued for 30 minutes. 205 grams of BD were then added to the reactor, and the polymer solution was cooled to 35 °C for transfer to a rotary evaporator. Volatile solvents were removed from the polymer solution over a 5 hour period by maintaining the temperature of the rotary evaporator at 65 °C and slowly increasing the vacuum from 250 mbar to 20 mbar. A final polymer solution was obtained as a clear highly viscous liquid.

### Example 71

A monomer mixture was prepared by stirring together 120.7 grams M1, 96.3 grams M3, 41.25 grams M6 and 51.6 grams M8 with 55 grams of IPA and 165 grams of BD.

The monomer mixture was warmed to 50 °C and 212 grams (40%) were charged to a 1 L glass resin reactor fitted with a stainless steel stirrer and reflux condenser, and then sparged with dry nitrogen for 30 minutes while heating to 65 °C by immersion in a 67 °C water bath. 27.5 grams additional IPA were added to the remaining 318 grams of monomer mixture, sparged with nitrogen and poured into a 500 ml Pyrex addition funnel for delayed addition to the reactor with a FMI QG-50-1CKC metering pump. An initiator mixture, consisting of 1.4 grams V50 dissolved in 12.1 grams of DI water, was added to a 50 ml syringe barrel for delayed addition to the reactor with a FMI QG-6-0SSY metering pump.

At 65 °C, polymerization was initiated by adding a mixture of 1.3 grams V50 in 7.2 grams of DI water all at once to the stirred reactor. Within 1 minute, the contents of the reactor began rising in temperature, but retained its transparency. After 8 minutes, the temperature of the contents had risen to 80 °C and pumps were turned on for both delayed addition initiator and monomer mixtures. The monomer mixture had been adjusted to deliver in 60 minutes and the initiator mixture had been adjusted to deliver in 90 minutes. The water bath temperature was increased stepwise to 76 °C while the reactor temperature increased slowly to 73 °C by the time all the monomer mixture had been delivered to the reactor. The contents were viscous and remained nearly transparent. After all the initiator mixture had been delivered, the reactor was heated further and the process held at 80-85 °C for an additional 2 hours.

Nitrogen sparging was then started and a water aspirator attached to apply partial vacuum and remove volatile solvent. After 15 minutes sparging, a mixture of 1.45 grams V50 in 17.4 grams BD and 1.8 grams water was added and the sparging continued for 2 more hours. Finally, the polymer solution was cooled to room temperature and 3.0 grams of 10% aqueous hydrogen peroxide was added as preservative. The final appearance was a transparent viscous liquid.

### Example 72

A starting monomer mixture was prepared by stirring together 64.1 grams M3, 13.8 grams M5, and 17.2 grams M8 with 81 grams IPA.

The mixture was charged to a 1 L glass resin reactor fitted with a stainless steel stirrer and reflux condenser, and then sparged with dry nitrogen for 20 minutes while heating to 55 °C by immersion in a 58 °C water bath.

A second monomer mixture was prepared by stirring together 149.5 grams M3, 32.1 grams M5 and 40.1 grams M8 with 153 grams BD. This was sparged with nitrogen, warmed to 50 °C and poured into a 500 ml Pyrex addition funnel for delayed addition to the reactor with a FMI QG-50-1CKC metering pump.

A starting initiator mixture was prepared, consisting of 0.73 grams V50 dissolved in 3.3 grams of DI water and 3.3 grams methanol.

A delayed addition initiator mixture was prepared, consisting of 3.70 grams V50 dissolved in 16.7 grams of DI water and 16.7 grams methanol. The mixture was added to a 50 ml syringe barrel for delayed addition to the reactor with a FMI QG-6-0SSY metering pump, adjusted to deliver it in 95 minutes.

Polymerization was started by adding the starting initiator mixture all at once to the reactor. The temperature rose quickly to 68 °C in 3 minutes and the pump was started for delayed monomer feed adjusted to finish in 64 minutes. After 1 more minute, the delayed addition initiator feed was started. The water bath was increased stepwise to 80 °C during delayed feeds to hold the process temperature around 70 to 74 °C. After both the monomer and initiator feeds were completed, the reactor was held at 70 °C for another 90 minutes. A chase step was initiated by adding 3.3 grams t-butyl hydroperoxide (70 wt% in water) to the reactor; then 1.8 grams SFS and 16.5 grams of water was added to the reactor over a 20 minute period followed by an additional 30 minute hold at 65 to 70 °C.

A nitrogen sparge and partial vacuum was applied to remove volatile solvents over a period of 3 hours. After cooling to room temperature a transparent viscous polymer solution was obtained.

### Example 73

A monomer mixture was prepared by stirring together 120.99 grams of M1, 41.27 grams of M6, 96.25 grams of M3, and 51.60 grams of M8 with 55 grams of IPA and 165 grams of glycerol.

The monomer mixture was warmed to 50 °C and 212 grams (40%) were charged to a 1 L glass resin reactor fitted with a stainless steel stirrer and reflux condenser, and then sparged with dry nitrogen for 30 minutes while heating to 68 °C by immersion in a 71 °C water bath. 27.5 grams additional IPA were added to the remaining 318 grams of monomer mixture and poured into a 500 ml Pyrex addition funnel for delayed addition to the reactor with a FMI QG-50-1CKC metering pump. An initiator mixture, consisting of 1.4 grams V50 dissolved in 12.1 grams of DI water, was added to a 50 ml syringe barrel for delayed addition to the reactor with a FMI QG-6-0SSY metering pump.

At 68 °C, polymerization was initiated by adding a mixture of 1.4 grams V50 in 7 grams of DI water all at once to the stirred reactor. The mixture was cloudy. In less than one minute, the reactor temperature had begun to rise. At 3 minutes, the temperature setting was adjusted to 90 °C. At 5 minutes, the batch had two visible phases and the temperature was at 71.5 °C. The feed pumps were turned on for the delayed addition initiator and monomer mixtures at that time. The monomer mixture had been adjusted to deliver in 60 minutes and the initiator mixture had been adjusted to deliver in 90 minutes. By the time all the monomer and initiator mixtures had been delivered to the reactor, the temperature was 82.4 °C and the content was white/opaque. The process was held above 80 °C for an additional 3 hours and 10 minutes.

At this point, the temperature was at 80 °C and nitrogen sparging was started with a water aspirator attached to apply partial vacuum to remove volatile solvent. After a 1 hour sparge, a chaser of 1.65 grams of V50, 13.20 grams of glycerol and 7.15 grams of water was added directly to the reactor. The chasing continued for 1 hour and 30 minutes and finally, at 7 hours, the polymer solution was cooled to room temperature and 3.03 grams of 10% aqueous hydrogen peroxide was added as preservative. The final appearance was a clear viscous liquid.

For each of the 73 examples, the weight of associative Z moieties as a percentage of the total weight of solids in the copolymer was calculated, as was the weight percentage ionogenic and ionic units, based on the particular mixture of starting monomers employed. In the examples, the associative Z moieties in each case were alkyl groups having eight or more carbon atoms (i.e., which are derived from monomers M1, M3 and M9), the total of ionic and ionogenic units were those which contained quaternium groups (i.e., repeating units derived from monomers M8, M11 and M13) or methacrylic acid groups (i.e., repeating units derived from M7). The ionic units were only those which contained quaternium groups. Two ratios were also calculated: the weight ratio of associative Z moieties to ionic groups, and the weight ratio of associative Z moieties to the sum of ionogenic and ionic groups. Table 1 also contains certain measured values for the samples, including the pH of the copolymer (5 wt% solids) in water, the viscosity of the final polymer solution in water at 50 and 20 °C, along with the temperature (in °C) of any endothermic thermal peak with a heat of fusion greater than 3 J/g as measured by differential scanning calorimetry on the final polymer after removal of water or solvent (100 wt% solids), most of which occur below 20 °C. Where vacancies appear in Table 1 with respect to viscosity, no measurement was obtained; where dashes appear in the thermal peak column, no thermal peak was observed that met the stated criteria.

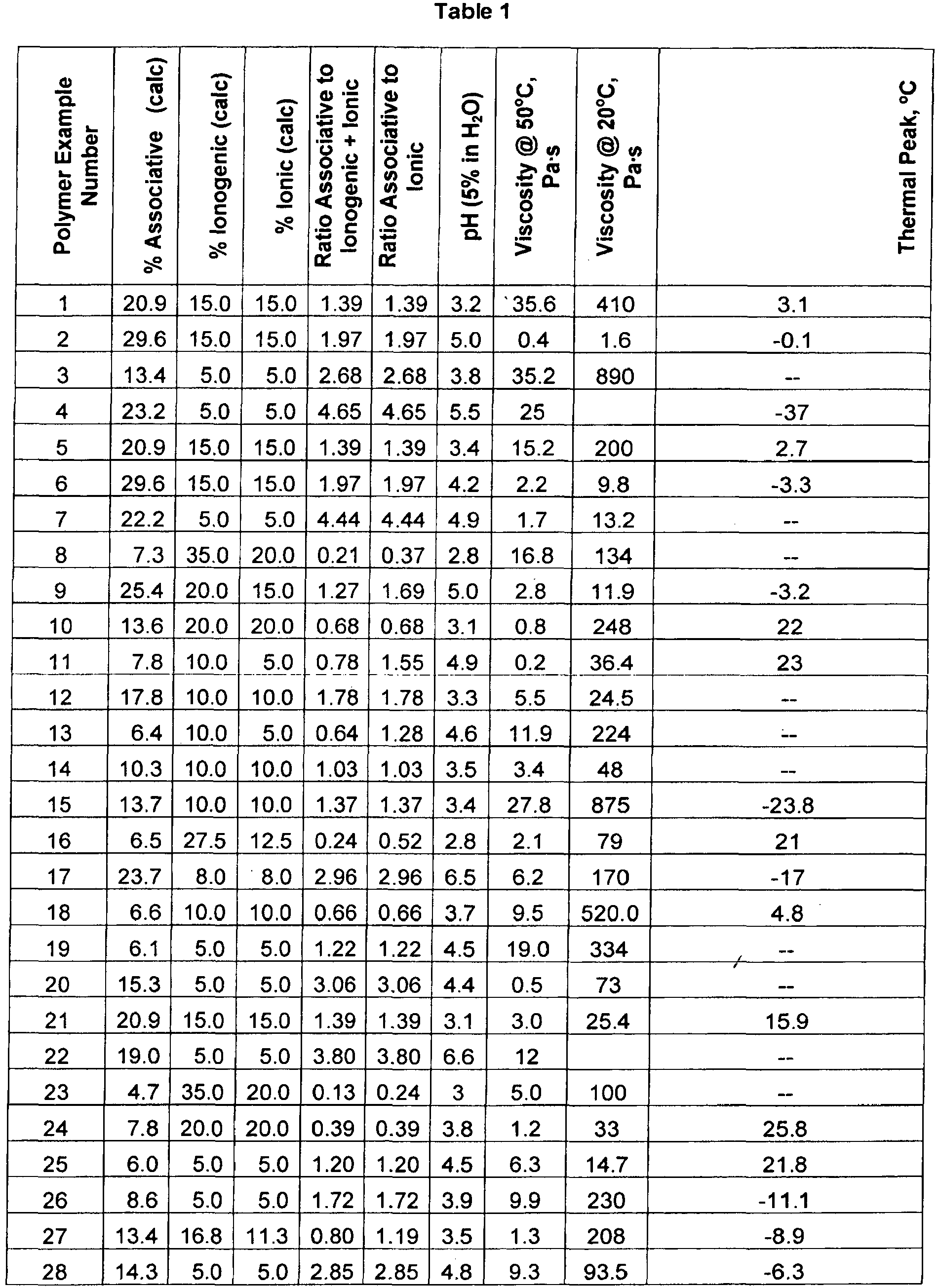

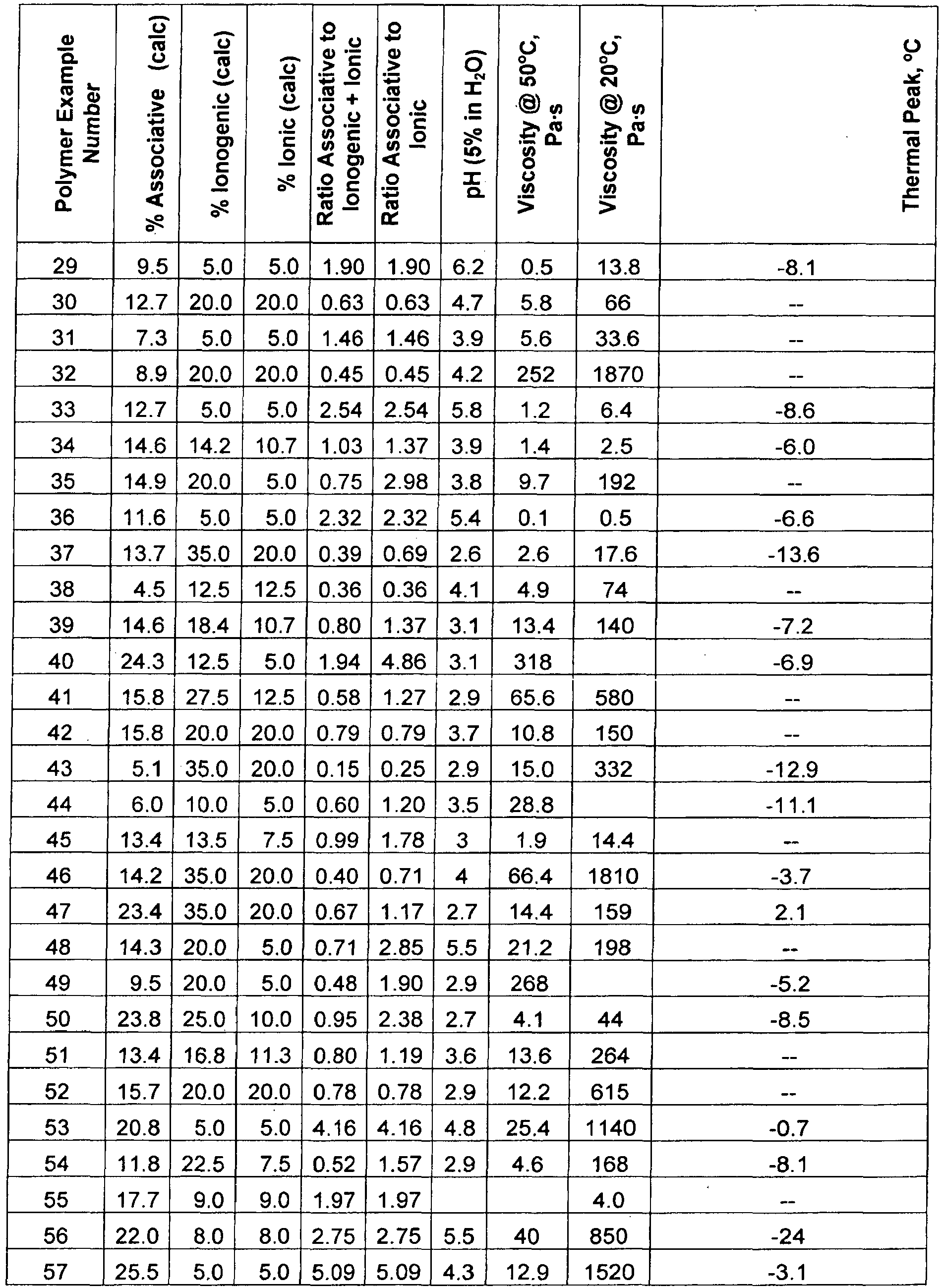

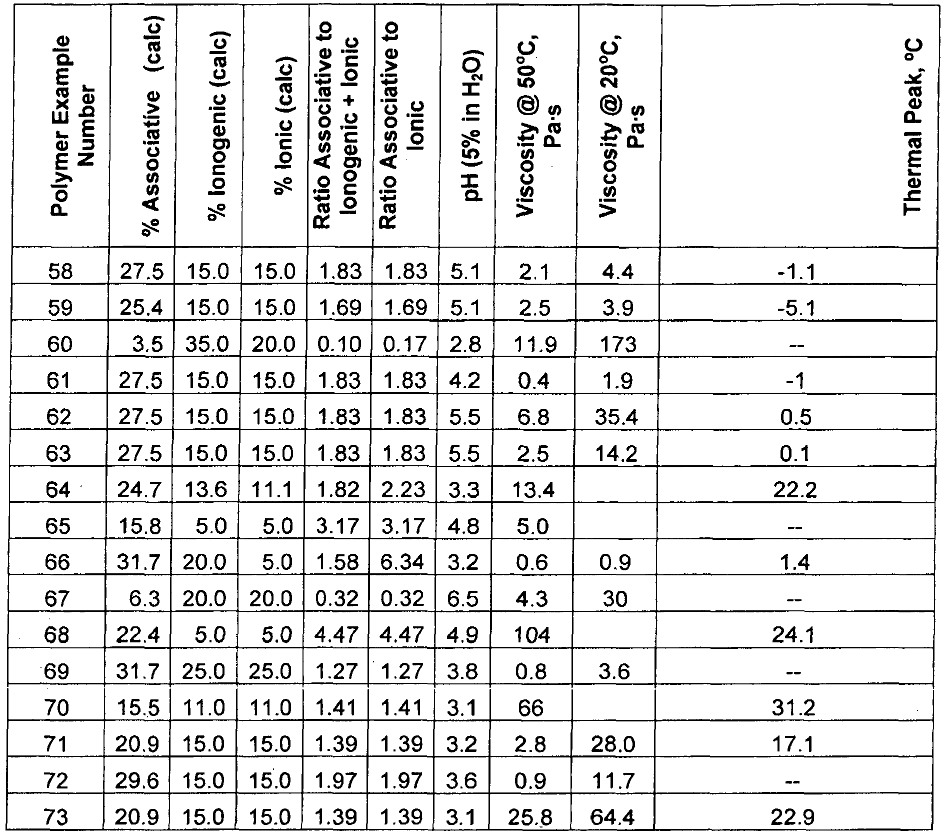

### Discussion of exemplary formulations incorporating sample copolymers

Cationic/cationogenic comb copolymers obtained in examples 1 through 72 were separately added as an ingredient to one or more of six different personal care products formulations as further described below, designated as series A through F. Within the formulations of series A through F, the indicated amount of cationic/cationogenic comb copolymer was added on a 100% polymer basis. These formulations were prepared as representative of common personal care product and/or to assist in analysis of features attained through the use of the copolymers when compared to a control formulation.

The A series formulation was designed as representative of a low pH formulation as might be found, for example, in facial cleansers. This formulation had the total composition reflected in Table 2.

**Table 2 - Low pH formulation (A series)**

| Ingredients | % weight |
|---|---|
| **Phase A** | |
| Caprylic/caprylate triglyceride | 13.0 |
| Cetearyl alcohol | 2.0 |
| Ceteareth 20 | 4.0 |
| Glyceryl Stearate | 3.0 |

| **Phase B** | |
|---|---|
| Water | balance |
| Salicylic acid | 2.0 |
| Glycerin | 3.0 |
| Cationic/cationogenic comb copolymer | 1.0 |
| DMDM Hydantoin | 0.5 |
| **Total** | 100.0 |

For each case in which one of the cationic/cationogenic comb copolymers formed in examples 1 to 72 was used in creating a series A formulation, the creation followed the same following procedure. First, all of phase A ingredients were added together in a vessel and heated to 80°C. Deionized (DI) water was added to a separate phase B vessel and the sample polymer was added while mixing under heat. In a step wise manner, glycerin was added to the phase B vessel, followed by salicylic acid until the mixture had reached 80°C and the salicylic acid was fully dissolved. The phase A mixture was added to phase B, heat was removed, and the mixture was allowed to cool. DMDM Hydantoin was added when the temperature had descended bebw 60°C. The formulation was continuously mixed until it had cooled to room temperature.

The A series formulations were then studied to determine the effectiveness of deposition of the active ingredient, salicylic acid, using in-vitro synthetic skin. Tests were conducted by applying and rubbing 2 mg/cm² of the A series formulation for 30 seconds on the skin. After application, the skin was left to dry for 15 minutes, then immersed in a petri-dish with 30 mL DI water under agitation with a smooth magnetic stirrer set at a speed of 300 rpm. Water temperature was kept close to body temperature (35 °C ± 2) for tests of formulations containing copolymers of examples 1 through 68, and was maintained at room temperature (22 °C ± 2) for tests of formulations containing copolymers of examples 69 through 72.

At 1, 2, 15 and 20 minutes after immersion, samples of the water were removed and filtered through a 5 micron filter. These samples were subsequently subjected_to high performance liquid chromatography (HPLC) to analyze the amount of salicylic acid extracted with the sample and thus permit calculation of the amount of salicylic acid deposited, which was then averaged across the various time intervals at which samples were collected.

The results of this study are found in Table 12, which contains a comprehensive listing of the results obtained for studies conducted on each of series A through F.

The series B formulation was designed as representative of a skin cream formulation, except that a higher percentage of water than ordinarily would be present was included to study sensory feel imparted by the cationic/cationogenic comb copolymer compositions. The B series formulation had the total composition reflected in Table 3.

**Table 3 - Skin Cream (B series)**

| Ingredients | % weight |
|---|---|
| **Phase A** | |
| DI water | balance |
| Cationic/cationogenic comb copolymer | 1.0 |

| **Phase B** | |
|---|---|
| Octinoxate | 1.0 |
| Cetearyl Alcohol | 5.5 |
| Ceteareth-25 | 0.5 |
| **Total** | 100.0 |

For each case in which one of the cationic/cationogenic comb copolymers formed in examples 1 to 72 was used in creating a series B formulation, the creation followed the same following procedure. First, all of the phase B ingredients were added together in a vessel and were mixed and heated to 80°C. The DI water and cationic/cationogenic comb copolymer were added to a separate vessel and also heated and mixed to 80°C, at which point phase B was added to Phase A. The mixture cooled to 65°C and homogenized at5000 rpm for 3 min. The formulation continued to be mixed until it had reached room temperature and was pH adjusted to about 5.5.

The B series formulations were evaluated for sensory characteristics according to the following protocol carried out by an expert sensory panel.

An unwetted back of the hand and inside of the forearm were used in this study, to which 0.2-0.4 grams of the B series formulation was dabbed with one finger, and with another finger, the same amount was applied of a comparative commercial product used as a positive control and which contained 2.5% by weight dimethicone.

The sample and control formulations were applied on the forearm or back of the hand simultaneously at a distance apart from each other of approximately 2 cm. Both were rubbed in at the same time using circular and front-and-back movements of the fingers. Application of the formulations required 1-2 minutes.

Properties of each sample were evaluated for feel, drag, slip and tack/stickiness as compared to the commercial product. Feel was the perception of a nice feel substance, typically characterized from a desirable light silky feel to an undesirable heavy greasy feel. Drag was the perception of resistance encountered when moving fingers across the skin to which the formulations had been applied, particularly as the formulation reach the near-dry and dry stages. Slip was the perception of a smooth film with slight to no hesitation when moving the finger across the skin over the area where the formulations had been applied, particularly as the formulation was wet, and tack was the perception of adhesive quality of the product to the skin during and after the rubbing process.

Baseline assessment and rating criteria were established priorto the start of the study based on three control formulations: a negative control (the B series formulation of Table 3 minus the cationic/cationogenic comb copolymer), a medium control (the B series formulation of Table 3 where Polyquaternium 7 is used in place of the cationic/cationogenic comb copolymer), and a positive control (Aveeno® "Active Naturals™ Skin Relief Moisturizing Lotion" available from Johnson & Johnson Consumer Companies, Inc.). Non-smoking panelists with normal to dry skin, male and female between ages 35 to 60 were used in this study. Panelists were asked not to use any other product on the hand and arm before the study. Panelists were trained in sensory attributes, and the same evaluators were used throughout the entirety of the sensory study. Panelists were asked to rate subjectively each of the experimental formulations relative to the commercial formulation. A grading scale of 1 to 5, with 1 being "very poor relative to the commercial product and same as the negative control," 3 being "same as the medium control," 4 denoting "same as the positive control commercial product," and 5 being "excellent relative to the commercial product" was used to assess the formulations.

The C series formulation was designed as representative of a facial cream formulation and had the total composition reflected Table 4.

**Table 4 - Facial cream (C series)**

| Ingredients | % weight |
|---|---|
| **Phase A** | |
| DI Water | balance |
| Glycerin | 4.0 |
| DMDM Hydantoin | 0.5 |
| Cationic/cationogenic comb copolymer | 1.0 |

| **Phase B** | |
|---|---|
| Isopropyl Palmitate | 6.0 |
| C12-15 Alkyl Benzoate | 5.0 |
| PEG-100 Stearate | 2.5 |
| Glyceryl Stearate | 2.5 |
| Cetearyl Alcohol | 2.0 |
| Dimethicone | 2.5 |
| **Total** | 100.0 |

For each case in which one of the cationic/cationogenic comb copolymers formed in examples 1 to 68 was used in creating a series C formulation, the creation followed the same following procedure. First, the phase B ingredients were added together in a vessel and were mixed while heating to 80°C. In a separate vessel, DI water and the sample cationic/cationogenic comb copolymer were added while mixing and heating was begun. To this mixture, the glycerin was then added. After both phases had reached 80°C, the phase B mixture was added into the phase A mixture and heat was removed. The DMDM hydantoin was added after the temperature had descended below 60°C, and the formulation was continuously mixed until it has reached room temperature, at which point the pH was adjusted using triethanolamine to between about 5.0 and 5.5.

The D series formulation was designed as representative of a hair conditioner formulation and had the total composition reflected in Table 5.

**Table 5 - Hair conditioner (D series)**

| Ingredients | % weiqht |
|---|---|
| **Phase A** | |
| DI water | balance |
| Cationic/cationogenic comb copolymer | 1.0 |
| Dimethiconol (and) TEA-Dodecylbenzenesulfonate | 1.0 |

| **Phase B** | |
|---|---|
| Octinoxate | 1.0 |
| Cetearyl Alcohol | 5.5 |
| Ceteareth-25 | 0.5 |
| **Total** | 100.0 |

For each case in which one of the cationic/cationogenic comb copolymers formed in examples 1 to 72 was used in creating a D series formulation, the creation followed the same following procedure. First, all of the phase B ingredients were added together in a vessel and heated under mixing to 80°C. DI water and the cationic/cationogenic comb copolymer was added to a separate vessel, which was also heated under mixing. Next, the dimethiconol and TEA-Dodecylbenzenesulfonate were added to the phase A vessel. After both phases reached 80°C, the phase B mixture was added to the phase A mixture, at which point heat was removed. When the composition reached 65°C, it was homogenized at 5000 rpm for 3 min, followed by continual mixing until it had cooled to room temperature.

The E series formulation was designed as representative of a skin cream formulation and had the total composition reflected in Table 6.

**Table 6 - Skin cream (E series)**

| Ingredients | % weight |
|---|---|
| **Phase A** | |
| Mineral Oil | 8.0 |
| PPG-15 stearyl ether | 1.0 |
| Steareth-21 | 2.5 |
| Steareth-2 | 1.1 |
| Cetearyl Alcohol | 4.0 |
| Dimethiconol (and) TEA-Dodecylbenzenesulfonate | 2.5 |
| **Phase B** | |
| DI water | balance |
| Glycerine | 3.0 |
| Propylene Glycol | 1.0 |
| Cationic/cationogenic comb copolymer | 1.0 |
| DMDM Hydantoin | 0.5 |
| **Total** | 100.0 |

For each case in which one of the cationic/cationogenic comb copolymers formed in examples 1 to 72 was used in creating a series E formulation, the creation followed the same following procedure. DI water was added to a vessel along with the cationic/cationogenic comb copolymer and mixing under heating was initiated. Glycerin and propylene glycol were then added to the vessel. In a separate vessel, all of the phase A ingredients were added together and mixed under heating. After both phases had been heated to 80°C, the vessel containing the phase A materials was to the vessel containing the phase B materials and heat was removed. DMDM hydantoin was added after the temperature had descended below 60°C and the formulation was continuously mixed until it has reached room temperature, at which point the pH was adjusted using triethanolamine to between about 5.0 and 5.5.

All of the series C, D, and E formulations were tested for effectiveness of the deposition of silicone as an active ingredient (present as dimethicone or dimethiconol). The silicone deposition studies were conducted using in-vitro synthetic skin to which 2 mg/cm² of the formulation was applied and rubbed in for 30 seconds. After application, the skin was left to dry for 15 minutes. The skin was then immersed in a Petri-dish with 30 mL DI water under agitation with a smooth magnetic stirrer having a speed of 300 rpm. Water temperature was kept close to body temperature (35 °C ± 2) for tests of formulations containing copolymers of examples 1 through 68, and was maintained at room temperature (22 °C ± 2) for tests of formulations containing copolymers of examples 69 through 72.

At 1, 2 and 15 minutes after immersion, samples of the water were removed and filtered through a 5 micron filter. These samples were subsequently subjected to inductively coupled plasma atomic emission spectroscopy (ICP-OES) using a Perkin-Elmer Optima 5000 dual view emission spectrometer with axial viewing with background correction. Results for silicon were corfirmed using three analytical wavelengths: 212.412 nm, 251.611 nm and 288.158 nm. Based on the ICP silicone results for each extract, the percentage of silicone deposited on the skin was calculated and then averaged across the various time intervals at which samples were collected.

The F series was designed as representative of a personal cleansing formulation and had the total composition reflected in Table 7.

**Table 7 - Cleanser (F Series)**

| Ingredients | % weight |
|---|---|
| **Phase A** | |
| Ammonium Lauryl Sulfate | 11.0 |
| Cocamidopropyl Betaine | 12.0 |
| Sodium C12-15 Pareth-15 Sulfate | 11.0 |

| **Phase B** | |
|---|---|
| Sodium Cocoyl Isothionate | 6.0 |
| DI Water | balance |
| Cationic/cationogenic comb copolymer | 1.0 |
| Sodium Chloride | 3.0 |
| **Total** | 100.0 |

For each case in which one of the cationic/cationogenic comb copolymers formed in examples 1 to 68 was used in creating a series F formulation, the creation followed the same following procedure. First, the phase A ingredients were added together in a vessel and mixed under heating. In a separate vessel, the phase B ingredients were addedtogether with the exception of NaCl, and were also mixed under heating. When both phases had reached 45°C, the phase A materials were added to the phase B materials. The NaCl was then added and mixed until fully dissolved, at which point heat was removed and the formulation was allowed to cool to room temperature and the pH was adjusted to between 5.0 and 5.5 using triethanolamine.

The series G formulation was designed as representative of an anti-aging formulation and had the total composition reflected in Table 8.

**Table 8 - Anti-aging cream 1 (G series)**

| Ingredients | % weight |
|---|---|
| **Phase A** | |

| DI water | balance |
|---|---|
| Cationic/cationogenic comb copolymer | 1.0 |
| Niacinamide | 2.0 |

| **Phase B** | |
|---|---|
| Octinoxate | 1.0 |
| Cetearyl Alcohol | 5.5 |
| Ceteareth-25 | 0.5 |
| **Total** | 100.0 |

For each case in which one of the cationic/cationogenic comb copolymers formed in examples 71 and 72 was used in creating a series G formulation, the creation followed the same procedure as described for the series B formulation. Within the formulations of series G and H, the indicated amount of cationic/cationogenic comb copolymer was added on a 100% polymer basis.

The series H formulation was designed as representative of an anti-aging formulation and had the total composition reflected in Table 9.

**Table 9 - Anti-aging cream 2 (H series)**

| Ingredients | % weight |
|---|---|
| **Phase A** | |
| DI water | balance |
| Cationic/cationogenic comb copolymer | 1.0 |
| Erythorbic acid | 1.0 |

| **Phase B** | |
|---|---|
| Octinoxate | 1.0 |
| Cetearyl Alcohol | 5.5 |
| Ceteareth-25 | 0.5 |
| **Total** | 100.0 |

For each case in which one of the cationic/cationogenic comb copolymers formed in examples 71 and 72 was used in creating a series H formulation, the creation followed the same procedure as described for the series B formulation.

Both the series G and H formulations were then studied to determine the effectiveness of deposition of the active anti-aging ingredient, niacinamide or erythorbic acid, using in-vitro synthetic skin. Tests were conducted by applying and rubbing 2 mg/cm² of the formulation for 30 seconds on the skin. After application, the skin was left to dry for 15 minutes, then immersed in a petri-dish with 30 mL DI water under agitation with a smooth magnetic stirrer set at a speed of 300 rpm. Water temperature was maintained at room temperature (22 °C ± 2).

At 1, 2 and 15 minutes after immersion, samples of the water were removed and filtered through a 5 micron filter. These samples were subsequently subjected to high performance liquid chromatography (HPLC) to analyze the amount of niacinamide or erythorbic acid extracted with the sample and thus permit calculation of the amount of anti-aging ingredient deposited, which was then averaged across the various time intervals at which samples were collected.

The results of this study are found in Table 13, which contains a comprehensive listing of the results obtained for studies conducted on each of series G through J.

For each of series A through H, a "blank" formulation was created as a control for comparative purposes that was identical except for the exclusion of any exemplary cationic/cationogenic comb copolymer composition.

The series I formulation was prepared by mixing 1 weight percent (on a 100% polymer basis) of the cationic/cationogenic comb copolymer of Example 72 into the commercial product "head & shoulders® pyrithione zinc dandruff shampoo, classic clean" available from Procter & Gamble. Mixing of the cationic/cationogenic comb copolymer and shampoo was conducted at room temperature. The commercial shampoo as purchased, without the cationic/cationogenic comb copolymer, served as the "blank" formulation for the series I formulation.

The series J formulation was prepared by mixing 1 weight percent (on a 100% polymer basis) of the cationic/cationogenic comb copolymer of Example 72 into the commercial product "pyrithione zinc dandruff shampoo, classic clean" available from Rite Aid Corporation. Mixing of the cationic/cationogenic comb copolymer and shampoo was conducted at room temperature. The commercial shampoo as purchased, without the cationic/cationogenic comb copolymer, served as the "blank" formulation for the series J formulation.

Both the series I and J formulations were then studied to determine the effectiveness of deposition of the active anti-dandruff ingredient, zinc pyrithione, using in-vitro synthetic skin. Tests were conducted by applying and rubbing 2 mg/cm² of the formulation for 30 seconds on the skin. After application, the skin was left to dry for 5 minutes, then immersed in a petri-dish with 30 mL DI water under agitation with a smooth magnetic stirrer set at a speed of 300 rpm. Water temperature was maintained at room temperature (22 °C ± 2).

At 30 seconds, 1 and 2 minutes after immersion, samples of the water were removed and filtered through a 5 micron filter. These samples were subsequently subjected to inductively coupled plasma atomic emission spectroscopy (ICP-OES) using a Perkin-Elmer Optima 5000 dual view emission spectrometer with axial viewing with background correction to analyze the amount of zinc pyrithione extracted with the sample and thus permit calculation of the amount of anti-dandruff ingredient deposited, which was then averaged across the various time intervals at which samples were collected.

Results of the testing conducted on the various formulations of series A through F are reflected in Table 12. For formulations in which effectiveness of active deposition was studied, Table 12 also shows comparative results reflecting the increase in deposition achieved with respect to the comparative blank formulation.

Table 12 also reflects the viscosity of formulations A-C and E-F as measured in Pascal seconds (Pa-s) employing a Brookfield rotating spindle viscometer, (Brookfield Model DV-III RV) using Helipath spindle B to E at about 10 revolutions per minute (rpm), at ambient room temperature (about 20-25 °C). Both spindle and rpm were adjusted to obtain measurements at torque readings within the recommended range for the instrument. Formulations were allowed to equilibrate at room temperature overnight prior to measurement. Three readings, each from a different location within the sample container, were taken for each sample and the results averaged.

Table 12 also reflects two additional propertes, appearance and viscosity of a solution of the copolymer (3 wt% solids) in distilled water for tests that were conducted on a limited number of representative samples. Vacant spaces within the table indicate that a particular sample was not used with respect to the corresponding formulation or that no results were obtained for that sample.

Results of the testing conducted on the various formulations of series G through J are reflected in Table 13, where comparative results reflecting the increase in deposition achieved with respect to the comparative blank formulation are also shown. Vacant spaces within the table indicate that a particular sample was not used with respect to the corresponding formulation or that no results were obtained for that sample.

Formulation K was designed as representative of a high ethanol content sunscreen formulation and had the total composition reflected in Table 10.

**Table 10 - High ethanol content sunscreen (K Formulation)**

| Ingredients | % weight |
|---|---|
| Ethanol | 70.0 |
| Cationic/cationogenic comb copolymer of Example 72 | 5.0 |
| C12-15 Alkyl Benzoate (and) Dipropylene Glycol Dibenzoate(and) PPG-15 Stearyl Ether Benzoate | 5.0 |
| Octocrylene | 5.0 |
| Ethylhexyl salicylate | 3.0 |
| Homosalate | 5.0 |
| Butyl Methoxydibenzoylmethane | 4.0 |
| Benzophenone-3 | 3.0 |
| **Total** | 100.0 |

Formulation K was created by addition of ingredients in the order shown at room temperature (22 °C ± 2). Within the formulations K and L, the indicated amount of cationic/cationogenic comb copolymer was added on a 100% polymer basis. Following addition of each ingredient, the mixture was stirred by hand until uniform. A stable and homogeneous sunscreen formulation was obtained.

The SPF of Formulation K is subsequently measured by IMS Inc. using the "In Vitro Water Resistance Protocol". An SPF of 50+ (i.e., greater than or equal to 50) is obtained and the sunscreen formulation is water resistant.

Formulation L was designed as representative of a sunscreen cream formulation and had the total composition reflected in Table 11.

**Table 11 - Sunscreen cream (L Formulation)**

| Ingredients | % weight |
|---|---|
| **Phase A** | |
| Mineral Oil | 2.0 |
| Emulsifying wax NF | 5.0 |
| Steareth-21 | 1.0 |
| Steareth-2 | 0.5 |
| Cetyl Alcohol | 1.0 |
| Octinoxate | 7.5 |
| Oxybenzone | 6.0 |
| Octisalate | 5.0 |

| **Phase B** | |
|---|---|
| DI water | balance |
| Hydroxyethyl cellulose | 0.3 |
| Cationic/cationogenic comb copolymer of Example 72 | 1.5 |
| Propylene glycol, diazolidinyl urea, methylparaben, propylparaben | 1.0 |
| **Total** | 100.0 |

Formulation L was created by the following procedure. First, all of the phase A ingredients were added together in a vessel and were mixed and heated to 80°C. The DI water, hydroxyethyl cellulose and cationic/cationogenic comb copolymer were added to a separate vessel and also heated and mixed to 80°C, at which point phase A was added b phase B. The mixture cooled to 65°C and homogenized at 5000 rpm for 3 min. The preservative (mixture of diazolidinyl urea and parabens in propylene glycol) was added once the formulation had cooled below 60 °C, followed by continual mixing until it reached room temperature.

The SPF of Formulation L is subsequently measured by IMS Inc. using the "In Vitro Water Resistance Protocol". An SPF of 30 is obtained and the sunscreen formulation is water resistant

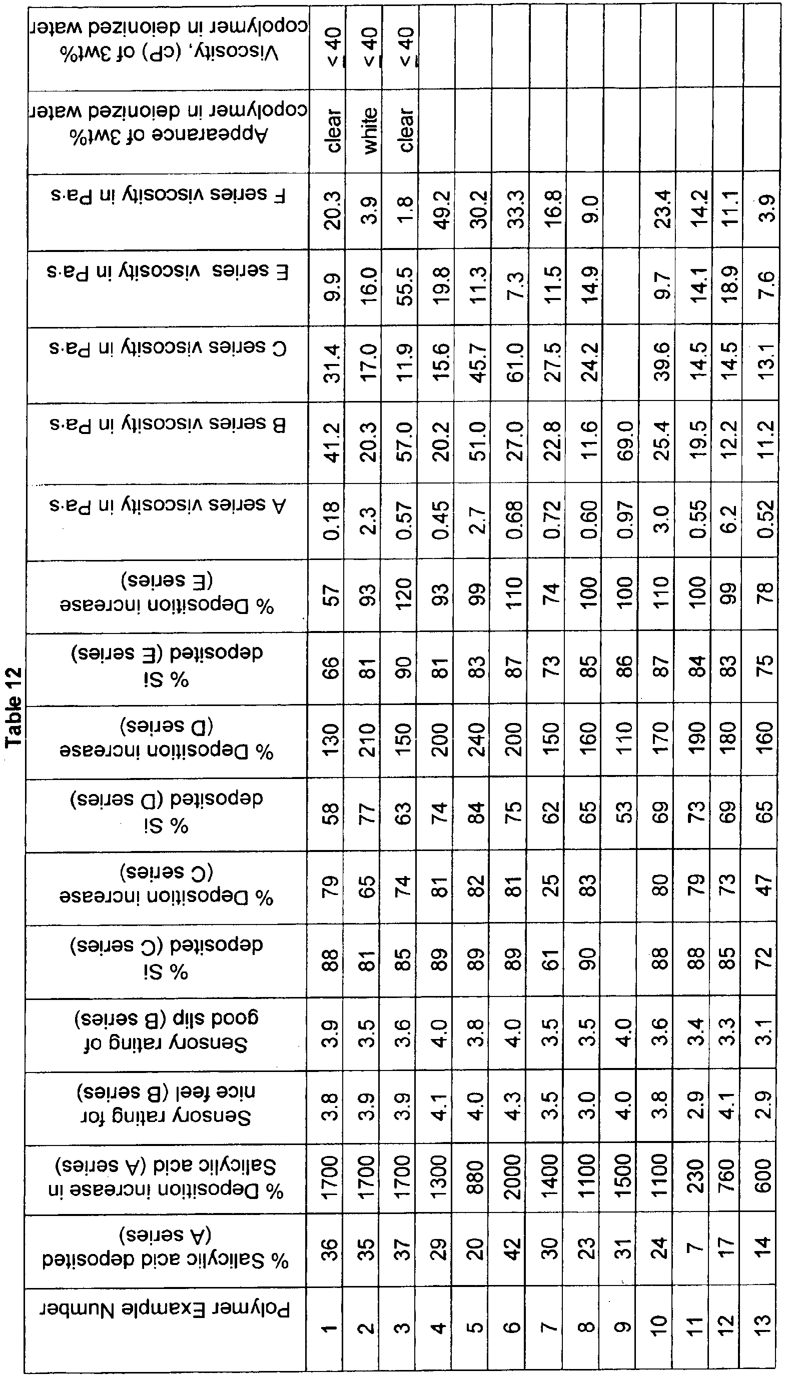

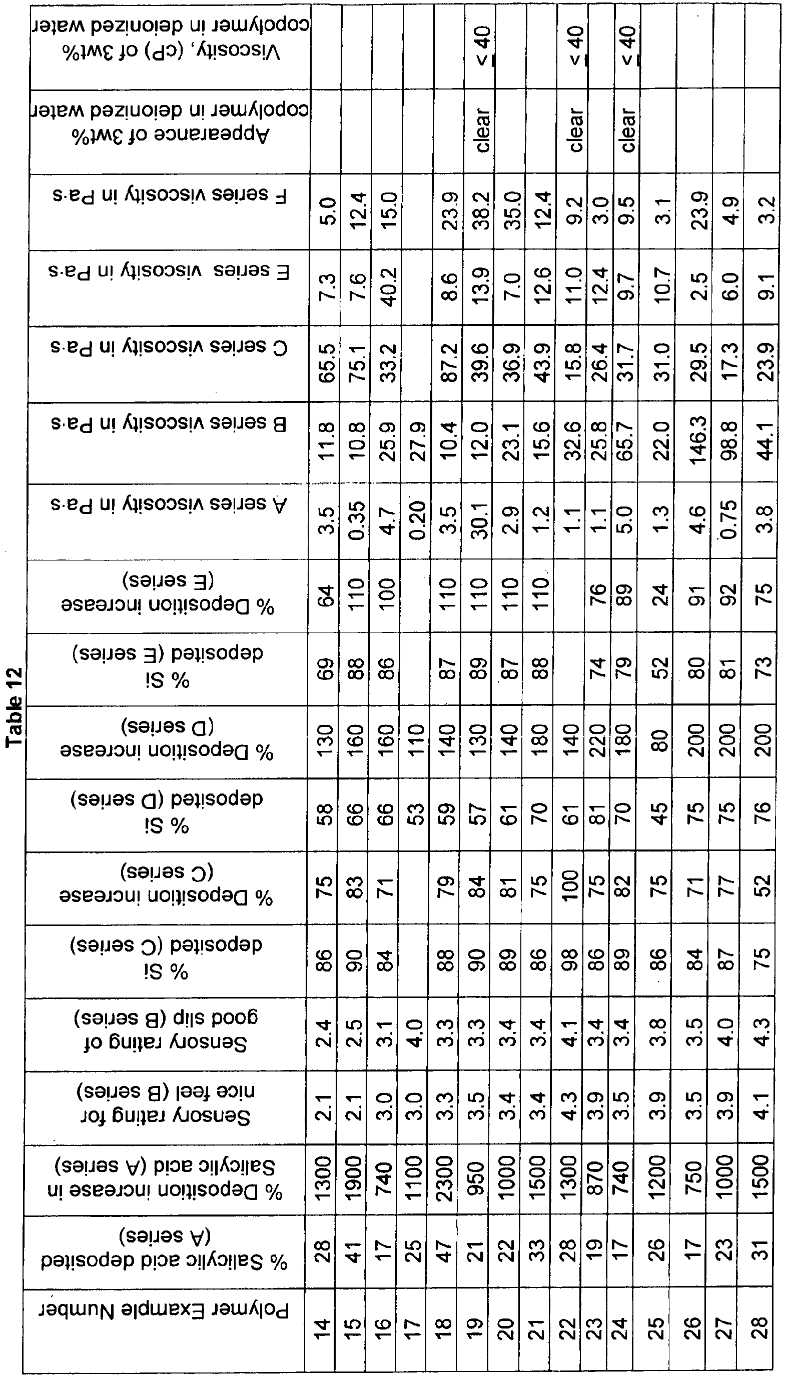

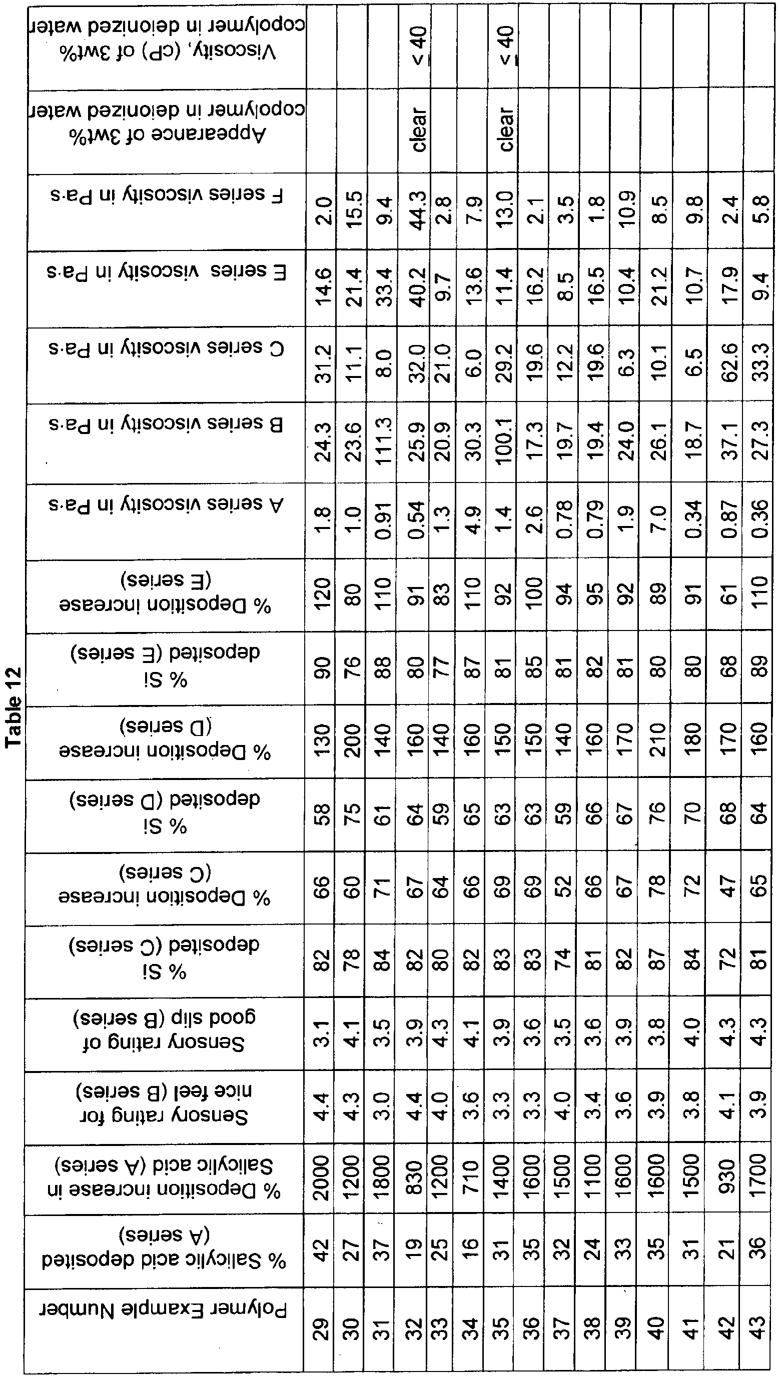

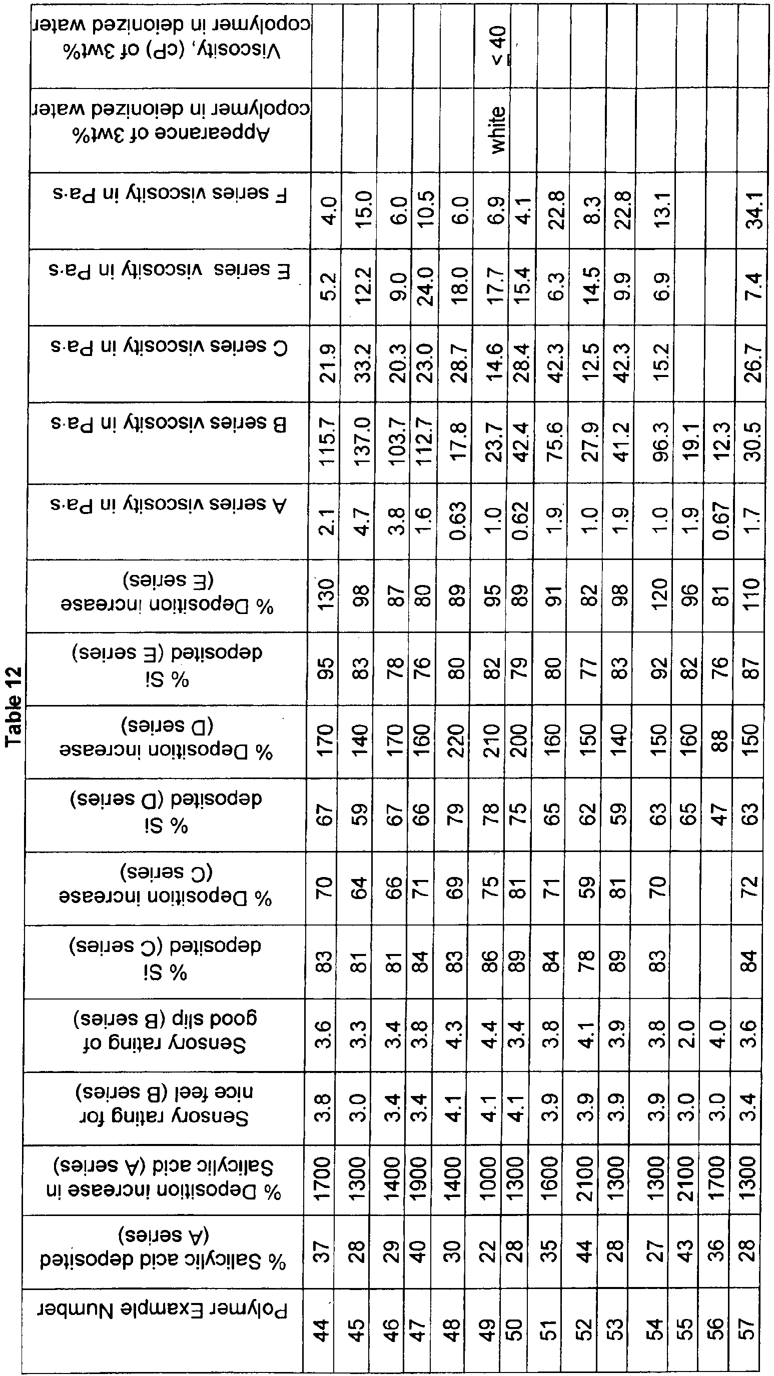

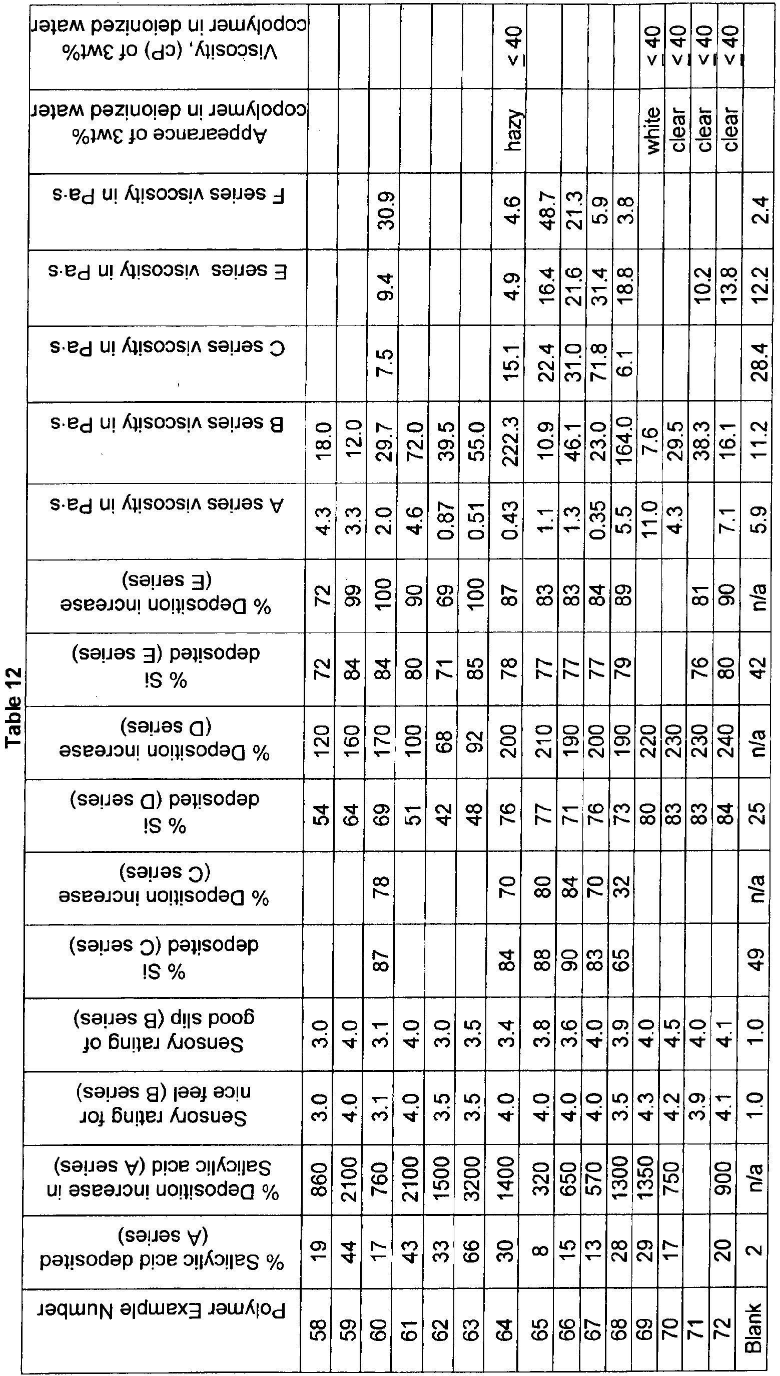

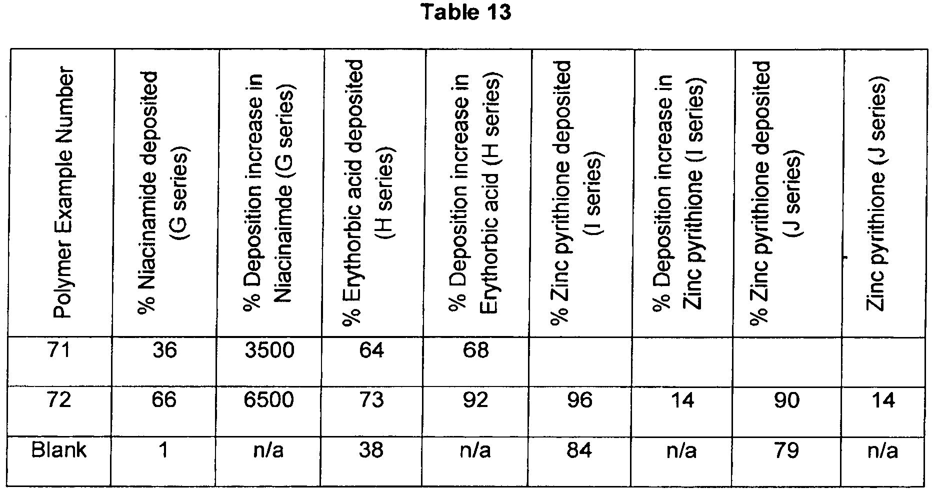

### Brief discussion of results of experimental personal care formulations

The results compiled in Table 12 demonstrated a significant increase in deposition of the active ingredients (salicylic acid and silicone) for every copolymer sample in each of the four formulations containing an active ingredient. Likewise, the sensory ratings for feel and slip were superior in each case compared to the "blank" formulation which rated poorly in the sensory test. The results compiled in Table 13 also demonstrated a significant increase in deposition of active ingredients (niacinamide, erythorbic acid and zinc pyrithione) for formulations containing a cationic/cationogenic comb copolymer.

Increased thickening of the formulations compared to the blank formulations was achieved in many cases. Because the formulations exhibited significant enhancement of active deposition and superior sensory effects, in cases where thickening was not observed, a thickening agent could be used as an additive in those cases. While additives can be used to achieve thickening, deposition and sensory attributes cannot readily be achieved by the inclusion of additives, illustrating the effectiveness of cationic/cationogenic comb copolymers in accordance with exemplary embodiments which can achieve such results.

In fact, for situations in which the copolymer did exhibit thickening of the overall formulation, in each case where the viscosity was measured of the copolymer sample in DI water alone (at 3 wt% solids copolymer), the viscosity was less than the viscometer's lowest measurement capability 0.4 Pa·s (40 cP), which demonstrates non-thickening of water. While not wishing to be bound by theory or explanation, it is believed that the cationic/cationogenic comb copolymers in accordance with exemplary embodiments collapse in water, but not within the personal care product formulations as a result of interactions due to the presence of other organic constituents.

### Discussion of other products in which copolyner may be employed

Although described primarily in conjunction for use with personal care products, it will be appreciated that cationic/cationogenic comb copolymer compositions in accordance with exemplary embodiments may also be useful as an ingredient for other commercial applications.

Exemplary applications include cleaning products used in household, industrial and/or institutional settings such as surface cleansers used for cleaning kitchens and bathrooms (e.g. cleaning countertops and tiled surfaces), toilet cleansers, toilet bowl rim cleansers, floor cleansers, wall cleansers, vehicle cleansers, air fresheners, dishwasher detergents and other dish soaps, laundry treatments and detergents, fabric softeners, spot reducers, fabric treatments and the like, all by way of example only. Other uses may include the use of such copolymers as ingredients in metal cleaners, scale removers, paint and varnish strippers, and polishes for furniture, shoes, cars, or metals, again all by way of example only.

The cationic/cationogeniccomb copolymers disclosed herein are also suitable for use in various industrial processes and applications. Exemplary applications include the use of such polymers as processing and finishing aids for textile dyeing/coating; in printing and finishing formulation inks; as stabilizers for chemical processes (e.g. when polymerizations are carried out in aqueous solution or emulsion, such as the preparation of photographic emulsions); as flocculants for wastewater treatment, as auxiliaries for papermaking such as the production of papers for use with inkjet printers, and as a humectant or gel former, for example.

As with the personal care products, it will further be appreciated that these other types of products including the cationic/cationogenic comb copolymers disclosed herein can be in any form, including but not limited to, liquid, gel, spray, emulsion, semisolid (such as pastes), and solid (such as stick, tablet or bar form), as may be desirable for the intended function of the particular composition.

The invention has been described with reference to certain aspects, but other aspects and embodiments are apparent to persons of skill in the art, and are included within the scope of the claims.

## Claims

1. A comb copolymer comprising:
A) one or more repeating units derived from olefinically unsaturated cationic or cationogenic comonomers; and
B) one or more repeating units having the formula 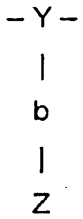 wherein Y is a moiety forming part of the copolymer backbone, Z is a moiety that exhibits association with other Z moieties or with other moieties within a formulation in which the copolymer will be employed, and b is a bond or moiety that links the Z moiety to the Y moiety, wherein the Z moiety is alkyl, aryl, aralkyl, fluoroalkyl, silicone or silane, and
wherein the weight ratio of associative moieties Z to ionic and ionogenic monomers is between 0.1 and 5.5, or
wherein the weight ratio of associative moieties Z to ionic monomers is between 0.15 and 6.5.

2. The comb copolymer of claim 1, further comprising one or more repeating units derived from a monomer selected from at least one of the following groups:
C) acrylamide monomers;
D) one or more olefinically unsaturated hydrophilic monomers that are not A, B or C; or
E) one or more olefinically unsaturated monomers that are not A, B, C or D.

3. The comb copolymer of claim 2, comprising one or more repeating units derived from monomers selected from at least two of groups C, D or E.

4. The comb copolymer of claim 3, comprising one or more repeating units derived from monomers selected from each of groups C, D and E.

5. The comb copolymer of claim 2, wherein the weight percent of ionic and ionogenic monomers in the copolymer is between 2 and 45 percent.

6. The comb copolymer of claim 2, wherein the weight percent of ionic monomers in the copolymer is between 2 and 45 percent.

7. The comb copolymer of claim 2, wherein the weight percent of associative moieties Z is between 2 and 50 percent.

8. The comb copolymer of claim 2, wherein the copolymer is non-crystalline.

9. The comb copolymer of claim 2, wherein the copolymer has a thermal peak at temperature less than 100 °C.

10. The comb copolymer of claim 2, wherein the viscosity of a 3 wt % (solids) solution of the copolymer in distilled water at 25 °C is less than 0.25 Pa·s (250 cP).

11. The comb copolymer of claim 2, wherein the copolymer is not crosslinked.

12. The comb copolymer of claim 2, wherein the monomers of group E are mono-olefinically unsaturated.

13. A personal care product comprising
a cosmetic base media; and
0.1 % to 20% by weight of the comb copolymer of any one of claims 1 to 12.

14. The personal care product of claim 13, further comprising an active ingredient, wherein the comb copolymer increases the deposition of the active ingredient to a keratinous structure by at least 10% over a personal care product having a same formulation without the comb copolymer.

15. A personal care composition comprising:
a cosmetic base media and at least one comb copolymer comprising:
A) one or more repeating units derived from olefinically unsaturated cationic or cationogenic comonomers; and
B) one or more repeating units having the formula 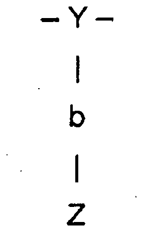
wherein Y is a moiety forming part of the copolymer backbone, Z is a moiety that exhibits association with other Z moieties or with other moieties within a formulation in which the copolymer will be employed, and b is a bond or moiety that links the Z moiety to the Y moiety; wherein the Z moiety is alkyl, aryl, aralkyl, fluoroalkyl, silicone or silane, wherein the weight percent of ionic monomers in the copolymer is between 2 and 45 percent, the weight percent of associative moieties Z is between 2 and 50 percent and the weight ratio of associative moieties Z to ionic and ionogenic monomers is between 0.1 and 5.5.

16. The personal care composition of claim 15 wherein the amount of comb polymer is sufficient to modify at least one of the following characteristics of the cosmetic media: deposition and retention of an active ingredient deposition and retention of a silicone oil, sensory perception and viscosity.

17. The personal care composition of claim 15 wherein the comb polymer is not crosslinked.

18. The personal care composition of claim 15 wherein the comb polymer has a Tm of less than 25 °C.

19. The personal care composition of claim 15 wherein the polymer exhibits a viscosity in water of less than 0.25 Pa·s (250cps) when measured at a temperature of 25 °C.

20. The personal care composition of claim 17 wherein the comb polymer has a water solubility of less than 50 wt%.

21. The personal care composition of claim 15 wherein the comb copolymer is comprised of 12 to 22 wt% of monomers of Group A, is comprised of 25 to 75 wt% of a monomer having the structure: 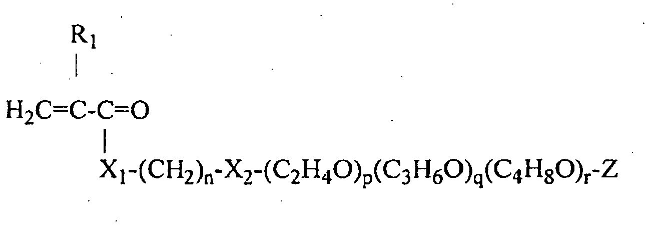 wherein X₁=O, n=q=r=0, p is between 3 and 30 and Z is an alkyl group having in the range of 10 to 18 carbon atoms, has between 7 and 30 wt% of associative moieties Z, is not crosslinked, and exhibits a solution viscosity in water at three weight percent (solids) of less than 0.1 Pa·s (100 cP) when measured at 25 °C.

22. The personal care composition of claim 15 wherein the comb copolymer is comprised of 12 to 22 wt% of monomers of Group A, is comprised of 25 to 75 wt% of a monomer having the structure: 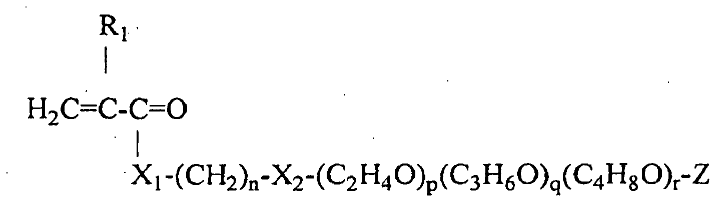 wherein X₁=O, n=q=r=0, p is between 3 and 30 and Z is an alkyl group having in the range of 10 to 18 carbon atoms, is comprised of an acrylamide monomer of Group C, has between 7 and 30 wt% of associative moieties Z, is not crosslinked, and exhibits a solution viscosity in water at three weight percent (solids) of less than 0.1 Pa·s (100 cP) when measured at 25 °C.

23. The personal care composition of claim 15 wherein the comb copolymer is comprised of 12 to 22 wt% of monomers of Group A, is comprised of about 25 to about 75 wt% of a monomer having the structure: 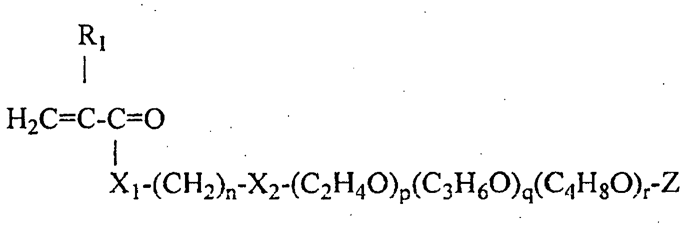 wherein X₁=O, n=q=r=0, p is between 3 and 30 and Z is an alkyl group having in the range of 10 to 18 carbon atoms, is comprised of a hydroxyalkyi [meth]acrylate monomer, has between 7 and 30 wt% of associative moieties Z, is not crosslinked, and exhibits a solution viscosity in water at three weight percent (solids) of less than 0.1 Pa·s (100 cP) when measured at 25 °C.

## Patentansprüche

1. Kammcopolymer, umfassend:
A) ein oder mehrere Wiederholungseinheiten, die sich von olefinisch ungesättigten kationischen oder kationogenen Comonomeren ableiten; und
B) ein oder mehrere Wiederholungseinheiten, die die Formel 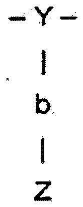 haben,
wobei Y eine Hälfte ist, die einen Teil des Copolymer-Rückgrats bildet, Z eine Hälfte ist, die eine Assoziation mit anderen Z-Hälften oder mit anderen Hälften innerhalb einer Formulierung aufweist, in der das Copolymer eingesetzt werden wird, und b eine Bindung oder Hälfte ist, die die Z-Hälfte an die Y-Hälfte bindet,
wobei die Z-Hälfte Alkyl, Aryl, Aralkyl, Fluoralkyl, Silikon oder Silan ist, und wobei das Gewichtsverhältnis der assoziativen Hälften Z zu ionischen und ionogenen Monomeren zwischen 0,1 und 5,5 ist, oder
wobei das Gewichtsverhältnis der assoziativen Hälften Z zu ionischen Monomeren zwischen 0,15 und 6,5 ist.

2. Kammpolymer nach Anspruch 1, ferner umfassend ein oder mehrere Wiederholungseinheiten, die sich von einem Monomer ableiten, das aus mindestens einer der folgenden Gruppen ausgewählt ist:
C) Acrylamidmonomere;
D) ein oder mehrere olefinisch ungesättigte hydrophile Monomere, die nicht A, B oder C sind; oder
E) ein oder mehrere olefinisch ungesättigte Monomere, die nicht A, B, C oder D sind.

3. Kammpolymer nach Anspruch 2, umfassend ein oder mehrere Wiederholungseinheiten, die sich von Monomeren ableiten, die zumindest aus zwei der Gruppen C, D oder E ausgewählt sind.

4. Kammpolymer nach Anspruch 3, umfassend ein oder mehrere Wiederholungseinheiten, die sich von Monomeren ableiten, die aus jeder der Gruppen C, D und E ausgewählt sind.

5. Kammpolymer nach Anspruch 2, wobei der Gewichtsprozentsatz der ionischen und ionogenen Monomere in dem Copolymer zwischen 2 und 45 Prozent ist.

6. Kammpolymer nach Anspruch 2, wobei der Gewichtsprozentsatz der ionischen Monomere in dem Copolymer zwischen 2 und 45 Prozent ist.

7. Kammpolymer nach Anspruch 2, wobei der Gewichtsprozentsatz der assoziativen Hälften Z zwischen 2 und 50 Prozent ist.

8. Kammpolymer nach Anspruch 2, wobei das Copolymer nicht kristallin ist.

9. Kammpolymer nach Anspruch 2, wobei das Copolymer eine thermische Spitze bei einer Temperatur von weniger als 100 °C hat.

10. Kammpolymer nach Anspruch 2, wobei die Viskosität einer 3 Gew.-% (Feststoffe) Lösung des Copolymers in destilliertem Wasser bei 25 °C weniger als 0,25 Pa·s (250 cP) ist.

11. Kammpolymer nach Anspruch 2, wobei das Copolymer nicht quervernetzt ist.

12. Kammpolymer nach Anspruch 2, wobei die Monomere der Gruppe E monoolefinisch ungesättigt sind.

13. Körperpflegeprodukt, umfassend
ein kosmetisches Basismedium; und 0,1 Gew.-% bis 20 Gew.-% des Kammpolymers nach einem der Ansprüche 1 bis 12.

14. Körperpflegeprodukt nach Anspruch 13, ferner umfassend einen aktiven Bestandteil, wobei das Kammpolymer die Deposition des aktiven Bestandteils auf eine keratinöse Struktur um mindestens 10 % gegenüber einem Körperpflegeprodukt, das eine gleiche Formulierung ohne das Kammpolymer hat, erhöht.

15. Körperpflegezusammensetzung, umfassend:
ein kosmetisches Basismedium und mindestens ein Kammpolymer,
umfassend:
A) ein oder mehrere Wiederholungseinheiten, die sich von olefinisch ungesättigten kationischen oder kationogenen Comonomeren ableiten; und
B) ein oder mehrere Wiederholungseinheiten, die die Formel 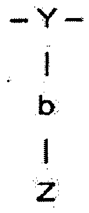
haben,
wobei Y eine Hälfte ist, die einen Teil des Copolymer-Rückgrats bildet, Z eine Hälfte ist, die eine Assoziation mit anderen Z-Hälften oder mit anderen Hälften innerhalb einer Formulierung aufweist, in der das Copolymer eingesetzt werden wird, und b eine Bindung oder Hälfte ist, die die Z-Hälfte an die Y-Hälfte bindet; wobei die Z-Hälfte Alkyl, Aryl, Aralkyl, Fluoralkyl,
Silikon oder Silan ist, wobei der Gewichtsprozentsatz von ionischen Monomere in dem Copolymer zwischen 2 und 45 Prozent ist, der Gewichtsprozentsatz der assoziativen Hälften Z zwischen 2 und 50 Prozent ist und das Gewichtsverhältnis der assoziativen Hälften Z zu ionischen und
ionogenen Monomeren zwischen 0,1 und 5,5 ist.

16. Körperpflegezusammensetzung nach Anspruch 15, wobei die Menge an Kammpolymer ausreichend ist, um mindestens eines der folgenden Merkmale von dem kosmetischen Medium zu modifizieren: Deposition und Retention eines aktiven Bestandteils, Deposition und Retention eines Silikonöls, Sinneswahrnehmung und Viskosität.

17. Körperpflegezusammensetzung nach Anspruch 15, wobei das Kammpolymer nicht quervernetzt ist.

18. Körperpflegezusammensetzung nach Anspruch 15, wobei das Kammpolymer eine Tm von weniger als 25 °C hat.

19. Körperpflegezusammensetzung nach Anspruch 15, wobei das Polymer eine Viskosität in Wasser von weniger als 0,25 Pa·s (250 cP) aufweist, wenn sie bei einer Temperatur von 25 °C gemessen wird.

20. Körperpflegezusammensetzung nach Anspruch 17, wobei das Kammpolymer eine Wasserlöslichkeit von weniger als 50 Gew.-% hat.

21. Körperpflegezusammensetzung nach Anspruch 15, wobei das Kammpolymer zu 12 bis 22 Gew.-% aus Monomeren der Gruppe A gebildet ist, zu 25 bis 75 Gew.-% aus einem Monomer gebildet ist, das die Struktur: 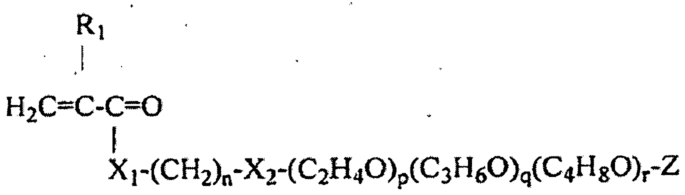 hat,
wobei X₁ = O, n = q = r = 0, p zwischen 3 und 30 ist und Z eine Alkylgruppe ist, die in dem Bereich von 10 bis 18 Kohlenstoffatome hat, das Kammpolymer zwischen 7 und 30 Gew.-% assoziative Hälften Z hat, es nicht quervernetzt ist und es eine Lösungsviskosität in Wasser bei drei Gewichtsprozent (Feststoffe) von weniger als 0,1 Pa·s (100 cP) aufweist, wenn sie bei 25 °C gemessen wird.

22. Körperpflegezusammensetzung nach Anspruch 15, wobei das Kammpolymer zu 12 bis 22 Gew.-% aus Monomeren der Gruppe A gebildet ist, zu 25 bis 75 Gew.-% aus einem Monomer gebildet ist, das die Struktur: 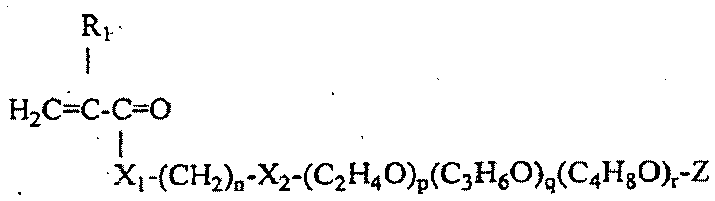 hat,
wobei X₁ = O, n = q = r = 0, p zwischen 3 und 30 ist und Z eine Alkylgruppe ist, die in dem Bereich von 10 bis 18 Kohlenstoffatome hat, das Kammpolymer aus einem Acrylamidmonomer der Gruppe C gebildet ist, es zwischen 7 und 30 Gew.-% assoziative Hälften Z hat, es nicht quervernetzt ist und es eine Lösungsviskosität in Wasser bei drei Gewichtsprozent (Feststoffe) von weniger als 0,1 Pa·s (100 cP) aufweist, wenn sie bei 25 °C gemessen wird.

23. Körperpflegezusammensetzung nach Anspruch 15, wobei das Kammpolymer zu 12 bis 22 Gew.-% aus Monomeren der Gruppe A gebildet ist, zu 25 bis 75 Gew.-% aus einem Monomer gebildet ist, das die Struktur: 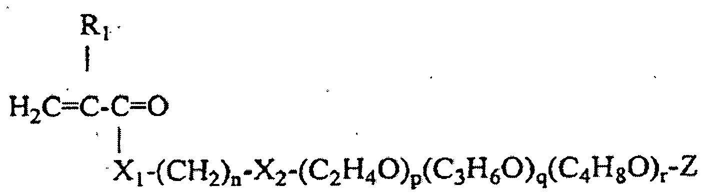 hat,
wobei X₁ = O, n = q = r = 0, p zwischen 3 und 30 ist und Z eine Alkylgruppe ist, die in dem Bereich von 10 bis 18 Kohlenstoffatome hat, das Kammpolymer aus einem Hydroxylkylmethacrylat-Momonomer gebildet ist, es zwischen 7 und 30 Gew.-% assoziative Hälften Z hat, es nicht quervernetzt ist und es eine Lösungsviskosität in Wasser bei drei Gewichtsprozent (Feststoffe) von weniger als 0,1 Pa·s (100 cP) aufweist, wenn sie bei 25 °C gemessen wird.

## Revendications

1. Copolymère en peigne comprenant :
A) un ou plusieurs motifs répétitifs dérivés de comonomères cationiques ou cationogènes à insaturation oléfinique ; et
B) un ou plusieurs motifs répétitifs répondant à la formule 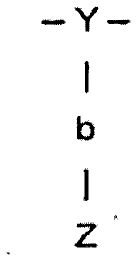 dans laquelle Y est une partie formant un groupement du squelette du copolymère, Z est un groupement qui présente une association avec d'autres groupements Z ou avec d'autres groupements dans une formulation dans laquelle le copolymère va être utilisé, et b est une liaison ou un groupement qui lie le groupement Z au groupement Y,
dans lequel le groupement Z est un groupe alkyle, aryle, aralkyle, fluoroalkyle, silicone ou silane, et
dans lequel le rapport en poids des groupements associatifs Z aux monomères ioniques et ionogènes est compris entre 0,1 à 5,5, ou
dans lequel le rapport en poids des groupements associatifs Z aux monomères ioniques est compris entre 0,15 et 6,5.

2. Copolymère en peigne selon la revendication 1, comprenant en outre un ou plusieurs motifs répétitifs dérivés d'un monomère choisi parmi au moins un des groupes suivants :
C) les monomères d'acrylamide ;
D) un ou plusieurs monomères hydrophiles à insaturation éthylénique qui ne sont pas A, B ou C ; ou
E) un ou plusieurs monomères à insaturation éthylénique qui ne sont pas A, B, C ou D.

3. Copolymère en peigne selon la revendication 2, comprenant un ou plusieurs motifs répétitifs dérivés de monomères choisis parmi au moins deux des groupes C, D ou E.

4. Copolymère en peigne selon la revendication 3, comprenant un ou plusieurs motifs répétitifs dérivés de monomères choisis parmi chacun des groupes C, D et E.

5. Copolymère en peigne selon la revendication 2, dans lequel le pourcentage en poids des monomères ioniques et ionogènes dans le copolymère est compris entre 2 et 45 %.

6. Copolymère en peigne selon la revendication 2, dans lequel le pourcentage en poids des monomères ioniques dans le copolymère est compris entre 2 et 45 %.

7. Copolymère en peigne selon la revendication 2, dans lequel le pourcentage en poids de groupements associatifs Z est compris entre 2 et 50 %.

8. Copolymère en peigne selon la revendication 2, dans lequel le copolymère n'est pas cristallin.

9. Copolymère en peigne selon la revendication 2, dans lequel le copolymère a un pic thermique à une température inférieure à 100 °C.

10. Copolymère en peigne selon la revendication 2, dans lequel la viscosité d'une solution à 3 % en poids (matières solides) du copolymère dans de l'eau distillée à 25 °C est inférieure à 0,25 Pa.s (250 cP).

11. Copolymère en peigne selon la revendication 2, dans lequel le copolymère n'est pas réticulé.

12. Copolymère en peigne selon la revendication 2, dans lequel les monomères du groupe E sont à insaturation mono-oléfinique.

13. Produit d'hygiène corporelle comprenant
un milieu de base cosmétique ; et
de 0,1 % à 20 % en poids du copolymère en peigne selon l'une quelconque des revendications 1 à 12.

14. Produit d'hygiène corporelle selon la revendication 13, comprenant en outre un principe actif, dans lequel le copolymère en peigne augmente le dépôt du principe actif sur une structure kératinique d'au moins 10 % par rapport à un produit d'hygiène corporelle de même formulation, mais sans le copolymère en peigne.

15. Composition d'hygiène corporelle comprenant :
un milieu de base cosmétique et au moins un copolymère en peigne comprenant :
A) un ou plusieurs motifs répétitifs dérivés de comonomères cationiques ou cationogènes à insaturation oléfinique ; et
B) un ou plusieurs motifs répétitifs répondant à la formule 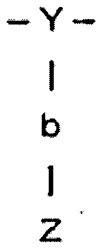
dans laquelle Y est une partie formant un groupement du squelette du copolymère, Z est un groupement qui présente une association avec d'autres groupements Z ou avec d'autres groupements dans une formulation dans laquelle le copolymère va être utilisé, et b est une liaison ou un groupement qui lie le groupement Z au groupement Y ; dans laquelle le groupement Z est un groupe alkyle, aryle, aralkyle, fluoroalkyle, silicone ou silane ; dans laquelle le pourcentage en poids des monomères ioniques dans le copolymère est compris entre 2 et 45 %, le pourcentage en poids des fragments associatifs Z est compris entre 2 et 50 % et le rapport en poids des groupements associatifs Z aux monomères ioniques et ionogènes est compris entre 0,1 et 5,5.

16. Composition d'hygiène corporelle selon la revendication 15, dans laquelle la quantité du polymère en peigne est suffisante pour modifier au moins une des caractéristiques suivantes du milieu cosmétique : dépôt et rétention du principe actif, dépôt et rétention d'une huile de silicone, perception sensorielle et viscosité.

17. Composition d'hygiène corporelle selon la revendication 15, dans laquelle le polymère en peigne n'est pas réticulé.

18. Composition d'hygiène corporelle selon la revendication 15, dans laquelle le polymère en peigne a une Tm inférieure à 25 °C.

19. Composition d'hygiène corporelle selon la revendication 15, dans laquelle le polymère présente une viscosité dans l'eau inférieure à 0,25 Pa.s (250 cps) lorsqu'elle est mesurée à une température de 25 °C.

20. Composition d'hygiène corporelle selon la revendication 17, dans laquelle le polymère en peigne a une solubilité dans l'eau inférieure à 50 % en poids.

21. Composition d'hygiène corporelle selon la revendication 15, dans laquelle le polymère en peigne se compose de 12 à 22 % en poids des monomères du groupe A, se compose de 25 à 75 % en poids d'un monomère ayant la structure : 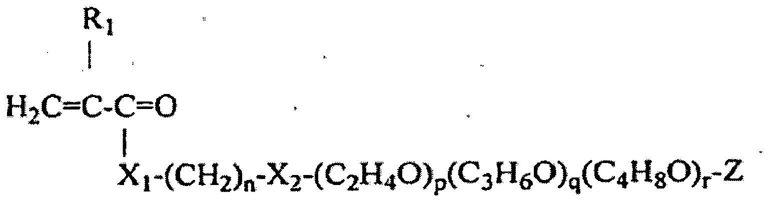 dans laquelle Xᵢ = O, n = q = r = 0, p est compris entre 3 et 30 et Z est un groupe alkyle ayant de 10 à 18 atomes de carbone, a entre 7 et 30 % en poids de groupements associatifs Z, n'est pas réticulé et présente une viscosité en solution dans l'eau à 3 % en poids (matières solides) inférieure à 0,1 Pa.s (100 cP) lorsqu'elle est mesurée à 25 °C.

22. Composition d'hygiène corporelle selon la revendication 15, dans laquelle le polymère en peigne se compose de 12 à 22 % en poids des monomères du groupe A, se compose de 25 à 75 % en poids d'un monomère ayant la structure : 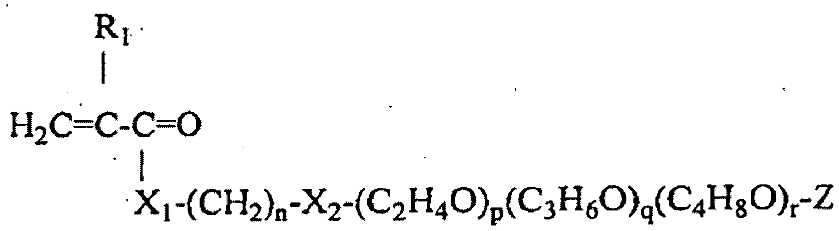 dans laquelle X₁ = O, n = q = r = 0, p est compris entre 3 et 30 et Z est un groupe alkyle ayant de 10 à 18 atomes de carbone, se compose d'un monomère d'acrylamide du groupe C, a entre 7 et 30 % en poids de groupements associatifs Z, n'est pas réticulé et présente une viscosité en solution dans l'eau à 3 % en poids (matières solides) inférieure à 0,1 Pa.s (100 cP) lorsqu'elle est mesurée à 25 °C.

23. Composition d'hygiène corporelle selon la revendication 15, dans laquelle le polymère en peigne se compose de 12 à 22 % en poids des monomères du groupe A, se compose de 25 à 75 % en poids d'un monomère ayant la structure : 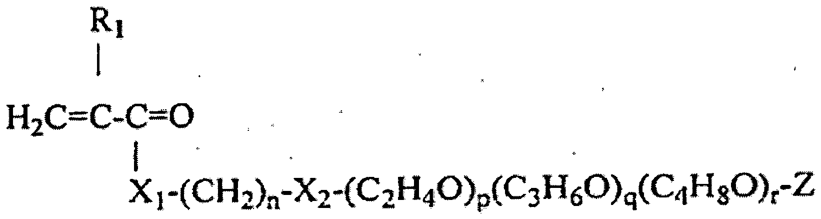 dans laquelle X₁ = O, n = q = r = 0, p est compris entre 3 et 30 et Z est un groupe alkyle ayant de 10 à 18 atomes de carbone, se compose d'un monomère de (méth)acrylate d'hydroxyalkyle, a entre 7 et 30 % en poids de groupements associatifs Z, n'est pas réticulé et présente une viscosité en solution dans l'eau à 3 % en poids (matières solides) inférieure à 0,1 Pa.s (100 cP) lorsqu'elle est mesurée à 25 °C.
